# EUROPEAN PATENT APPLICATION

(11) **EP 4 410 941 A1**
(43) Date of publication of application: **07.08.2024**
(21) Application number: 24155354.4
(22) Date of filing: 01.02.2024
(51) Int. Cl.: C11D 3/386

(54) **DETERGENT COMPOSITIONS CONTAINING ENZYMES**

(30) Priority: 01.02.2023 US 202363442516 P
(71) Applicant: The Procter & Gamble Company, Cincinnati, OH 45202 (US)
(72) Inventor: BABE, Lilia Maria, Palo Alto, CA 94304 (US); BASLER, Joshua Roy, Palo Alto, CA 94304 (US); BRADLEY, Neil, Newcastle upon Tyne, NE12 9BZ (GB); ESTELL, David Aaron, Palo Alto, CA 94304 (US); GARSKE, Adam, Palo Alto, CA 94304 (US); JACKSON, Michelle, Newcastle upon Tyne, NE12 9BZ (GB); KAPER, Thijs, Palo Alto, CA 94304 (US); KORZYCKA, Karolina Anna, Newcastle upon Tyne, NE12 9BZ (GB); STONER, Michael, Palo Alto, CA 94304 (US)
(74) Representative: P&G Patent Belgium UK

(57) **Abstract**

Cleaning compositions particularly for laundry, can include one or more subtilisin variant and cleaning adjunct. Methods of treating surfaces, particularly fabrics, can include contacting the surface with an aqueous wash liquor having the cleaning composition therein. The subtilisin variant may have improved stability and/or soil removal compared to one or more reference subtilisin.

## Description

### REFERENCE TO A SEQUENCE LISTING

This application contains a Sequence Listing in computer readable form. The contents of the electronic sequence listing in computer readable form (CM05530M_Sequence_listing.xml; 15,692 bytes; created February 1, 2024) is hereby incorporated by reference.

### FIELD OF THE INVENTION

This invention relates to cleaning compositions comprising subtilisin variants which are effective in cold water, and that have improved stability and/or stain/soil removal compared to compositions comprising reference subtilisin. The invention also relates to methods of making and using such cleaning compositions. The invention is particularly useful for laundry cleaning.

### BACKGROUND OF THE INVENTION

Detergent manufacturers incorporate proteases into their products to provide good cleaning of stains (such as blood). It is a constant challenge to provide improved cleaning efficacy. Cleaning efficacy is improved by providing cleaning compositions comprising a protease which has good proteolytic activity and preferably also has good stability. Examples of stability include, for example in the cleaning composition over time, such that the protease will provide good levels of proteolytic cleaning even after addition to the other materials in the cleaning composition and/or in the conditions on storage and/or shipment of the cleaning composition, and/or in the conditions in the wash liquor.

Many well-known procedures exist for measuring proteolytic activity (Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a commercial substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and Keratin Azure (Sigma-Aldrich K8500). Colorimetric assays utilizing these substrates are well known in the art (see, e.g., WO 99/34011 and U.S. Pat. No. 6,376,450, both of which are incorporated herein by reference).

Serine proteases are enzymes (EC No. 3.4.21) having an active site serine that initiates hydrolysis of peptide bonds of proteins. Serine proteases comprise a diverse class of enzymes having a wide range of specificities and biological functions that are further divided based on their structure into chymotrypsin-like (trypsin-like) and subtilisin-like. The prototypical subtilisin (EC No. 3.4.21.62) was initially obtained from Bacillus subtilis. Subtilisins and their homologues are members of the S8 peptidase family of the MEROPS classification scheme (Rawlings, N.D. et al (2016) Twenty years of the MEROPS database of proteolytic enzymes, their substrates and inhibitors. Nucleic Acids Res 44, D343-D350). Members of family S8 have a catalytic triad in the order Asp, His and Ser in their amino acid sequence. Although a number of useful variant proteases have been developed for cleaning applications, there remains a need for cleaning compositions providing good levels of proteolytic cleaning even with increased use of low wash temperatures (e.g., cold water) and shorter washing cycles which tend to reduce stain/soil removal efficacy of detergent compositions.

Thus, it is an object of the present invention to provide a cleaning composition, particularly for laundry, comprising one or more protease enzymes, which can be used in a washing process to provide good cleaning, soil/stain removal even at low temperatures and short wash times.

### SUMMARY OF THE INVENTION

The present invention provides a cleaning composition, particularly for laundry, comprising (a) a subtilisin variant comprising two or more substitutions selected from the group consisting of X006W, X024K, X055P, X109Q, X162Q, X182Q, X183N, X204Q, X206Y, X222Q, X248A, or X254A, where the positions are numbered by correspondence with the amino acid sequence of SEQ ID NO: 1, wherein the variant has at least 75% identity to the amino acid sequence of SEQ ID NO: 1; and (b) a cleaning adjunct.

The invention also provides a method of treating a surface, the method comprising contacting the surface with an aqueous wash liquor comprising a subtilisin variant comprising two or more substitutions selected from the group consisting of X006W, X024K, X055P, X109Q, X162Q, X182Q, X183N, X204Q, X206Y, X222Q, X248A, or X254A, where the positions are numbered by correspondence with the amino acid sequence of SEQ ID NO: 1, wherein the variant has at least 75% identity to the amino acid sequence of SEQ ID NO: 1; and a cleaning adjunct. Preferably the surface is a fabric surface.

Preferably the subtilisin variant comprises two substitutions selected from the group consisting of X006W, X024K, X055P, X109Q, X162Q, X182Q, X183N, X204Q, X206Y, X222Q, X248A, or X254A, and further comprises one, two or more additional substitutions from the group consisting of X003Q, X022Y, X024Q, X033T, X045V, X053G, X076D, X078N, X087D, X101N, X118R, X128A, X128S, X145R, X166Q, X169A, X217Q, and X218S where the positions are numbered by correspondence with the amino acid sequence of SEQ ID NO:1, wherein the variant has at least 75% identity to the amino acid sequence of SEQ ID NO: 1.

The subtilisin variant herein demonstrates one or more improved properties, such as an improved stability, or both an improved cleaning performance and an improved stability when compared to a subtilisin having the amino acid sequence of SEQ ID NO: 1.

The present invention also provides a cleaning composition comprising one or more subtilisin variant which may be one or more isolated, recombinant, substantially pure, or non-naturally occurring subtilisin variant. Preferably, the subtilisin variant comprises two, three, four, or more amino acid substitutions at a position selected from the group consisting of 6, 24, 55, 109, 162, 182, 183, 204, 206, 222, 248, and 254 where the positions are numbered according to SEQ ID NO: 1, and where the variant has at least 75% identity to the amino acid sequence of SEQ ID NO: 1.

Preferably the parent subtilisin comprises an amino acid sequence of SEQ ID NO:1, or the parent subtilisin may comprise a polypeptide having the amino acid sequence of SEQ ID NO:1. The cleaning composition preferably comprises an improved property which is improved stability, wherein the subtilisin variant has a greater residual activity compared to the parent or reference subtilisin. The improved stability may be measured in accordance with the stability assays of Example 2. In still yet another embodiment the improved property is cleaning performance, where the variant has a greater performance index compared to the parent reference subtilisin. In still yet another embodiment the improved property is cleaning performance is measured in accordance with the stability assays of Example 2.

The composition of the invention comprises one or more subtilisin variant described herein preferably having one or more improved property when compared to a reference or parent subtilisin; wherein the improved property is selected from improved cleaning performance in detergents, improved stability; and combinations thereof.

The term "enhanced stability" or "improved stability" in the context of an oxidation, chelator, denaturant, surfactant, thermal and/or pH stable protease refers to a higher retained proteolytic activity of a subtilisin variant over time as compared to a reference or parent subtilisin protease, for example, a wild-type protease or parent protease, such as SEQ ID NO: 1. Autolysis has been identified as one mode of subtilisin activity loss in liquid detergents. (Stoner et al., 2004 Protease autolysis in heavy-duty liquid detergent formulations: effects of thermodynamic stabilizers and protease inhibitors, Enzyme and Microbial Technology 34:114-125.).

### BRIEF DESCRIPTION OF THE DRAWINGS

FIG. 1 is a box plot depicting the cumulative contribution to improve stability of substitutions in BPN' backbone as a plot of the number of mutations versus the percent (%) residual activity for variants described in Table 6, tested in Detergent C at 51°C;
FIG.2 is a box plot depicting the cumulative contribution to improve stability of substitutions in BPN' backbone as a plot of the number of mutations versus the percent (%) residual activity for variants described in Table 6, tested in Detergent B at 53°C; and
FIG.3 is a box plot depicting the cumulative contribution to improve stability of substitutions in BPN' backbone as a plot of the number of mutations versus the percent (%) residual activity for variants described in Table 6, tested in PNB Detergent at 40°C.

### DETAILED DESCRIPTION OF THE INVENTION

### Definitions

Unless otherwise indicated herein, one or more subtilisin variants described herein can be made and used by a variety of techniques used in molecular biology, microbiology, protein purification, protein engineering, protein and DNA sequencing, recombinant DNA fields, and industrial enzyme use and development. Unless otherwise indicated, nucleic acid sequences are written left to right in 5' to 3' orientation; and amino acid sequences are written left to right in amino to carboxy orientation. Each numerical range used herein includes every narrower numerical range that falls within such broader numerical range, as if such narrower numerical ranges were all expressly written herein.

The nomenclature of the amino acid substitutions of the one or more subtilisin variants described herein uses one or more of the following: position; position:amino acid substitution(s); or starting amino acid(s):position:amino acid substitution(s). Reference to a "position" (e.g. 5, 8, 17, 22, etc.) encompasses any starting amino acid that may be present at such position, and any substitution that may be present at such position. Reference to a "position: amino acid substitution(s)" (e.g. 1S/T/G, 3G, 17T, etc.) encompasses any starting amino acid that may be present at such position and the one or more amino acid(s) with which such starting amino acid may be substituted. Reference to a position can be recited in several forms, for example, position 003 can also be referred to as position 03 or 3. Reference to a starting or substituted amino acid may be further expressed as several starting, or substituted amino acids separated by a foreslash ("/"). For example, D275S/K indicates position 275 is substituted with serine (S) or lysine (K) and P/S197K indicates that starting amino acid proline (P) or serine (S) at position 197 is substituted with lysine (K). Reference to an X as the amino acid in a position, refers to any amino acid at the recited position.

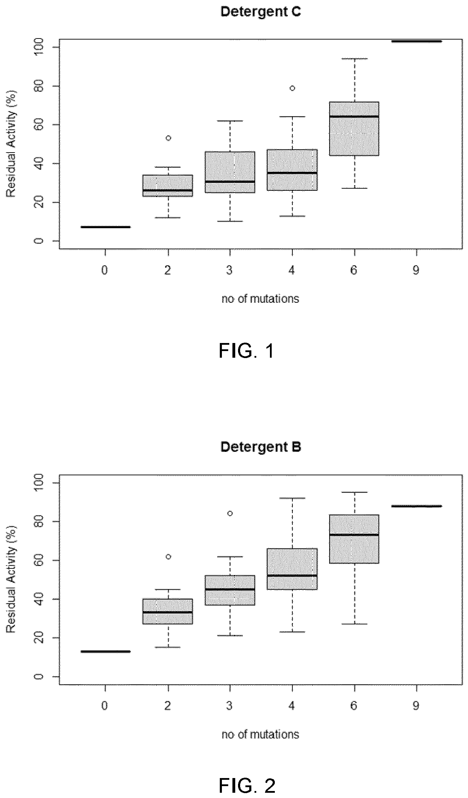

The position of an amino acid residue in a given amino acid sequence is numbered by correspondence with the amino acid sequence of SEQ ID NO:1. That is, the amino acid sequence of SEQ ID NO:1 serves as a reference sequence for numbering of positions of an amino acid residue. For example, the amino acid sequence of one or more subtilisin variant described herein is aligned with the amino acid sequence of SEQ ID NO:1 using an alignment algorithm as described herein, and each amino acid residue in the given amino acid sequence that aligns (preferably optimally aligns) with an amino acid residue in SEQ ID NO:1 is conveniently numbered by reference to the numerical position of that corresponding amino acid residue. Sequence alignment algorithms, such as, for example, described herein will identify the location or locations where insertions or deletions occur in a subject sequence when compared to a query sequence (also sometimes referred to as a "reference sequence"). Sequence alignment with other subtilisin amino acid sequences can be determined using an amino acid alignment, for example, as provided in Figure 1 of PCT Publication No. WO2018118917.

The terms "protease" and "proteinase" refer to an enzyme that has the ability to break down proteins and peptides. A protease has the ability to conduct "proteolysis," by hydrolysis of peptide bonds that link amino acids together in a peptide or polypeptide chain forming the protein. This activity of a protease as a protein-digesting enzyme is referred to as "proteolytic activity." Many well-known procedures exist for measuring proteolytic activity. For example, proteolytic activity may be ascertained by comparative assays that analyze the respective protease's ability to hydrolyze a suitable substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and Keratin Azure (Sigma-Aldrich K8500). Colorimetric assays utilizing these substrates are well known in the art (*See e.g.,* WO99/34011 and US 6,376,450). The pNA peptidyl assay (See e.g., Del Mar et al., Anal Biochem, 99:316-320, 1979) also finds use in determining the active enzyme concentration. This assay measures the rate at which p-nitroaniline is released as the enzyme hydrolyzes a soluble synthetic substrate, such as succinyl-alanine-alanine-proline-phenylalanine-p-nitroanilide (suc-AAPF-pNA). The rate of production of yellow color from the hydrolysis reaction is measured at 405 or 410 nm on a spectrophotometer and is proportional to the active enzyme concentration. In addition, absorbance measurements at 280 nanometers (nm) can be used to determine the total protein concentration in a sample of purified protein. The activity on substrate divided by protein concentration gives the enzyme specific activity.

As used herein, "the genus Bacillus" includes all species within the genus "Bacillus," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. gibsonii, B. sp TY145, B. patagonienis, B. pumilus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus",* or *B. polymyxa,* which is now *"Paenibacillus polymyxa".* The production of resistant endospores under stressful environmental conditions is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus, Alkalihalobacillus, Peribacillus, Cytobacillus, Mesobacillus, Neobacillus, Metabacillus, Virgibacillus, as well as the more recently proposed genera: Alteribacter, Ectobacillus, Evansella, Ferdinandcohnia, Gottfriedia, Heyndrickxia, Lederbergia, Litchfieldia, Margalitia, Niallia, Priestia, Robertmurraya, Rossellomorea, Schinkia, Siminovitchia, Sutcliffiella and Weizmannia.*

A *"Bacillus clade* subtilisin" includes any subtilisin obtained from, or derived from, a *Subtilis clade* source (genus *Bacillus sensu stricto),* described in Gupta RS , Patel S, Saini N, Chen S (2020) Robust demarcation of 17 distinct Bacillus species clades, proposed as novel Bacillaceae genera, by phylogenomics and comparative genomic analyses: description of Robertmurraya kyonggiensis sp. nov. and proposal for an emended genus Bacillus limiting it only to the members of the Subtilis and Cereus clades of species. Int J Syst Evol Microbiol 70:5753-5798, including *Bacillus amyloliquefaciens,* (BPN', WP_013351733.1,CAA24990.1).

The present invention provides a cleaning composition comprising "BPN' variants" (or "CAA24990.1 variants" or "WP_013351733.1 variants" or "BPN' subtilisin variants") wherein the mutations are present in the mature BPN' amino acid sequence set forth in SEQ ID NO: 1, and/or the BPN' subtilisins and variants thereof include those polypeptides having an amino acid sequence having at least 75% sequence identity to SEQ ID NO: 1. The protease is preferably a serine protease. The protease is preferably a subtilisin. The protease is preferably a *Bacillus clade* subtilisin. Preferably, the subtilisin is from a *Bacillus sp.* In some embodiments, the protease is from *B. amyloliquefaciens* (BPN'). In some embodiments, the protease is from a *B. xiamenensis.* In some embodiments, the protease is from *B. subtilis.* Preferably, the variant comprises an amino acid sequence with at least 75% or at least 80% or at least 85% or at least 90% sequence identity to a sequence selected from the group consisting of SEQ ID NO: 7, 8, or 9.

The term "vector" refers to a nucleic acid construct used to introduce or transfer nucleic acid(s) into a target cell or tissue. A vector is typically used to introduce foreign DNA into a cell or tissue. Vectors include plasmids, cloning vectors, bacteriophages, viruses (e.g., viral vector), cosmids, expression vectors, shuttle vectors, and the like. A vector typically includes an origin of replication, a multicloning site, and a selectable marker. The process of inserting a vector into a target cell is typically referred to as transformation. The present disclosure includes, a vector that comprises a DNA sequence encoding a serine protease polypeptide (e.g., precursor or mature serine protease polypeptide) that is operably linked to a suitable prosequence (e.g., secretory, signal peptide sequence, etc.) capable of effecting the expression of the DNA sequence in a suitable host, and the folding and translocation of the recombinant polypeptide chain.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction. Transformation refers to the genetic alteration of a cell which results from the uptake, optional genomic incorporation, and expression of genetic material (e.g., DNA).

The term "expression" refers to the transcription and stable accumulation of sense (mRNA) or anti-sense RNA, derived from a nucleic acid molecule of the disclosure. Expression may also refer to translation of mRNA into a polypeptide. Thus, the term "expression" includes any step involved in the "production of the polypeptide" including, but not limited to, transcription, post-transcriptional modifications, translation, post-translational modifications, secretion and the like.

The phrases "expression cassette" or "expression vector" refers to a nucleic acid construct or vector generated recombinantly or synthetically for the expression of a nucleic acid of interest (e.g., a foreign nucleic acid or transgene) in a target cell. The nucleic acid of interest typically expresses a protein of interest. An expression vector or expression cassette typically comprises a promoter nucleotide sequence that drives or promotes expression of the foreign nucleic acid. The expression vector or cassette also typically includes other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Some expression vectors have the ability to incorporate and express heterologous DNA fragments in a host cell or genome of the host cell. Many prokaryotic and eukaryotic expression vectors are commercially available. Selection of appropriate expression vectors for expression of a protein from a nucleic acid sequence incorporated into the expression vector is within the knowledge of those of skill in the art.

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

The term "gene" refers to a polynucleotide (e.g., a DNA segment), that encodes a polypeptide and includes regions preceding and following the coding regions. In some instances, a gene includes intervening sequences (introns) between individual coding segments (exons).

The term "recombinant", when used with reference to a cell typically indicates that the cell has been modified by the introduction of a foreign nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) that has been modified and reintroduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA in vitro and transfer of the recombinant DNA into cells where it may be expressed or propagated, thereby producing a recombinant polypeptide. "Recombination" and "recombining" of polynucleotides or nucleic acids refer generally to the assembly or combining of two or more nucleic acid or polynucleotide strands or fragments to generate a new polynucleotide or nucleic acid.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

The terms "host strain" and "host cell" refer to a suitable host for an expression vector comprising a DNA sequence of interest.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code.

The terms "prosequence" or "propeptide sequence" refer to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Bacterial serine proteases are often expressed as pro-enzymes. Examples of modified propeptides are provided, for example, in WO 2016/205710.

The terms "signal sequence" and "signal peptide" refer to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type", with respect to a polypeptide, refers to a naturally-occurring polypeptide that does not include a man-made substitution, insertion, or deletion at one or more amino acid positions. Similarly, the term "wild-type", with respect to a polynucleotide, refers to a naturally-occurring polynucleotide that does not include a man-made substitution, insertion, or deletion at one or more nucleotides. A polynucleotide encoding a wildtype polypeptide is, however, not limited to a naturally-occurring polynucleotide, and encompasses any polynucleotide encoding the wildtype or parental polypeptide.

The term "parent", with respect to a polypeptide, includes reference to a naturally-occurring, or wildtype, polypeptide or to a naturally-occurring polypeptide in which a man-made substitution, insertion, or deletion at one or more amino acid positions has been made that serves as the basis for introducing substitutions or additional substitutions to produce the variant enzymes provided herein. The term "parent" with respect to a polypeptide also includes any polypeptide that has protease activity that serves as the starting polypeptide for alteration, such as substitutions, additions, and/or deletions, to result in a variant having one or more alterations in comparison to the starting polypeptide. That is, a parental, or reference polypeptide is not limited to a naturally-occurring wildtype polypeptide, and encompasses any wildtype, parental, or reference polypeptide. Similarly, the term "parent," with respect to a polynucleotide, can refer to a naturally-occurring polynucleotide or to a polynucleotide that does include a man-made substitution, insertion, or deletion at one or more nucleotides. The term "parent" with respect to a polynucleotide also includes any polynucleotide that encodes a polypeptide having protease activity that serves as the starting polynucleotide for alteration to result in a variant protease having a modification, such as substitutions, additions, and/or deletions, in comparison to the starting polynucleotide. That is, a polynucleotide encoding a wildtype, parental, or reference polypeptide is not limited to a naturally-occurring polynucleotide, and encompasses any polynucleotide encoding the wildtype, parental, or reference polypeptide. For example, the the parent polypeptide herein, may comprise a polypeptide having the amino acid sequence set forth in SEQ ID NO: 1.

The term "naturally-occurring" refers to, for example, a sequence and residues contained therein (e.g., polypeptide sequence and amino acids contained therein or nucleotide sequence and nucleotides contained therein) that are found in nature. Conversely, the term "non-naturally occurring" refers to, for example, a sequence and residues contained therein (e.g., polypeptide sequences and amino acids contained therein or nucleotide sequence and nucleic acids contained therein) that are not found in nature.

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related protein or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question. To exemplify, "proteases derived from Bacillus" refers to those enzymes having proteolytic activity that are naturally produced by Bacillus, as well as to serine proteases like those produced by Bacillus sources but which through the use of genetic engineering techniques are produced by other host cells transformed with a nucleic acid encoding the serine proteases.

The term "identical" in the context of two polynucleotide or polypeptide sequences refers to the nucleotides or amino acids in the two sequences that are the same when aligned for maximum correspondence, as measured using sequence comparison or analysis algorithms described below and known in the art.

The phrases "% identity" or "percent identity" or "PID" refers to protein sequence identity. Percent identity may be determined using standard techniques known in the art. The percent amino acid identity shared by sequences of interest can be determined by aligning the sequences to directly compare the sequence information, e.g., by using a program such as BLAST, MUSCLE, or CLUSTAL. The BLAST algorithm is described, for example, in Altschul et al., J Mol Biol, 215:403-410 (1990) and Karlin et al., Proc Natl Acad Sci USA, 90:5873-5787 (1993). A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.

As used herein, "homologous proteins" or "homologous proteases" refers to proteins that have distinct similarity in primary, secondary, and/or tertiary structure. Protein homology can refer to the similarity in linear amino acid sequence when proteins are aligned. Homology can be determined by amino acid sequence alignment, e.g., using a program such as BLAST, MUSCLE, or CLUSTAL. Homologous search of protein sequences can be done using BLASTP and PSI-BLAST from NCBI BLAST with threshold (E-value cut-off) at 0.001. (Altschul et al., "Gapped BLAST and PSI BLAST a new generation of protein database search programs", Nucleic Acids Res, Set 1;25(17):3389-402(1997)). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997 and Schaffer et al., Nucleic Acids Res, 29:2994-3005, 2001). Exemplary default BLAST parameters for a nucleic acid sequence searches include: Neighboring words threshold=11; E-value cutoff=10; Scoring Matrix=NUC.3.1 (match=1, mismatch=-3);Gap Opening=5; and Gap Extension=2. Exemplary default BLAST parameters for amino acid sequence searches include: Word size = 3; E-value cutoff=10; Scoring Matrix=BLOSUM62; Gap Openings 1; and Gap extension=1. Using this information, protein sequences can be grouped and/or a phylogenetic tree built therefrom. Amino acid sequences can be entered in a program such as the Vector NTI Advance suite and a Guide Tree can be created using the Neighbor Joining (NJ) method (Saitou and Nei, Mol Biol Evol, 4:406-425, 1987). The tree construction can be calculated using Kimura's correction for sequence distance and ignoring positions with gaps. A program such as AlignX can display the calculated distance values in parenthesis following the molecule name displayed on the phylogenetic tree. Another useful algorithm for alignment and comparison of multiple protein sequences is the MUSCLE program (Robert C. Edgar. MUSCLE: multiple sequence alignment with high accuracy and high throughput Nucl. Acids Res. (2004) 32 (5): 1792-1797) available from Geneious software (Biomatters Ltd.).

Understanding the homology between molecules can reveal the evolutionary history of the molecules as well as information about their function; if a newly sequenced protein is homologous to an already characterized protein, there is a strong indication of the new protein's biochemical function. Two molecules are said to be homologous if they have been derived from a common ancestor. Homologous molecules, or homologs, can be divided into two classes, paralogs and orthologs. Paralogs are homologs that are present within one species. Paralogs often differ in their detailed biochemical functions. Orthologs are homologs that are present within different species and have very similar or identical functions. A protein superfamily is the largest grouping (clade) of proteins for which common ancestry can be inferred. Usually this common ancestry is based on sequence alignment and mechanistic similarity. Superfamilies typically contain several protein families which show sequence similarity within the family. The term "protein clan" is commonly used for protease superfamilies based on the MEROPS protease classification system. As used herein, the term "subtilisin" includes any member of the S8 serine protease family as described in MEROPS - The Peptidase Data base (Rawlings, N.D., et al (2016) Twenty years of the MEROPS database of proteolytic enzymes, their substrates and inhibitors. Nucleic Acids Res 44, D343-D350).

The CLUSTAL W algorithm is another example of a sequence alignment algorithm (See, Thompson et al., Nucleic Acids Res, 22:4673-4680, 1994). Default parameters for the CLUSTAL W algorithm include: Gap opening penalty=10.0; Gap extension penalty=0.05; Protein weight matrix=BLOSUM series; DNA weight matrix=IUB; Delay divergent sequences %=40; Gap separation distance=8; DNA transitions weight=0.50; List hydrophilic residues=GPSNDQEKR; Use negative matrix=OFF; Toggle Residue specific penalties=ON; Toggle hydrophilic penalties=ON; and Toggle end gap separation penalty=OFF. In CLUSTAL algorithms, deletions occurring at either terminus are included. For example, a variant with a five amino acid deletion at either terminus (or within the polypeptide) of a polypeptide of 500 amino acids would have a percent sequence identity of 99% (495/500 identical residues × 100) relative to the "reference" polypeptide. Such a variant would be encompassed by a variant having "at least 99% sequence identity" to the polypeptide.

A nucleic acid or polynucleotide is "isolated" when it is at least partially or completely separated from other components, including but not limited to, for example, other proteins, nucleic acids, cells, etc. Similarly, a polypeptide, protein or peptide is "isolated" when it is at least partially or completely separated from other components, including but not limited to, for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than in a starting composition.

The term "cleaning activity" refers to a cleaning performance achieved by a serine protease polypeptide, variant, or reference subtilisin under conditions prevailing during the proteolytic, hydrolyzing, cleaning, or other process of the disclosure. In some embodiments, cleaning performance of a serine protease or reference subtilisin may be determined by using various assays for cleaning one or more enzyme sensitive stain on an item or surface (e.g., a stain resulting from food, grass, blood, ink, milk, oil, and/or egg protein). Cleaning performance of one or more subtilisin variant described herein or reference subtilisin can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO99/34011 and US 6,605,458, as well as those cleaning assays and methods included in the Examples provided below.

The term "effective amount" of one or more subtilisin variants described herein or reference subtilisin refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (e.g., granular, tablet, bar) composition is required, etc.

The term "wash performance" of a protease (e.g., one or more subtilisin variant described herein, or recombinant polypeptide or active fragment thereof) refers to the contribution of one or more subtilisin variant described herein to washing that provides additional cleaning performance to the detergent as compared to the detergent without the addition of the one or more subtilisin variant described herein to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (e.g., hand or manual dishwashing, automatic dishwashing, dishware cleaning, tableware cleaning, fabric cleaning, etc.) are imitated.

The phrase "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

The term "dish wash" refers to both household and industrial dish washing and relates to both automatic dish washing (e.g. in a dishwashing machine) and manual dishwashing (e.g. by hand).

The term "variant" means a polypeptide having enzyme activity comprising an alteration/mutation, i.e., a substitution, insertion, and/or deletion, at one or more (e.g. several) positions relative to the parent. A substitution means a replacement of an amino acid occupying a position with a different amino acid; a deletion means removal of an amino acid occupying a position; and an insertion means adding 1-3 amino acids adjacent to and immediately following an amino acid occupying a position.

The term "wild-type" enzyme means an enzyme expressed by a naturally occurring microorganism, such as a bacterium, algae, yeast, or filamentous fungus found in nature.

### Cleaning Composition

The detergent/cleaning composition of the present invention is a cleaning composition: for cleaning hard surfaces, such as for glass, wood, ceramic and metal counter tops, windows, ovens; or for dishwashing, including hand or manual dishwashing compositions (e.g., "hand" or "manual" dishwashing detergents) and automatic dishwashing compositions (e.g., "automatic dishwashing detergents"); or for cleaning fabrics, such as fabric fresheners; fabric softeners; and textile, laundry booster cleaning or detergent compositions, laundry additive cleaning compositions, and laundry pre-spotter cleaning compositions or any other cleaning for consumer or institutional use and for handwashing or machine washing (automatic dish or laundry washing). The cleaning composition of the present invention does not include compositions for cleaning contact lenses, or ultrafiltration membranes, or in healing wounds or for medical treatment of skin conditions.

Preferably the composition is a laundry detergent composition or a dishwashing detergent composition, most preferably a laundry detergent, most preferably an automatic laundry detergent. The composition of the invention may be a solid, liquid and/or unit dose detergent composition, for example a solid and/or liquid encapsulated in a water-soluble pouch. Especially preferred is a liquid laundry detergent composition, optionally encapsulated in water-soluble material in the form of a pouch, optionally in the form of a multi-component pouch. The term liquid includes a gel, a solution, a dispersion, a paste, or a mixture thereof. The solid may be a powder, i.e. solid particulates, or may be a single homogenous solid, or compacted particles. Where liquid, the cleaning composition typically comprises less than 15%, or less than 12% by weight of the composition of water. The liquid cleaning composition may comprise a non-aqueous solvent selected from 1,2-propanediol, dipropylene glycol, tripropyleneglycol, glycerol, sorbitol, polyethylene glycol or a mixture thereof, for example from 10% to 40%, or between 15% and 30% by weight of the liquid laundry detergent composition of the non-aqueous solvent.

The cleaning composition comprises (a) a subtilisin variant comprising two or more substitutions selected from the group consisting of X006W, X024K, X055P, X109Q, X162Q, X182Q, X183N, X204Q, X206Y, X222Q, X248A, or X254A, wherein the variant has at least 75% identity to the amino acid sequence of SEQ ID NO: 1, wherein the positions are numbered by correspondence with the amino acid sequence of SEQ ID NO: 1 and (b) cleaning adjunct.

The cleaning composition of the present invention is preferably a laundry detergent. The composition may be in the form of a composition for use in a main wash step or as pre-treatment or rinse-added cleaning composition for consumer or institutional use.

### Subtilisin Variant

The cleaning composition comprising one or more subtilisin variants. Preferably the one or more subtilisin variant is present in the composition in an amount from 0.0001 to 15 mg active protein, more preferably from 0.0005 to 10 wt% active protein, or from 0.001 to 7 wt% active protein. The subtilisin variant comprises two, three, four, five, or more amino acid substitutions at a position selected from the group consisting of X006W, X024K, X055P, X109Q, X162Q, X182Q, X183N, X204Q, X206Y, X222Q, X248A, and X254A, where the positions are numbered according to SEQ ID NO: 1, and where the variant has at least 75% identity to the amino acid sequence of SEQ ID NO: 1.

Preferably, the subtilisin variant comprises a combination of substitutions selected from the group consisting of X006W-X024K, X006W-X055P, X006W-X109Q, X006W-X162Q, X006W-X183N, X006W-X182Q, X006W-X204Q, X006W-X206Y, X006W-X222Q, X006W-X248A, X006W-X254A, X024K-X055P, X024K-X109Q, X024K-X162Q, X024K-X183N, X024K-X182Q, X024K-X204Q, X024K-X206Y, X024K-X222Q, X024K-X248A, X024K-X254A, X055P-X109Q, X055P-X162Q, X055P-X183N, X055P-X182Q, X055P-X204Q, X055P-X206Y, X055P-X222Q, X055P-X248A, X055P-X254A, X109Q-X162Q, X109Q-X183N, X109Q-X182Q, X109Q-X204Q, X109Q-X206Y, X109Q-X222Q, X109Q-X248A, X109Q-X254A, X162Q-X183N, X162Q-X182Q, X162Q-X204Q, X162Q-X206Y, X162Q-X222Q, X162Q-X248A, X162Q-X254A, X183N-X182Q, X183N-X204Q, X183N-X206Y, X183N-X222Q, X183N-X248A, X183N-X254A, X182Q-X204Q, X182Q-X206Y, X182Q-X222Q, X182Q-X248A, X182Q-X254A, X204Q-X206Y, X204Q-X222Q, X204Q-X248A, X204Q-X254A, X206Y-X222Q, X206Y-X248A, X206Y-X254A, X222Q-X248A, X222Q-X254A, X248A-X254A, where the positions are numbered by correspondence with the amino acid sequence of SEQ ID NO:1, and where the variant has at least 75% identity to the amino acid sequence of SEQ ID NO: 1.

The variant preferably comprises a combination of substitutions selected from the group consisting of Y006W-S024K, Y006W-T055P, Y006W-N109Q, Y006W-S162Q, Y006W-S183N, Y006W-S182Q, Y006W-S204Q, Y006W-Q206Y, Y006W-M222Q, Y006W-S248A, Y006W-T254A, S024K-T055P, S024K-N109Q, S024K-S162Q, S024K-SI83N, S024K-S182Q, S024K-S204Q, S024K-Q206Y, S024K-M222Q, S024K-S248A, S024K-T254A, T055P-N109Q, T055P-S162Q, T055P-S183N, T055P-S182Q, T055P-S204Q, T055P-Q206Y, T055P-M222Q, T055P-S248A, T055P-T254A, N109Q-S162Q, N109Q-S183N, N109Q-S182Q, N109Q-S204Q, N109Q-Q206Y, N109Q-M222Q, N109Q-S248A, N109Q-T254A, S162Q-S183N, S162Q-S182Q, S162Q-S204Q, S162Q-Q206Y, S162Q-M222Q, S162Q-S248A, S162Q-T254A, S183N-S182Q, S183N-S204Q, S183N-Q206Y, S183N-M222Q, S183N-S248A, S183N-T254A, S182Q-S204Q, S182Q-Q206Y, S182Q-M222Q, S182Q-S248A, S182Q-T254A, S204Q-Q206Y, S204Q-M222Q, S204Q-S248A, S204Q-T254A, Q206Y-M222Q, Q206Y-S248A, Q206Y-T254A, M222Q-S248A, M222Q-T254A, S248A-T254A, where the positions are numbered by correspondence with the amino acid sequence of SEQ ID NO:1, and the variant subtilisin comprises an amino acid sequence having at least 75%, or at least 80%, or at least 81%, or at least 85%, or at least 90%, or at least 91%, or at least 92%, or at least 93%, or at least 94%, or at least 95%, or at least 96%, or at least 97%, or at least 98% amino acid sequence identity to the amino acid sequence of SEQ ID NO: 1.

In some embodiments, the combinations of substitutions include those selected from the group consisting of X006W-X024K-X055P, X006W-X024K-X109Q, X006W-X024K-X162Q, X006W-X024K-X183N, X006W-X024K-X182Q, X006W-X024K-X204Q, X006W-X024K-X206Y, X006W-X024K-X222Q, X006W-X024K-X248A, X006W-X024K-X254A, X006W-X055P-X109Q, X006W-X055P-X162Q, X006W-X055P-X183N, X006W-X055P-X182Q, X006W-X055P-X204Q, X006W-X055P-X206Y, X006W-X055P-X222Q, X006W-X055P-X248A, X006W-X055P-X254A, X006W-X109Q-X162Q, X006W-X109Q-X183N, X006W-X109Q-X182Q, X006W-X109Q-X204Q, X006W-X109Q-X206Y, X006W-X109Q-X222Q, X006W-X109Q-X248A, X006W-X109Q-X254A, X006W-X162Q-X183N, X006W-X162Q-X182Q, X006W-X162Q-X204Q, X006W-X162Q-X206Y, X006W-X162Q-X222Q, X006W-X162Q-X248A, X006W-X162Q-X254A, X006W-X183N-X182Q, X006W-X183N-X204Q, X006W-X183N-X206Y, X006W-X183N-X222Q, X006W-X183N-X248A, X006W-X183N-X254A, X006W-X182Q-X204Q, X006W-X182Q-X206Y, X006W-X182Q-X222Q, X006W-X182Q-X248A, X006W-X182Q-X254A, X006W-X204Q-X206Y, X006W-X204Q-X222Q, X006W-X204Q-X248A, X006W-X204Q-X254A, X006W-X206Y-X222Q, X006W-X206Y-X248A, X006W-X206Y-X254A, X006W-X222Q-X248A, X006W-X222Q-X254A, X006W-X248A-X254A, X024K-X055P-X109Q, X024K-X055P-X162Q, X024K-X055P-X183N, X024K-X055P-X182Q, X024K-X055P-X204Q, X024K-X055P-X206Y, X024K-X055P-X222Q, X024K-X055P-X248A, X024K-X055P-X254A, X024K-X109Q-X162Q, X024K-X109Q-X183N, X024K-X109Q-X182Q, X024K-X109Q-X204Q, X024K-X109Q-X206Y, X024K-X109Q-X222Q, X024K-X109Q-X248A, X024K-X109Q-X254A, X024K-X162Q-X183N, X024K-X162Q-X182Q, X024K-X162Q-X204Q, X024K-X162Q-X206Y, X024K-X162Q-X222Q, X024K-X162Q-X248A, X024K-X162Q-X254A, X024K-X183N-X182Q, X024K-X183N-X204Q, X024K-X183N-X206Y, X024K-X183N-X222Q, X024K-X183N-X248A, X024K-X183N-X254A, X024K-X182Q-X204Q, X024K-X182Q-X206Y, X024K-X182Q-X222Q, X024K-X182Q-X248A, X024K-X182Q-X254A, X024K-X204Q-X206Y, X024K-X204Q-X222Q, X024K-X204Q-X248A, X024K-X204Q-X254A, X024K-X206Y-X222Q, X024K-X206Y-X248A, X024K-X206Y-X254A, X024K-X222Q-X248A, X024K-X222Q-X254A, X024K-X248A-X254A, X055P-X109Q-X162Q, X055P-X109Q-X183N, X055P-X109Q-X182Q, X055P-X109Q-X204Q, X055P-X109Q-X206Y, X055P-X109Q-X222Q, X055P-X109Q-X248A, X055P-X109Q-X254A, X055P-X162Q-X183N, X055P-X162Q-X182Q, X055P-X162Q-X204Q, X055P-X162Q-X206Y, X055P-X162Q-X222Q, X055P-X162Q-X248A, X055P-X162Q-X254A, X055P-X183N-X182Q, X055P-X183N-X204Q, X055P-X183N-X206Y, X055P-X183N-X222Q, X055P-X183N-X248A, X055P-X183N-X254A, X055P-X182Q-X204Q, X055P-X182Q-X206Y, X055P-X182Q-X222Q, X055P-X182Q-X248A, X055P-X182Q-X254A, X055P-X204Q-X206Y, X055P-X204Q-X222Q, X055P-X204Q-X248A, X055P-X204Q-X254A, X055P-X206Y-X222Q, X055P-X206Y-X248A, X055P-X206Y-X254A, X055P-X222Q-X248A, X055P-X222Q-X254A, X055P-X248A-X254A, X109Q-X162Q-X183N, X109Q-X162Q-X182Q, X109Q-X162Q-X204Q, X109Q-X162Q-X206Y, X109Q-X162Q-X222Q, X109Q-X162Q-X248A, X109Q-X162Q-X254A, X109Q-X183N-X182Q, X109Q-X183N-X204Q, X109Q-X183N-X206Y, X109Q-X183N-X222Q, X109Q-X183N-X248A, X109Q-X183N-X254A, X109Q-X182Q-X204Q, X109Q-X182Q-X206Y, X109Q-X182Q-X222Q, X109Q-X182Q-X248A, X109Q-X182Q-X254A, X109Q-X204Q-X206Y, X109Q-X204Q-X222Q, X109Q-X204Q-X248A, X109Q-X204Q-X254A, X109Q-X206Y-X222Q, X109Q-X206Y-X248A, X109Q-X206Y-X254A, X109Q-X222Q-X248A, X109Q-X222Q-X254A, X109Q-X248A-X254A, X162Q-X183N-X182Q, X162Q-X183N-X204Q, X162Q-X183N-X206Y, X162Q-X183N-X222Q, X162Q-X183N-X248A, X162Q-X183N-X254A, X162Q-X182Q-X204Q, X162Q-X182Q-X206Y, X162Q-X182Q-X222Q, X162Q-X182Q-X248A, X162Q-X182Q-X254A, X162Q-X204Q-X206Y, X162Q-X204Q-X222Q, X162Q-X204Q-X248A, X162Q-X204Q-X254A, X162Q-X206Y-X222Q, X162Q-X206Y-X248A, X162Q-X206Y-X254A, X162Q-X222Q-X248A, X162Q-X222Q-X254A, X162Q-X248A-X254A, X183N-X182Q-X204Q, X183N-X182Q-X206Y, X183N-X182Q-X222Q, X183N-X182Q-X248A, X183N-X182Q-X254A, X183N-X204Q-X206Y, X183N-X204Q-X222Q, X183N-X204Q-X248A, X183N-X204Q-X254A, X183N-X206Y-X222Q, X183N-X206Y-X248A, X183N-X206Y-X254A, X183N-X222Q-X248A, X183N-X222Q-X254A, X183N-X248A-X254A, X182Q-X204Q-X206Y, X182Q-X204Q-X222Q, X182Q-X204Q-X248A, X182Q-X204Q-X254A, X182Q-X206Y-X222Q, X182Q-X206Y-X248A, X182Q-X206Y-X254A, X182Q-X222Q-X248A, X182Q-X222Q-X254A, X182Q-X248A-X254A, X204Q-X206Y-X222Q, X204Q-X206Y-X248A, X204Q-X206Y-X254A, X204Q-X222Q-X248A, X204Q-X222Q-X254A, X204Q-X248A-X254A, X206Y-X222Q-X248A, X206Y-X222Q-X254A, X206Y-X248A-X254A, and X222Q-X248A-X254A, where the positions are numbered by correspondence with the amino acid sequence of SEQ ID NO: 1, and the variant subtilisin comprises an amino acid sequence having at least 75%, 80%, 85%, 90%, 91%, 92%, 93%, 94%, 95%, 96%, 97%, or 98% amino acid sequence identity to the amino acid sequence of SEQ ID NO: 1.

In some embodiments, the combinations of substitutions include those selected from the group consisting of Y006W-S024K-T055P, Y006W-S024K-N109Q, Y006W-S024K-S162Q, Y006W-S024K-S183N, Y006W-S024K-S182Q, Y006W-S024K-S204Q, Y006W-S024K-Q206Y, Y006W-S024K-M222Q, Y006W-S024K-S248A, Y006W-S024K-T254A, Y006W-T055P-N109Q, Y006W-T055P-S162Q, Y006W-T055P-S183N, Y006W-T055P-S182Q, Y006W-T055P-S204Q, Y006W-T055P-Q206Y, Y006W-T055P-M222Q, Y006W-T055P-S248A, Y006W-T055P-T254A, Y006W-N109Q-S162Q, Y006W-N109Q-S183N, Y006W-N109Q-S182Q, Y006W-N109Q-S204Q, Y006W-N109Q-Q206Y, Y006W-N109Q-M222Q, Y006W-N109Q-S248A, Y006W-N109Q-T254A, Y006W-S162Q-S183N, Y006W-S162Q-S182Q, Y006W-S162Q-S204Q, Y006W-S162Q-Q206Y, Y006W-S162Q-M222Q, Y006W-S162Q-S248A, Y006W-S162Q-T254A, Y006W-S183N-S182Q, Y006W-S183N-S204Q, Y006W-S183N-Q206Y, Y006W-S183N-M222Q, Y006W-S183N-S248A, Y006W-S183N-T254A, Y006W-S182Q-S204Q, Y006W -S 1 82Q-Q206Y, Y006W-S182Q-M222Q, Y006W-S182Q-S248A, Y006W-S182Q-T254A, Y006W-S204Q-Q206Y, Y006W-S204Q-M222Q, Y006W-S204Q-S248A, Y006W-S204Q-T254A, Y006W-Q206Y-M222Q, Y006W-Q206Y-S248A, Y006W-Q206Y-T254A, Y006W-M222Q-S248A, Y006W-M222Q-T254A, Y006W-S248A-T254A, S024K-T055P-N109Q, S024K-T055P-S162Q, S024K-T055P-S183N, S024K-T055P-S182Q, S024K-T055P-S204Q, S024K-T055P-Q206Y, S024K-T055P-M222Q, S024K-T055P-S248A, S024K-T055P-T254A, S024K-N109Q-S162Q, S024K-N109Q-S183N, S024K-N109Q-S182Q, S024K-N109Q-S204Q, S024K-N109Q-Q206Y, S024K-N109Q-M222Q, S024K-N109Q-S248A, S024K-N109Q-T254A, S024K-S162Q-S183N, S024K-S162Q-S182Q, S024K-S162Q-S204Q, S024K-S162Q-Q206Y, S024K-S162Q-M222Q, S024K-S162Q-S248A, S024K-S162Q-T254A, S024K-S183N-S182Q, S024K-S183N-S204Q, S024K-S183N-Q206Y, S024K-S183N-M222Q, S024K-S183N-S248A, S024K-S183N-T254A, S024K-S182Q-S204Q, S024K-S182Q-Q206Y, S024K-S182Q-M222Q, S024K-S182Q-S248A, S024K-S182Q-T254A, S024K-S204Q-Q206Y, S024K-S204Q-M222Q, S024K-S204Q-S248A, S024K-S204Q-T254A, S024K-Q206Y-M222Q, S024K-Q206Y-S248A, S024K-Q206Y-T254A, S024K-M222Q-S248A, S024K-M222Q-T254A, S024K-S248A-T254A, T055P-N109Q-S162Q, T055P-N109Q-S183N, T055P-N109Q-S182Q, T055P-N109Q-S204Q, T055P-N109Q-Q206Y, T055P-N109Q-M222Q, T055P-N109Q-S248A, T055P-N109Q-T254A, T055P-S162Q-S183N, T055P-S162Q-S182Q, T055P-S162Q-S204Q, T055P-S162Q-Q206Y, T055P-S162Q-M222Q, T055P-S162Q-S248A, T055P-S162Q-T254A, T055P-S183N-S182Q, T055P-S183N-S204Q, T055P-S183N-Q206Y, T055P-S183N-M222Q, T055P-S183N-S248A, T055P-S183N-T254A, T055P-S182Q-S204Q, T055P-S182Q-Q206Y, T055P-S182Q-M222Q, T055P-S182Q-S248A, T055P-S182Q-T254A, T055P-S204Q-Q206Y, T055P-S204Q-M222Q, T055P-S204Q-S248A, T055P-S204Q-T254A, T055P-Q206Y-M222Q, T055P-Q206Y-S248A, T055P-Q206Y-T254A, T055P-M222Q-S248A, T055P-M222Q-T254A, T055P-S248A-T254A, N109Q-S162Q-S183N, N109Q-S162Q-S182Q, N109Q-S162Q-S204Q, N109Q-S162Q-Q206Y, N109Q-S162Q-M222Q, N109Q-S162Q-S248A, N109Q-S162Q-T254A, N109Q-S183N-S182Q, N109Q-S183N-S204Q, N109Q-S183N-Q206Y, N109Q-S183N-M222Q, N109Q-S183N-S248A, N109Q-S183N-T254A, N109Q-S182Q-S204Q, N109Q-S182Q-Q206Y, N109Q-S182Q-M222Q, N109Q-S182Q-S248A, N109Q-S182Q-T254A, N109Q-S204Q-Q206Y, N109Q-S204Q-M222Q, N109Q-S204Q-S248A, N109Q-S204Q-T254A, N109Q-Q206Y-M222Q, N109Q-Q206Y-S248A, N109Q-Q206Y-T254A, N109Q-M222Q-S248A, N109Q-M222Q-T254A, N109Q-S248A-T254A, S162Q-S183N-S182Q, S162Q-S183N-S204Q, S162Q-S183N-Q206Y, S162Q-S183N-M222Q, S162Q-S183N-S248A, S162Q-S183N-T254A, S162Q-S182Q-S204Q, S162Q-S182Q-Q206Y, S162Q-S182Q-M222Q, S162Q-S182Q-S248A, S162Q-S182Q-T254A, S162Q-S204Q-Q206Y, S162Q-S204Q-M222Q, S162Q-S204Q-S248A, S162Q-S204Q-T254A, S162Q-Q206Y-M222Q, S162Q-Q206Y-S248A, S162Q-Q206Y-T254A, S162Q-M222Q-S248A, S162Q-M222Q-T254A, S162Q-S248A-T254A, S183N-S182Q-S204Q, S183N-S182Q-Q206Y, S183N-S182Q-M222Q, S183N-S182Q-S248A, S183N-S182Q-T254A, S183N-S204Q-Q206Y, S183N-S204Q-M222Q, S183N-S204Q-S248A, S183N-S204Q-T254A, S183N-Q206Y-M222Q, S183N-Q206Y-S248A, S183N-Q206Y-T254A, S183N-M222Q-S248A, S183N-M222Q-T254A, S183N-S248A-T254A, S182Q-S204Q-Q206Y, S182Q-S204Q-M222Q, S182Q-S204Q-S248A, S182Q-S204Q-T254A, S182Q-Q206Y-M222Q, S182Q-Q206Y-S248A, S182Q-Q206Y-T254A, S182Q-M222Q-S248A, S182Q-M222Q-T254A, S182Q-S248A-T254A, S204Q-Q206Y-M222Q, S204Q-Q206Y-S248A, S204Q-Q206Y-T254A, S204Q-M222Q-S248A, S204Q-M222Q-T254A, S204Q-S248A-T254A, Q206Y-M222Q-S248A, Q206Y-M222Q-T254A, Q206Y-S248A-T254A, and M222Q-S248A-T254A, where the positions are numbered by correspondence with the amino acid sequence of SEQ ID NO:1, and the variant subtilisin comprises an amino acid sequence having at least 75%, 80%, 85%, 90%, 91%, 92%, 93%, 94%, 95%, 96%, 97%, or 98% amino acid sequence identity to the amino acid sequence of SEQ ID NO: 1.

In another embodiment, the subtilisin variant further comprises two or more substitutions selected from the group consisting of X003Q, X006W, X022Y, X024K, X024Q, X033T, X045V, X053G, X055P, X076D, X078N, X087D, X101N, X109Q, X118R, X128A, X128S, X145R, X162Q, X166Q, X169A, X182Q, X183N, X204Q, X206Y, X217Q, X218S, X222Q, X248A, or X254A, wherein the variant has at least 75% identity to the amino acid sequence of SEQ ID NO: 1, wherein the positions are numbered by correspondence with the amino acid sequence of SEQ ID NO:1 (BPN'), and wherein the variant does not have 100% sequence identity to a naturally-occurring amino acid sequence.

In yet another embodiment, the subtilisin variant further comprises two or more substitutions selected from the group consisting of S003Q, Y006W, T022Y, S024K, S024Q, S033T, A045V, S053G, T055P, N076D, S078N, S087D, S101N, N109Q, N118R, G128A, G128S, S145R, S162Q, G166Q, G169A, S182Q, S183N, S204Q, Q206Y, Y217Q, N218S, M222Q, S248A, or T254A, wherein the variant has at least 75% identity to the amino acid sequence of SEQ ID NO: 1, wherein the positions are numbered by correspondence with the amino acid sequence of SEQ ID NO:1 (BPN'), and wherein the variant does not have 100% sequence identity to a naturally-occurring amino acid sequence.

Preferably the variant comprises a combination of substitutions selected from the group consisting of S003Q-S024Q-S033T-A045V-S053G-N076D-S078N-S101N-N109Q-N118R-G128S-S145R-S162Q-G169A-S183N-Y217Q-S248A-T254A, S003Q-S024Q-S033T-A045V-S053G-N076D-S078N-S101N-N109Q-N118R-G128S-S145R-S162Q-S183N-Y217Q-S248A-T254A, S003Q-Y006W-S024Q-S033TA045V-S053G-N076D-S078N-S101N-N109Q-N118R-G128S-S145R-S162Q-S183N-Y217Q-S248AT254A, S003Q-T022Y-S024Q-S033T-A045V-S053G-N076D-S078N-S101N-N109Q-N118R-G128S-S145R-S162Q-S183N-Y217Q-S248A-T254A, S003Q-S024Q-S033T-A045V-S053G-N076D-S078N-S101N-N109Q-N118R-G128S-S145R-S162Q-S182Q-S183N-Y217Q-S248A-T254A, S003Q-S024Q-S033T-A045V-S053G-N076D-S078N-S101N-N109Q-N118R-G128S-S145R-S162Q-S183N-Y217Q-N218S-S248A-T254A, S003Q-T022Y-S024Q-S033T-A045V-S053G-N076D-S078N-S101N-N109Q-N118R-G128S-S145R-S162Q-S183N-Y217Q-N218S-S248A-T254A, S003Q-Y006W-T022Y-S024Q-S033T-A045V-S053G-N076D-S078N-S101N-N109Q-N118R-G128S-S145R-S162Q-G169A-S183N-Y217Q-S248A-T254A, S003Q-Y006W-T022Y-S024Q-S033T-A045V-S053G-N076D-S078N-S101N-N109Q-N118R-G128S-S145R-S162Q-G166Q-S183N-Y217Q-S248A-T254A, S003Q-T022Y-S024Q-S033T-A045V-S053G-N076D-S078N-S101N-N109Q-N118R-G128S-S145R-S162Q-G166Q-S183N-Y217Q-N218S-S248A-T254A, S003Q-Y006W-T022Y-S024Q-S033T-A045V-S053G-N076D-S078N-S101N-N109Q-N118R-G128S-S145R-S162Q-S183N-Y217Q-N218S-S248A-T254A, S003Q-T022Y-S024Q-S033T-A045V-S053G-N076D-S078N-S101N-N109Q-N118R-G128S-S145R-S162Q-G166Q-S182Q-S183N-Y217Q-S248A-T254A, S003Q-T022Y-S024Q-S033T-A045V-S053G-N076D-S078N-S101N-N109Q-N118R-G128S-S145R-S162Q-G166Q-G169A-S183N-Y217Q-S248A-T254A, S003Q-Y006W-T022Y-S024Q-S033T-A045V-S053G-N076D-S078N-S101N-N109Q-N118R-G128S-S162Q-G169A-S182Q-S183N-Y217Q-N218S-S248A-T254A, S003Q-Y006W-T022Y-S024Q-S033T-A045V-S053G-N076D-S078N-S101N-N109Q-N118R-G128S-S145R-S162Q-S182Q-S183N-Y217Q-S248AT254A, S003Q-T022Y-S024Q-S033T-A045V-S053G-N076D-S078N-S101N-N109Q-N118R-G128S-S145R-S162Q-G169A-S182Q-S183N-Y217Q-N218S-S248A-T254A, S003Q-Y006W-T022Y-S024Q-S033T-A045V-S053G-N076D-S078N-S101N-N109Q-N118R-G128S-S145R-S162Q-G169A-S182Q-S183N-Y217Q-S248A-T254A, S003Q-Y006W-T022Y-S024Q-S033T-A045V-S053G-N076D-S078N-S101N-N109Q-N118R-G128S-S145R-S162Q-S182Q-S183N-Y217Q-N218S-S248A-T254A, S003Q-Y006W-T022Y-S024Q-S033T-A045V-S053G-N076D-S078N-S101N-N109Q-G128S-S145R-S162Q-G169A-S182Q-S183N-Y217Q-N218S-S248A-T254A, S003Q-T022Y-S024Q-S033T-A045V-S053G-N076D-S078N-S101N-N109Q-N118R-G128S-S145R-S162Q-G169A-S183N-Y217Q-N218S-S248AT254A, S003Q-Y006W-T022Y-S024Q-S033T-A045V-S053G-N076D-S078N-S101N-N109Q-N118R-G128S-S145R-S162Q-S183N-Y217Q-S248A-T254A, S003Q-Y006W-T022Y-S024Q-S033T-A045V-S053G-N076D-S078N-S101N-N109Q-N118R-G128S-S145R-S162Q-G169A-S183N-Y217Q-N218S-S248A-T254A, S003Q-T022Y-S024Q-S033T-A045V-S053G-N076D-S078N-S101N-N109Q-N118R-G128S-S145R-S162Q-S182Q-S183N-Y217Q-N218S-S248A-T254A, S003Q-T022Y-S024Q-S033TA045V-S053G-N076D-S078N-S101N-N109Q-N118R-G128S-S145R-S162Q-G169A-S182Q-S183N-Y217Q-S248A-T254A, S003Q-Y006W-T022Y-S024Q-S033T-A045V-S053G-N076D-S078N-S101N-N109Q-N118R-G128S-S145R-S162Q-G169A-S182Q-S183N-Y217Q-N218S-S248A-T254A, S003Q-Y006W-T022Y-S024Q-S033T-A045V-S053G-N076D-S078N-S101N-N109Q-N118R-G128S-S145R-S162Q-G166Q-G169A-S182Q-S183N-Y217Q-N218S-S248A-T254A, S003Q-T022Y-S024K-S033T-S053G-N076D-S078N-S101N-N118R-G128S-S182Q-S183N-Y217Q-M222Q-S248A-T254A, S003Q-S024K-S033T-A045V-S053G-N076D-S078N-S101N-N109Q-N118R-G128S-S182Q-S183N-Q206Y-Y217Q-M222Q-T254A, S003Q-S024Q-S033T-A045V-S053G-N076D-S078N-S101N-N109Q-G128AS145R-S162Q-S182Q-S183N-Y217Q-M222Q-S248A-T254A, S003Q-S024Q-S033T-A045V-S053G-N076D-S078N-S101N-N109Q-G128A-S145R-S162Q-S182Q-S183N-Y217Q-S248A-T254A, S003Q-Y006W-T022Y-S024Q-S033T-A045V-S053G-N076D-S078N-S101N-N109Q-G128S-G169A-S182Q-Y217Q-S248A, S003Q-T022Y-S024Q-S033T-A045V-S053G-N076D-S078N-S101N-N109Q-G128A-G169A-S182Q-S183N-S204Q-Q206Y-Y217Q-N218S-T254A, S003Q-T022Y-S024Q-S033T-S053G-N076D-S078N-S101N-N118R-G128S-S182Q-Y217Q-N218S-T254A, S003Q-Y006W-S024Q-S033T-S053G-N076D-S078N-S101N-G128S-G169A-S182Q-Y217Q-N218S-S248A-T254A, Y006W-S024Q-S033T-A045V-S053G-N076D-S078N-S101N-G128S-S145R-S162Q-S182Q-Y217Q-N218S-T254A, S003Q-T022Y-S024Q-S033T-S053G-N076D-S078N-S101N-N118R-G128S-S162Q-S182Q-S183N-Y217Q-T254A, S024Q-S033T-A045V-S053G-N076D-S078N-S101N-G128A-S145R-Y217Q-S248AT254A, S024Q-S033T-S053G-N076D-S078N-S101N-N109Q-G128S-S145R-S162Q-G169A-Y217Q-N218S, S003Q-T022Y-S024Q-S033T-S053G-N076D-S078N-S101N-N109Q-N118R-G128S-S162Q-G169A-S183N-Y217Q-N218S-T254A, S003Q-Y006W-S024Q-S033T-S053G-N076D-S078N-S101N-N109Q-G128S-S145R-G169A-S183N-Y217Q-N218S-S248A, Y006W-S024Q-S033T-A045V-S053G-N076D-S078N-S101N-G128S-S162Q-S183N-Y217Q-N218S-T254A, T022Y-S024Q-S033T-S053G-N076D-S078N-S101N-N118R-G128S-S145R-S162Q-S182Q-Y217Q-S248A-T254A, S003Q-S024Q-S033T-A045V-S053G-N076D-S078N-S101N-G128S-S145R-S162Q-S182Q-S183N-Y217Q-S248AT254A, S003Q-T022Y-S024Q-S033T-S053G-N076D-S078N-S101N-G128S-S145R-S162Q-S182Q-S183N-Y217Q-S248A-T254A, Y006W-T022Y-S024Q-S033T-S053G-N076D-S078N-S101N-G128S-S183N-Y217Q-S248A-T254A, S003Q-Y006W-T022Y-S024Q-S033T-S053G-S078N-S087D-S101N-N118R-G128S-S162Q-S182Q-Y217Q-T254A, S003Q-T022Y-S024K-S033T-A045V-S053G-N076D-S078N-S087D-S101N-N118R-G128S-S182Q-S183N-S204Q-Q206Y-Y217Q-M222Q-S248A-T254A, S003Q-T022Y-S024K-S033T-S053G-N076D-S078N-S101N-G128S-S145R-S162Q-G166Q-S182Q-Q206Y-Y217Q-N218S-M222Q-T254A, S003Q-Y006W-T022Y-S024Q-S033T-A045V-S053G-T055P-N076D-S078N-S087D-S101N-N118R-G128S-S182Q-S183N-S204Q-Q206Y-Y217Q-N218S-M222Q-S248A-T254A, S003Q-S024Q-S033T-A045V-S053G-T055P-N076D-S078N-S101N-N109Q-G128AS145R-S162Q-S182Q-S204Q-Y217Q-M222Q-T254A, and S033T-S053G-N076D-S078N-S101N-G128S-S145R-S183N-Y217Q-S248A, where the positions are numbered by correspondence with the amino acid sequence of SEQ ID NO:1, and the variant subtilisin comprises an amino acid sequence having at least 75%, or at least 80%, or at least 81%, or at least 85%, or at least 90%, or at least 91%, or at least 92%, or at least 93%, or at least 94%, or at least 95%, or at least 96%, or at least 97%, or at least 98% amino acid sequence identity to the amino acid sequence of SEQ ID NO: 1.

In some embodiments, the combinations of substitutions are selected from the group consisting of X055P-X204Q, X182Q-X222Q, X162Q-X204Q, X006W-X024K, X222Q-X254A, X206Y-X222Q, X055P-X222Q, X109Q-X222Q, X183N-X204Q, X162Q-X222Q, X006W-X183N, X204Q-X222Q, X183N-X222Q, X006W-X204Q, X222Q-X248A, X204Q-X254A, X006W-X254A, X109Q-X162Q-X182Q, X076D-X182Q-X222Q, X045V-X204Q-X222Q, X101N-X162Q-X222Q, X055P-X109Q-X204Q, X006W-X024K-X222Q, X109Q-X162Q-X222Q, X055P-X162Q-X204Q, X162Q-X182Q-X204Q, X162Q-X182Q-X222Q, X101N-X182Q-X222Q, X055P-X182Q-X254A, X204Q-X217Q-X222Q, X006W-X183N-X206Y, X109Q-X182Q-X222Q, X055P-X204Q-X222Q, X109Q-X204Q-X254A, X055P-X182Q-X222Q, X055P-X162Q-X254A, X055P-X222Q-X254A, X003Q-X162Q-X254A, X078N-X162Q-X254A, X109Q-X204Q-X222Q, X003Q-X182Q-X254A, X003Q-X222Q-X254A, X033T-X222Q-X254A, X024Q-X204Q-X222Q, X022Y-X162Q-X254A, X078N-X182Q-X254A, X003Q-X162Q-X222Q, X109Q-X222Q-X254A, X022Y-X182Q-X254A, X087D-X162Q-X254A, X076D-X182Q-X254A, X204Q-X217Q-X254A, X076D-X162Q-X254A, X078N-X204Q-X254A, X128S-X204Q-X254A, X076D-X204Q-X254A, X087D-X182Q-X254A, X182Q-X204Q-X254A, X101N-X204Q-X254A, X024Q-X204Q-X254A, X128A-X162Q-X254A, X182Q-X222Q-X254A, X128A-X204Q-X254A, X055P-X109Q-X182Q-X204Q, X109Q-X162Q-X182Q-X222Q, X055P-X182Q-X204Q-X222Q, X055P-X109Q-X162Q-X204Q, X055P-X162Q-X182Q-X204Q, X006W-X109Q-X183N-X248A, X055P-X162Q-X182Q-X222Q, X024Q-X078N-X182Q-X254A, X055P-X162Q-X182Q-X254A, X162Q-X182Q-X248A-X254A, X055P-X162Q-X204Q-X222Q, X006W-X109Q-X182Q-X248A, X055P-X109Q-X162Q-X254A, X055P-X109Q-X162Q-X222Q, X183N-X222Q-X248A-X254A, X078N-X109Q-X145R-X254A, X055P-X087D-X145R-X254A, X109Q-X182Q-X204Q-X222Q, X162Q-X182Q-X204Q-X222Q, X006W-X162Q-X182Q-X254A, X109Q-X204Q-X222Q-X254A, X055P-X162Q-X204Q-X254A, X006W-X109Q-X162Q-X254A, X055P-X109Q-X182Q-X222Q, X006W-X024K-X183N-X254A, X055P-X182Q-X183N-X254A, X055P-X182Q-X204Q-X254A, X162Q-X182Q-X222Q-X254A, X055P-X182Q-X222Q-X254A, X162Q-X182Q-X183N-X254A, X006W-X024K-X182Q-X254A, X182Q-X204Q-X222Q-X254A, X006W-X183N-X248A-X254A, X182Q-X183N-X204Q-X254A, X006W-X109Q-X204Q-X254A, X109Q-X118R-X169A-X222Q, X109Q-X183N-X204Q-X254A, X006W-X055P-X182Q-X254A, X006W-X182Q-X248A-X254A, X006W-X162Q-X204Q-X254A, X024K-X033T-X166Q-X222Q, X003Q-X076D-X222Q-X254A, X162Q-X182Q-X204Q-X254A, X006W-X128S-X217Q-X254A, X006W-X183N-X204Q-X254A, X024Q-X045V-X055P-X078N-X109Q-X182Q, X024Q-X109Q-X145R-X162Q-X217Q-X222Q, X053G-X076D-X162Q-X182Q-X204Q-X217Q, X003Q-X053G-X101N-X109Q-X183N-X254A, X024Q-X033T-X076D-X182Q-X183N-X254A, X003Q-X053G-X055P-X076D-X128A-X254A, X033T-X076D-X145R-X162Q-X182Q-X254A, X006W-X055P-X128S-X217Q-X222Q-X254A, X024Q-X076D-X162Q-X204Q-X222Q-X254A, X101N-X145R-X182Q-X183N-X204Q-X218S, X024K-X162Q-X182Q-X206Y-X222Q-X254A, X024K-X087D-X145R-X183N-X222Q-X254A, X024Q-X101N-X145R-X182Q-X204Q-X222Q, X022Y-X087D-X109Q-X183N-X222Q-X254A, X024Q-X118R-X169A-X182Q-X183N-X248A, X024Q-X101N-X182Q-X204Q-X222Q-X254A, X003Q-X109Q-X128S-X162Q-X166Q-X183N, X076D-X109Q-X145R-X182Q-X222Q-X254A, X006W-X024K-X101N-X128A-X169A-X183N, X101N-X166Q-X169AX182Q-X204Q-X206Y, X109Q-X182Q-X183N-X204Q-X206Y-X254A, X024K-X087D-X169AX204Q-X218S-X222Q, X006W-X045V-X128A-X145R-X182Q-X254A, X006W-X087D-X109Q-X145R-X162Q-X169A, X006W-X055P-X182Q-X183N-X204Q-X206Y-X222Q-X248A-X254Axxx, where the positions are numbered by correspondence with the amino acid sequence of SEQ ID NO:1, and the variant subtilisin comprises an amino acid sequence having at least 75%, 80%, 85%, 90%, 91%, 92%, 93%, 94%, 95%, 96%, 97%, or 98% amino acid sequence identity to the amino acid sequence of SEQ ID NO: 1.

In some embodiments, the combinations of substitutions are selected from the group consisting of T055P-S204Q, S182Q-M222Q, S162Q-S204Q, Y006W-S024K, M222Q-T254A, Q206Y-M222Q, T055P-M222Q, N109Q-M222Q, S183N-S204Q, S162Q-M222Q, Y006W-S183N, S204Q-M222Q, S183N-M222Q, Y006W-S204Q, M222Q-S248A, S204Q-T254A, Y006W-T254A, N109Q-S162Q-S182Q, N076D-S182Q-M222Q, A045V-S204Q-M222Q, S101N-S162Q-M222Q, T055P-N109Q-S204Q, Y006W-S024K-M222Q, N109Q-S162Q-M222Q, T055P-S162Q-S204Q, S162Q-S182Q-S204Q, S162Q-S182Q-M222Q, S101N-S182Q-M222Q, T055P-S182Q-T254A, S204Q-Y217Q-M222Q, Y006W-S183N-Q206Y, N109Q-S182Q-M222Q, T055P-S204Q-M222Q, N109Q-S204Q-T254A, T055P-S182Q-M222Q, T055P-S162Q-T254A, T055P-M222Q-T254A, S003Q-S162Q-T254A, S078N-S162Q-T254A, N109Q-S204Q-M222Q, S003Q-S182Q-T254A, S003Q-M222Q-T254A, S033T-M222Q-T254A, S024Q-S204Q-M222Q, T022Y-S162Q-T254A, S078N-S182Q-T254A, S003Q-S162Q-M222Q, N109Q-M222Q-T254A, T022Y-S182Q-T254A, S087D-S162Q-T254A, N076D-S182Q-T254A, S204Q-Y217Q-T254A, N076D-S162Q-T254A, S078N-S204Q-T254A, G128S-S204Q-T254A, N076D-S204Q-T254A, S087D-S182Q-T254A, S182Q-S204Q-T254A, S101N-S204Q-T254A, S024Q-S204Q-T254A, G128A-S162Q-T254A, S182Q-M222Q-T254A, G128A-S204Q-T254A, T055P-N109Q-S182Q-S204Q, N109Q-S162Q-S182Q-M222Q, T055P-S182Q-S204Q-M222Q, T055P-N109Q-S162Q-S204Q, T055P-S162Q-S182Q-S204Q, Y006W-N109Q-S183N-S248A, T055P-S162Q-S182Q-M222Q, S024Q-S078N-S182Q-T254A, T055P-S162Q-S182Q-T254A, S162Q-S182Q-S248A-T254A, T055P-S162Q-S204Q-M222Q, Y006W-N109Q-S182Q-S248A, T055P-N109Q-S162Q-T254A, T055P-N109Q-S162Q-M222Q, S183N-M222Q-S248AT254A, S078N-N109Q-S145R-T254A, T055P-S087D-S145R-T254A, N109Q-S182Q-S204Q-M222Q, S162Q-S182Q-S204Q-M222Q, Y006W-S162Q-S182Q-T254A, N109Q-S204Q-M222Q-T254A, T055P-S162Q-S204Q-T254A, Y006W-N109Q-S162Q-T254A, T055P-N109Q-S182Q-M222Q, Y006W-S024K-S183N-T254A, T055P-S182Q-S183N-T254A, T055P-S182Q-S204Q-T254A, S162Q-S182Q-M222Q-T254A, T055P-S182Q-M222Q-T254A, S162Q-S182Q-S183N-T254A, Y006W-S024K-S182Q-T254A, S182Q-S204Q-M222Q-T254A, Y006W-S183N-S248A-T254A, S182Q-S183N-S204Q-T254A, Y006W-N109Q-S204Q-T254A, N109Q-N118R-G169A-M222Q, N109Q-S183N-S204Q-T254A, Y006W-T055P-S182Q-T254A, Y006W-S182Q-S248A-T254A, Y006W-S162Q-S204Q-T254A, S024K-S033T-G166Q-M222Q, S003Q-N076D-M222Q-T254A, S162Q-S182Q-S204Q-T254A, Y006W-G128S-Y217Q-T254A, Y006W-S183N-S204Q-T254A, S024Q-A045V-T055P-S078N-N109Q-S182Q, S024Q-N109Q-S145R-S162Q-Y217Q-M222Q, S053G-N076D-S162Q-S182Q-S204Q-Y217Q, S003Q-S053G-S101N-N109Q-S183N-T254A, S024Q-S033T-N076D-S182Q-S183N-T254A, S003Q-S053G-T055P-N076D-G128AT254A, S033T-N076D-S145R-S162Q-S182Q-T254A, Y006W-T055P-G128S-Y217Q-M222Q-T254A, S024Q-N076D-S 162Q-S204Q-M222Q-T254A, S101N-S145R-S182Q-S183N-S204Q-N218S, S024K-S162Q-S182Q-Q206Y-M222Q-T254A, S024K-S087D-S145R-S183N-M222Q-T254A, S024Q-S101N-S145R-S182Q-S204Q-M222Q, T022Y-S087D-N109Q-S183N-M222Q-T254A, S024Q-N118R-G169AS182Q-S183N-S248A, S024Q-S101N-S182Q-S204Q-M222Q-T254A, S003Q-N109Q-G128S-S162Q-G166Q-S183N, N076D-N109Q-S145R-S182Q-M222Q-T254A, Y006W-S024K-S101N-G128A-G169AS183N, S101N-G166Q-G169A-S182Q-S204Q-Q206Y, N109Q-S182Q-S183N-S204Q-Q206Y-T254A, S024K-S087D-G169A-S204Q-N218S-M222Q, Y006W-A045V-G128A-S145R-S182Q-T254A, Y006W-S087D-N109Q-S145R-S162Q-G169A, Y006W-T055P-S182Q-S183N-S204Q-Q206Y-M222Q-S248AT254Axxx, where the positions are numbered by correspondence with the amino acid sequence of SEQ ID NO: 1, and the variant subtilisin comprises an amino acid sequence having at least 75%, 80%, 85%, 90%, 91%, 92%, 93%, 94%, 95%, 96%, 97%, or 98% amino acid sequence identity to the amino acid sequence of SEQ ID NO: 1.

Typically, a cleaning composition comprises at least about 0.0001 to about 20 wt %, from about 0.0001 to about 10 wt %, from about 0.0001 to about 1 wt %, from about 0.001 to about 1 wt %, or from about 0.01 to about 0.2 wt % of one or more subtilisin variant described herein. In another embodiment, one or more cleaning composition described herein comprises from about 0.01 to about 10 mg, about 0.01 to about 5 mg, about 0.01 to about 2 mg, about 0.01 to about 1 mg, about 0.05 to about 1 mg, about 0.5 to about 10 mg, about 0.5 to about 5 mg, about 0.5 to about 4 mg, about 0.5 to about 3 mg, about 0.5 to about 2 mg, about 0.5 to about 1 mg, about 0.1 to about 10 mg, about 0.1 to about 5 mg, about 0.1 to about 4 mg, about 0.1 to about 3 mg, about 0.1 to about 2 mg, about 0.1 to about 2 mg, about 0.1 to about 1 mg, or about 0.1 to about 0.5 mg of one or more subtilisin variant described herein per gram of composition.

The subtilisin variants preferably comprise one or more modification at a surface exposed amino acid to change the surface property of the variant. Surface modifications in the enzyme variants can be useful in a detergent composition by having a minimum performing index for wash performance, stability of the enzyme in detergent compositions and thermostability of the enzyme, while having at least one of these characteristics improved from a parent subtilisin enzyme. In some embodiments, the surface modification changes the hydrophobicity and/or charge of the amino acid at that position. Hydrophobicity can be determined using techniques known in the art, such as those described in White and Wimley (White,S.H. and Wimley, W.C,. (1999) Annu. Rev. Biophys. Biomol. Struct. 28:319-65.

The "surface property" which is altered or changed may be electrostatic charge, or a property such as the hydrophobicity and hydrophilicity exhibited by the surface of a protein. Preferably the one or more subtilisin variant described herein has one or more improved property when compared to a reference or parent subtilisin; wherein the improved property is selected from improved cleaning performance in detergents, improved stability; and combinations thereof.

The parent subtilisin may comprise an amino acid sequence of SEQ ID NO: 1, or a polypeptide having the amino acid sequence of SEQ ID NO:1. Preferably the variant has the improved property of improved stability, where the variant has a greater residual activity compared to the parent or reference subtilisin. The improved stability in detergent may be as measured in accordance with the stability assays of Example 2.

The term "enhanced stability" or "improved stability" in the context of an oxidation, chelator, denaturant, surfactant, thermal and/or pH stable protease refers to a higher retained proteolytic activity of a subtilisin variant over time as compared to a reference or parent subtilisin protease, for example, a wild-type protease or parent protease, such as SEQ ID NO: 1. Autolysis has been identified as one mode of subtilisin activity loss in liquid detergents. (Stoner et al., 2004 Protease autolysis in heavy-duty liquid detergent formulations: effects of thermodynamic stabilizers and protease inhibitors, Enzyme and Microbial Technology 34:114-125.).

The terms "thermally stable" and "thermostable" and "thermostability" with regard to a protease variant refer to a protease that retains a greater amount of residual activity when compared to the parent or reference protease after exposure to altered temperatures over a given period of time under conditions (or "stress conditions") prevailing during proteolytic, hydrolysing, cleaning or other process. Residual activity is the amount of activity remaining after the test compared to the initial activity of the sample and can be reported as a percentage e.g. % remaining activity. "Altered temperatures" encompass increased or decreased temperatures. In some embodiments, the variant proteases provided herein retain at least about 5%, about 10%, about 20%, about 30%, about 40%, about 50%, about 60%, about 70%, about 80%, about 85%, about 90%, about 92%, about 95%, about 96%, about 97%, about 98%, or about 99% proteolytic activity after exposure to temperatures of 40°C to 80°C, over a given time period, for example, at least about 5 minutes, at least about 20 minutes, at least about 60 minutes, about 90 minutes, about 120 minutes, about 180 minutes, about 240 minutes, about 300 minutes, about 360 minutes, about 420 minutes, about 480 minutes, about 540 minutes, about 600 minutes, about 660 minutes, about 720 minutes, about 780 minutes, about 840 minutes, about 900 minutes, about 960 minutes, about 1020 minutes, about 1080 minutes, about 1140 minutes, or about 1200 minutes. In some embodiments, the variant subtilisins provided herein have a residual activity that is greater than that of the parent or reference protease using the method set forth in Example 2. In some embodiments, the variant subtilisins provided herein have at least a 5% improved residual activity compared to the parent subtilisin when measured after 20 minutes at 40-80 degrees Celsius in a liquid detergent. In some embodiments, the variant subtilisins provided herein have at least a 10%, 15%, 20%, 25%, 30%, 40%, 50%, 60%, 70%, 80%, 90% or 100% improved residual activity compared to the parent subtilisin when measured after 20 minutes at 40-80 degrees Celsius in a liquid detergent.

The subtilisin variant described herein can be subject to various changes, such as one or more amino acid insertion, deletion, and/or substitution, either conservative or non-conservative, including where such changes do not substantially alter the enzymatic activity of the variant. Similarly, a nucleic acid of the invention can also be subject to various changes, such as one or more substitution of one or more nucleotide in one or more codon such that a particular codon encodes the same or a different amino acid, resulting in either a silent variation (e.g., when the encoded amino acid is not altered by the nucleotide mutation) or non-silent variation; one or more deletion of one or more nucleotides (or codon) in the sequence; one or more addition or insertion of one or more nucleotides (or codon) in the sequence; and/or cleavage of, or one or more truncation, of one or more nucleotides (or codon) in the sequence. Many such changes in the nucleic acid sequence may not substantially alter the enzymatic activity of the resulting encoded polypeptide enzyme compared to the polypeptide enzyme encoded by the original nucleic acid sequence. A nucleic acid sequence described herein can also be modified to include one or more codon that provides for optimum expression in an expression system (e.g., bacterial expression system), while, if desired, said one or more codon still encodes the same amino acid(s).

Described herein is one or more isolated, non-naturally occurring, or recombinant polynucleotide comprising a nucleic acid sequence that encodes one or more subtilisin variant described herein, or recombinant polypeptide or active fragment thereof. One or more nucleic acid sequence described herein is useful in recombinant production (e.g., expression) of one or more subtilisin variant described herein, typically through expression of a plasmid expression vector comprising a sequence encoding the one or more subtilisin variant described herein or fragment thereof. One embodiment provides nucleic acids encoding one or more subtilisin variant described herein, wherein the variant is a mature form having proteolytic activity. In some embodiments, one or more subtilisin variant described herein is expressed recombinantly with a homologous pro-peptide sequence. In other embodiments, one or more subtilisin variant described herein is expressed recombinantly with a heterologous pro-peptide sequence (e.g., pro-peptide sequence from *Bacillus amyloliquefaciens* (SEQ ID NO:4) or variants thereof).

One or more nucleic acid sequence described herein can be generated by using any suitable synthesis, manipulation, and/or isolation techniques, or combinations thereof. For example, one or more polynucleotide described herein may be produced using standard nucleic acid synthesis techniques, such as solid-phase synthesis techniques that are well-known to those skilled in the art. In such techniques, fragments of up to 50 or more nucleotide bases are typically synthesized, then joined (e.g., by enzymatic or chemical ligation methods) to form essentially any desired continuous nucleic acid sequence. The synthesis of the one or more polynucleotide described herein can be also facilitated by any suitable method known in the art, including but not limited to chemical synthesis using the classical phosphoramidite method (*See e.g.,* Beaucage et al. Tetrahedron Letters 22:1859-69 (1981)), or the method described in Matthes et al., EMBO J. 3:801-805 (1984) as is typically practiced in automated synthetic methods. One or more polynucleotide described herein can also be produced by using an automatic DNA synthesizer. Customized nucleic acids can be ordered from a variety of commercial sources (e.g., ATUM (DNA 2.0), Newark, CA, USA; Life Tech (GeneArt), Carlsbad, CA, USA; GenScript, Ontario, Canada; Base Clear B. V., Leiden, Netherlands; Integrated DNA Technologies, Skokie, IL, USA; Ginkgo Bioworks (Gen9), Boston, MA, USA; and Twist Bioscience, San Francisco, CA, USA). Other techniques for synthesizing nucleic acids and related principles are described by, for example, Itakura et al., Ann. Rev. Biochem. 53:323 (1984) and Itakura et al., Science 198:1056 (1984).

Recombinant DNA techniques useful in modification of nucleic acids are well known in the art, such as, for example, restriction endonuclease digestion, ligation, reverse transcription and cDNA production, and polymerase chain reaction (e.g., PCR). One or more polynucleotide described herein may also be obtained by screening cDNA libraries using one or more oligonucleotide probes that can hybridize to or PCR-amplify polynucleotides which encode one or more subtilisin variant described herein, or recombinant polypeptide or active fragment thereof. Procedures for screening and isolating cDNA clones and PCR amplification procedures are well known to those of skill in the art and described in standard references known to those skilled in the art. One or more polynucleotide described herein can be obtained by altering a naturally occurring polynucleotide backbone (e.g., that encodes one or more subtilisin variant described herein or reference subtilisin) by, for example, a known mutagenesis procedure (e.g., site-directed mutagenesis, site saturation mutagenesis, and in vitro recombination). A variety of methods are known in the art that are suitable for generating modified polynucleotides described herein that encode one or more subtilisin variant described herein, including, but not limited to, for example, site-saturation mutagenesis, scanning mutagenesis, insertional mutagenesis, deletion mutagenesis, random mutagenesis, site-directed mutagenesis, and directed-evolution, as well as various other recombinatorial approaches.

A further embodiment disclosed herein is directed to one or more vector comprising one or more subtilisin variant described herein (e.g., a polynucleotide encoding one or more subtilisin variant described herein); expression vectors or expression cassettes comprising one or more nucleic acid or polynucleotide sequence described herein; isolated, substantially pure, or recombinant DNA constructs comprising one or more nucleic acid or polynucleotide sequence described herein; isolated or recombinant cells comprising one or more polynucleotide sequence described herein; and compositions comprising one or more such vector, nucleic acid, expression vector, expression cassette, DNA construct, cell, cell culture, or any combination or mixtures thereof.

Some embodiments are directed to one or more recombinant cell comprising one or more vector (e.g., expression vector or DNA construct) described herein which comprises one or more nucleic acid or polynucleotide sequence described herein. Some such recombinant cells are transformed or transfected with such at least one vector, although other methods are available and known in the art. Such cells are typically referred to as host cells. Some such cells comprise bacterial cells, including, but not limited to *Bacillus sp.* cells, such as *B. subtilis* cells. Other embodiments are directed to recombinant cells (e.g., recombinant host cells) comprising one or more subtilisin described herein.

In some embodiments, one or more vector described herein is an expression vector or expression cassette comprising one or more polynucleotide sequence described herein operably linked to one or more additional nucleic acid segments required for efficient gene expression (e.g., a promoter operably linked to one or more polynucleotide sequence described herein). A vector may include a transcription terminator and/or a selection gene (e.g., an antibiotic resistant gene) that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pC194, pJH101, pE194, pHP13 (See, Harwood and Cutting [eds.], Chapter 3, Molecular Biological Methods for Bacillus, John Wiley & Sons (1990); suitable replicating plasmids for *B. subtilis* include those listed on p. 92). (*See also,* Perego, "Integrational Vectors for Genetic Manipulations in Bacillus subtilis"; Sonenshein et al., [eds.]; "Bacillus subtilis and Other Gram-Positive Bacteria: Biochemistry, Physiology and Molecular Genetics", American Society for Microbiology, Washington, D.C. (1993), pp. 615-624); and p2JM103BBI).

For expression and production of a protein of interest (e.g., one or more subtilisin variant described herein) in a cell, one or more expression vector comprising one or more copy of a polynucleotide encoding one or more subtilisin variant described herein, and in some instances comprising multiple copies, is transformed into the cell under conditions suitable for expression of the variant. In some embodiments, a polynucleotide sequence encoding one or more subtilisin variant described herein (as well as other sequences included in the vector) is integrated into the genome of the host cell, while in other embodiments, a plasmid vector comprising a polynucleotide sequence encoding one or more subtilisin variant described herein remains as autonomous extra-chromosomal element within the cell. Some embodiments provide both extrachromosomal nucleic acid elements as well as incoming nucleotide sequences that are integrated into the host cell genome. The vectors described herein are useful for production of the one or more subtilisin variant described herein. In some embodiments, a polynucleotide construct encoding one or more subtilisin variant described herein is present on an integrating vector that enables the integration and optionally the amplification of the polynucleotide encoding the variant into the host chromosome. Examples of sites for integration are well known to those skilled in the art. In some embodiments, transcription of a polynucleotide encoding one or more subtilisin variant described herein is effectuated by a promoter that is the wild-type promoter for the parent subtilisin. In some other embodiments, the promoter is heterologous to the one or more subtilisin variant described herein, but is functional in the host cell. Exemplary promoters for use in bacterial host cells include, but are not limited to the amyE, amyQ, amyL, pstS, sacB, pSPAC, pAprE, pVeg, pHpaII promoters; the promoter of the *B. stearothermophilus* maltogenic amylase gene; the *B. amyloliquefaciens* (BAN) amylase gene; the *B. subtilis* alkaline protease gene; the *B. clausii* alkaline protease gene; the *B. pumilis* xylosidase gene; the *B. thuringiensis* cryIIIA; and the *B. licheniformis* alpha-amylase gene. Additional promoters include, but are not limited to the A4 promoter, as well as phage Lambda PR or PL promoters and the *E*. *coli* lac, trp or tac promoters.

The subtilisin variant described herein can be produced in host cells of any suitable microorganism, including bacteria and fungi. In some embodiments, one or more subtilisin variant described herein can be produced in Gram-positive bacteria. In some embodiments, the host cells are *Bacillus spp., Streptomyces spp., Escherichia spp., Aspergillus spp., Trichoderma spp., Pseudomonas spp., Corynebacterium spp., Saccharomyces spp.,* or *Pichia spp.* In some embodiments, one or more subtilisin variant described herein is produced by *Bacillus sp.* host cells. Examples of *Bacillus sp.* host cells that find use in the production of the one or more subtilisin variant described herein include, but are not limited to *B. licheniformis, B. gibsonii, B. lentus, B. subtilis, B. amyloliquefaciens, B. brevis, B. stearothermophilus, B. alkalophilus, B. coagulans, B. circulans, B. pumilis, B. thuringiensis, B. clausii,* and *B. megaterium,* as well as other organisms within the genus *Bacillus.* In some embodiments, *B. subtilis* host cells are used to produce the variants described herein. USPNs 5,264,366 and 4,760,025 (RE 34,606) describe various *Bacillus* host strains that can be used to produce one or more subtilisin variant described herein, although other suitable strains can be used.

Several bacterial strains that can be used to produce one or more subtilisin variant described herein include non-recombinant (i.e., wild-type) *Bacillus sp.* strains, as well as variants of naturally-occurring strains and/or recombinant strains. In some embodiments, the host strain is a recombinant strain, wherein a polynucleotide encoding one or more subtilisin variant described herein has been introduced into the host. In some embodiments, the host strain is a *B. subtilis* host strain and particularly a recombinant *B. subtilis* host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (*See e.g.,* Hoch et al., Genetics 73:215-228 (1973); *See also,* US 4,450,235; US 4,302,544; and EP 0134048). The use of *B. subtilis* as an expression host cell is well known in the art (*See e.g.,* Palva et al., Gene 19:81-87 (1982); Fahnestock and Fischer, J. Bacteriol., 165:796-804 (1986); and Wang et al., Gene 69:39-47 (1988)).

The *Bacillus* host cell may be a *Bacillus sp.* that includes a mutation or deletion in at least one of the following genes: degU, degS, degR and degQ. In some embodiments, the mutation is in a degU gene, and in some embodiments the mutation is degU(Hy)32 (*See e.g.,* Msadek et al., J. Bacteriol. 172:824-834 (1990); and Olmos et al., Mol. Gen. Genet. 253:562-567 (1997)). In some embodiments, the *Bacillus* host comprises a mutation or deletion in scoC4 (*See e.g.,* Caldwell et al., J. Bacteriol. 183:7329-7340 (2001)); spoIIE (*See e.g.,* Arigoni et al., Mol. Microbiol. 31:1407-1415 (1999)); and/or oppA or other genes of the opp operon (*See e.g.,* Perego et al., Mol. Microbiol. 5: 173-185 (1991)). Indeed, it is contemplated that any mutation in the opp operon that causes the same phenotype as a mutation in the oppA gene will find use in some embodiments of the altered *Bacillus* strain described herein. In some embodiments, these mutations occur alone, while in other embodiments, combinations of mutations are present. In some embodiments, an altered *Bacillus* host cell strain that can be used to produce one or more subtilisin variant described herein is a *Bacillus* host strain that already includes a mutation in one or more of the above-mentioned genes. In addition, *Bacillus sp.* host cells that comprise mutation(s) and/or deletion(s) of endogenous protease genes find use. In some embodiments, the *Bacillus* host cell comprises a deletion of the aprE and the nprE genes. In other embodiments, the *Bacillus sp.* host cell comprises a deletion of 5 protease genes, while in other embodiments the *Bacillus sp.* host cell comprises a deletion of 9 protease genes (*See e.g.,* US 2005/0202535).

Host cells may be transformed with one or more nucleic acid sequence encoding one or more subtilisin variant described herein using any suitable method known in the art. Methods for introducing a nucleic acid (e.g., DNA) into *Bacillus* cells or *E*. *coli* cells utilizing plasmid DNA constructs or vectors and transforming such plasmid DNA constructs or vectors into such cells are well known. In some embodiments, the plasmids are subsequently isolated from *E*. *coli* cells and transformed into *Bacillus* cells. However, it is not essential to use intervening microorganisms such as *E*. *coli,* and in some embodiments, a DNA construct or vector is directly introduced into a *Bacillus* host.

Exemplary methods for introducing one or more nucleic acid sequence described herein into *Bacillus* cells are described in, for example, Ferrari et al., "Genetics," in Harwood et al. [eds.], Bacillus, Plenum Publishing Corp. (1989), pp. 57-72; Saunders et al., J. Bacteriol. 157:718-726 (1984); Hoch et al., J. Bacteriol. 93:1925-1937 (1967); Mann et al., Current Microbiol. 13:131-135 (1986); Holubova, Folia Microbiol. 30:97 (1985); Chang et al., Mol. Gen. Genet. 168:11-115 (1979); Vorobjeva et al., FEMS Microbiol. Lett. 7:261-263 (1980); Smith et al., Appl. Env. Microbiol. 51:634 (1986); Fisher et al., Arch. Microbiol. 139:213-217 (1981); and McDonald, J. Gen. Microbiol. 130:203 (1984)). Indeed, such methods as transformation, including protoplast transformation and transfection, transduction, and protoplast fusion are well known and suited for use herein. Methods known in the art to transform *Bacillus* cells include such methods as plasmid marker rescue transformation, which involves the uptake of a donor plasmid by competent cells carrying a partially homologous resident plasmid (*See,* Contente et al., Plasmid 2:555-571 (1979); Haima et al., Mol. Gen. Genet. 223:185-191 (1990); Weinrauch et al., J. Bacteriol. 154:1077-1087 (1983); and Weinrauch et al., J. Bacteriol. 169:1205-1211 (1987)). In this method, the incoming donor plasmid recombines with the homologous region of the resident "helper" plasmid in a process that mimics chromosomal transformation.

In addition to commonly used methods, host cells may be directly transformed with a DNA construct or vector comprising a nucleic acid encoding one or more subtilisin variant described herein (i.e., an intermediate cell is not used to amplify, or otherwise process, the DNA construct or vector prior to introduction into the host cell). Introduction of a DNA construct or vector described herein into the host cell includes those physical and chemical methods known in the art to introduce a nucleic acid sequence (e.g., DNA sequence) into a host cell without insertion into the host genome. Such methods include, but are not limited to calcium chloride precipitation, electroporation, naked DNA, and liposomes. In additional embodiments, DNA constructs or vector are co-transformed with a plasmid, without being inserted into the plasmid. In further embodiments, a selective marker is deleted from the altered *Bacillus* strain by methods known in the art (*See,* Stahl et al., J. Bacteriol. 158:411-418 (1984); and Palmeros et al., Gene 247:255 - 264 (2000)).

The transformed cells may be cultured in conventional nutrient media. The suitable specific culture conditions, such as temperature, pH and the like are known to those skilled in the art and are well described in the scientific literature. Some embodiments provide a culture (e.g., cell culture) comprising one or more subtilisin variant or nucleic acid sequence described herein.

Host cells transformed with one or more polynucleotide sequences encoding one or more subtilisin variant described herein may be cultured in a suitable nutrient medium under conditions permitting the expression of the variant, after which the resulting variant is recovered from the culture. The variant produced by the cells may be recovered from the culture medium by conventional procedures, including, but not limited to, for example, separating the host cells from the medium by centrifugation or filtration, precipitating the proteinaceous components of the supernatant or filtrate by means of a salt (e.g., ammonium sulfate), and chromatographic purification (e.g., ion exchange, gel filtration, affinity, etc.).

One or more subtilisin variant produced by a recombinant host cell may be secreted into the culture medium. A nucleic acid sequence that encodes a purification facilitating domain may be used to facilitate purification of the variant. A vector or DNA construct comprising a polynucleotide sequence encoding one or more subtilisin variant described herein may further comprise a nucleic acid sequence encoding a purification facilitating domain to facilitate purification of the variant (*See e.g.,* Kroll et al., DNA Cell Biol. 12:441-53 (1993)). Such purification facilitating domains include, but are not limited to, for example, metal chelating peptides such as histidine-tryptophan modules that allow purification on immobilized metals (See, Porath, Protein Expr. Purif. 3:263-281 [1992]), protein A domains that allow purification on immobilized immunoglobulin, and the domain utilized in the FLAGS extension/affinity purification system. The inclusion of a cleavable linker sequence such as Factor XA or enterokinase (e.g., sequences available from Invitrogen, San Diego, CA) between the purification domain and the heterologous protein also find use to facilitate purification.

The variant proteins may be produced in host cells, for example, by secretion or intracellular expression, using methods well-known in the art. Fermentation, separation, and concentration techniques are well known in the art and conventional methods can be used to prepare a concentrated, enzyme-containing solution. Host cells may be further processed, such as to release enzyme or to improve cell separation, for example by heating or by changing pH or salt content or by treating with enzymes including hen egg white lysozyme, T4 lysozyme, or enzymes described in WO2022047149. For production scale recovery, variant polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules, or non-aqueous particles.

A variety of methods can be used to determine the level of production of one or more mature subtilisin variant described herein in a host cell. Such methods include, but are not limited to, for example, methods that utilize either polyclonal or monoclonal antibodies specific for the protease. Exemplary methods include, but are not limited to enzyme-linked immunosorbent assays (ELISA), radioimmunoassays (RIA), fluorescent immunoassays (FIA), and fluorescent activated cell sorting (FACS). These and other assays are well known in the art (*See e.g.,* Maddox et al., J. Exp. Med. 158:1211 (1983)).

Also disclosed herein are methods for making or producing one or more mature subtilisin variant described herein. A mature subtilisin variant does not include a signal peptide or a propeptide sequence. Some methods comprise making or producing one or more subtilisin variant described herein in a recombinant bacterial host cell, such as for example, a *Bacillus sp.* cell (e.g., a *B. subtilis* cell). Other embodiments provide a method of producing one or more subtilisin variant described herein, wherein the method comprises cultivating a recombinant host cell comprising a recombinant expression vector comprising a nucleic acid sequence encoding one or more subtilisin variant described herein under conditions conducive to the production of the variant. Some such methods further comprise recovering the variant from the culture.

Also disclosed herein are methods of producing one or more subtilisin variant described herein, wherein the methods comprise: (a) introducing a recombinant expression vector comprising a nucleic acid encoding the variant into a population of cells (e.g., bacterial cells, such as *B. subtilis* cells); and (b) culturing the cells in a culture medium under conditions conducive to produce the variant encoded by the expression vector. Some such methods further comprise: (c) isolating the variant from the cells or from the culture medium.

A further embodiment is directed to a method of improving the cleaning performance or stability of a subtilisin comprising modifying a subtilisin to include one or more substitutions, or combination of substitutions, as provided herein. The subtilisin variants suitable for use herein may be used in the production of various compositions, such as enzyme compositions and cleaning or detergent compositions. An enzyme composition comprises a subtilisin variant as provided herein. The enzyme composition can be in any form, such as liquid formulations, gels, granules, or enzyme slurries, suitable for incorporation into a cleaning composition.

Enzyme granules may be made by, *e.g*., rotary atomization, wet granulation, dry granulation, spray drying, disc granulation, extrusion, pan coating, spheronization, drum granulation, fluid-bed agglomeration, high-shear granulation, fluid-bed spray coating, crystallization, precipitation, emulsion gelation, spinning disc atomization and other casting approaches, and prilling processes. The core of the granule may be the granule itself or the inner nucleus of a layered granule.

The core may comprise one or more water soluble or dispersible agent(s), including but not limited to, sodium sulfate, sodium chloride, magnesium sulfate, zinc sulfate, and ammonium sulfate), citric acid, sugars (*e.g*., sucrose, lactose, glucose, granulated sucrose, maltodextrin and fructose), plasticizers (*e.g*., polyols, urea, dibutyl phthalate, and dimethyl phthalate), fibrous material (*e.g*., cellulose and cellulose derivatives such as hydroxyl-propyl-methyl cellulose, carboxy-methyl cellulose, and hydroxyl-ethyl cellulose), phosphate, calcium, a protease inhibitor and combinations thereof. Suitable dispersible agents include, but are not limited to, clays, nonpareils (combinations of sugar and starch; *e.g*., starch-sucrose nonpareils - ASNP), talc, silicates, carboxymethyl cellulose, starch, and combinations thereof.

In some embodiments, the core comprises mainly sodium sulfate. In some embodiments, the core consists essentially of sodium sulfate. In a particular embodiment, the core consists of only sodium sulfate.

In some embodiments, the core comprises a subtilisin variant as provided herein. In other embodiments, the core comprises one or more enzymes in addition to protease. In other embodiments, the core is inert and does not comprise enzymes.

In some embodiments, the core is an enzyme powder, including UFC containing an enzyme. The enzyme powder may be spray dried and may optionally be admixed with any of the water soluble or dispersible agents listed, herein. The enzyme may be, or may include, the protease to be stabilized, in which case the enzyme power should further include a stabilizer.

In some embodiments, the core is coated with at least one coating layer. In a particular embodiment, the core is coated with at least two coating layers. In another particular embodiment the core is coated with at least three coating layers. The materials used in the coating layer(s) can be suitable for use in cleaning and/or detergent compositions (see, *e.g*., US20100124586, WO9932595 and US5324649.

In some embodiments, a coating layer comprises one of more of the following materials: an inorganic salt (*e.g*., sodium sulfate, sodium chloride, magnesium sulfate, zinc sulfate, and ammonium sulfate), citric acid, a sugar (*e.g*., sucrose, lactose, glucose, and fructose), a plasticizer (*e.g*., polyols, urea, dibutyl phthalate, and dimethyl phthalate), fibrous material (*e.g*., cellulose and cellulose derivatives such as hydroxyl-propyl-methyl cellulose, carboxy-methyl cellulose, and hydroxyl-ethyl cellulose), clay, nonpareil (a combination of sugar and starch), silicate, carboxymethyl cellulose, phosphate, starch (*e.g*., corn starch), fats, oils (*e.g*., rapeseed oil, and paraffin oil), lipids, vinyl polymers, vinyl copolymers, polyvinyl alcohol (PVA), plasticizers (*e.g*., polyols, urea, dibutyl phthalate, dimethyl phthalate, and water), anti-agglomeration agents (*e.g*., talc, clays, amorphous silica, and titanium dioxide), anti-foam agents (such as FOAMBLAST 882^{®} and EROL 6000K^{®}), and talc. US20100124586, WO9932595, and US5324649 detail suitable components for the coating layers.

In some embodiments, the coating layer comprises sugars (*e.g*., sucrose, lactose, glucose, granulated sucrose, maltodextrin and fructose). In some embodiments, the coating layer comprises a polymer such as polyvinyl alcohol (PVA). Suitable PVA for incorporation in the coating layer(s) of the multi-layered granule include partially hydrolyzed, fully hydrolyzed and intermediately hydrolyzed having low to high degrees of viscosity. In some embodiments, the coating layer comprises an inorganic salt, such as sodium sulfate.

In some embodiments, at least one coating layer is an enzyme coating layer. In some embodiments, the core is coated with at least two enzyme layers. In another embodiment, the core is coated with at least three or more enzyme layers.

In some embodiments, the enzyme granules comprise a subtilisin variant as provided herein in combination with one or more additional enzymes which may be wild type, recombinant and variant enzymes of bacterial, fungal, yeast sources, and acid, neutral or alkaline enzymes. The additional enzyme(s) may be selected from the group consisting of acyl transferases, alginate lyases, alpha-amylases, beta-amylases, alpha-galactosidases, arabinosidases, aryl esterases, beta-galactosidases, beta-glucanases, carrageenases, catalases, cellobiohydrolases, cellulases, chondroitinases, cutinases, dispersins, endo-beta-1, 4-glucanases, endo-beta-mannanases, esterases, exo-mannanases, fructosidases, galactanases, glucoamylases, hemicellulases, hexosaminidase, hyaluronidases, keratinases, laccases, lactases, laminarinases, lichenases, ligninases, lipases, lipoxygenases, lysozymes, mannanases, metalloproteases, nucleases (e.g. DNases and/or RNases), oxidases, oxidoreductases, pectate lyases, pectin acetyl esterases, pectinases, pentosanases, perhydrolases, peroxidases, phenoloxidases, phosphatases, phosphodiesterase, phospholipases, phytases, polygalacturonases, polyesterases, additional proteases, pullulanases, reductases, rhamnogalacturonases, beta-glucanases, tannases, transglutaminases, xanthan lyases, xylan acetyl-esterases, xylanases, xyloglucanases, xylosidases, and any combination or mixture thereof. Generally, at least one enzyme coating layer comprises at least one subtilisin variant as provided herein.

### Adjunct Material

In addition to subtilisin variant protease enzyme the cleaning composition comprises a cleaning/detergent adjunct material, preferably a mixture of cleaning/detergent adjunct materials. The terms cleaning adjunct and cleaning adjunct material are used interchangeably herein. Each cleaning adjunct material is typically selected depending on the purpose and form of cleaning composition (e.g., liquid, granule, powder, bar, paste, spray, tablet, gel, foam, or other composition). The term "adjunct material" refers to any liquid, solid, or gaseous material included in cleaning composition other than one or more subtilisin variant described herein, or recombinant polypeptide or active fragment thereof. The cleaning adjunct materials are typically selected depending on the particular type and form of cleaning composition (e.g., liquid, granule, powder, bar, paste, spray, tablet, gel, foam, or other composition). Where possible, preferred adjunct materials are bio-based or comprise at least a portion of bio-based materials, for example greater than 10 wt%, or greater than 25 wt% or greater than 40 or greater than 50 or greater than 60 or greater than 70 or greater than 80 or greater than 90 wt% bio-based." Preferably, the, or most cleaning adjunct material is compatible with the protease enzyme used in the composition. If one or more adjunct ingredient is not compatible with the protease variant, suitable methods may be employed to reduce contact and/or to keep the cleaning adjunct ingredient and proteases separated (*i.e.,* not in contact with each other) until combination of the two components is appropriate. Such separation methods include any suitable method known in the art (*e.g*., gelcaps, encapsulation, tablets, physical separation, etc.).

Typically, the cleaning adjunct will be present in the composition in an amount from 1 to 98.9 wt%, more typically from 5 to 90 wt% cleaning adjunct. Suitable cleaning adjuncts comprise: additional surfactants, builders, bleach ingredients, colorants, chelating agents, dye transfer agents, deposition aids, dispersants, additional enzymes, optionally encapsulated, and enzyme stabilizers, catalytic materials, optical brighteners, photoactivators, fluorescers, fabric hueing agents (shading dyes), fabric conditioners, preformed peracids, cleaning polymers such as polymeric dispersing agents, soil release polymers and/or clay soil removal/anti-redeposition agents, fabric conditioning polymers including Polyquaternium 10 (CathEC), filler salts, hydrotropes, brighteners, suds suppressors, structure elasticizing agents, fabric softeners, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents, encapsulates, polymers, antibacterial agents including Tinosan HP100, probiotics, or one or more microorganisms or microbes or microbial extracts or microbial spores, or mixture thereof. Microorganisms may be used as the only biologically active ingredient, but they may also be used in conjunction with one or more of the enzymes described herein.

For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide; dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes which may be free and/or encapsulated for example in perfume microcapsules, and/or in the form of starch encapsulated accords, and/or perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; polyethylene glycol, diethylene glycol, ethanol, dipropylene glycol, tripropyleneglycol, glycerol, sorbitol, and any mixture thereof; hydrotropes such as sodium cumene sulphonate, sodium xylene sulphonate, sodium toluene sulphonate, and any mixtures; organic acids and salts thereof, such as citric acid/citrate salts; microorganisms such as the bacillus strain having the deposit accession number PTA-7543, for example, may be used to reduce malodor as described in WO 2012/112718, exemplary commercial microbial products include but are not limited to Microvia^{™} (Novozymes), or for example, microorganisms which provide in-situ production of desirable biological compounds, or inoculation/population of a locus with the microorganism(s) to competitively prevent other non-desirable microorganisms form populating the same locus (competitive exclusion);and any combination thereof.

Preferably the composition comprises one or more selected from the group consisting of (i) perfumes, preferably comprising at least some perfume in a microcapsule; (ii) amylase; (iii) amphiphilic cleaning polymer; or (iv) mixtures thereof.

Preferably, the cleaning composition has a pH between 6 and 10, between 6.5 and 8.9, or between 7 and 8, wherein the pH of the cleaning composition is measured as a 10% concentration in demineralized water at 20°C. When liquid, the cleaning composition may be Newtonian or non-Newtonian. In some examples, the liquid laundry detergent composition is non-Newtonian. Without wishing to be bound by theory, a non-Newtonian liquid has properties that differ from those of a Newtonian liquid, more specifically, the viscosity of non-Newtonian liquids is dependent on shear rate, while a Newtonian liquid has a constant viscosity independent of the applied shear rate. The decreased viscosity upon shear application for non-Newtonian liquids is thought to further facilitate liquid detergent dissolution. The liquid laundry detergent composition described herein can have any suitable viscosity depending on factors such as formulated ingredients and purpose of the composition.

### Anionic Surfactant

The detergent composition preferably comprises from 1 to 60 wt% an anionic surfactant. Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, or from 1 to 3, or from 2 to 3. Example alkyl fraction of the alkyl sulphate anionic surfactant are derived from fatty alcohols, oxo-synthesized alcohols, Guerbet alcohols, or mixtures thereof. In some examples, the laundry detergent composition comprises between 10% and 50%, between 15% and 45%, between 20% and 40%, or between 30% and 40% by weight of the laundry detergent composition of the non-soap anionic surfactant. The alkyl sulphate, alkyl alkoxylated sulphate and alkyl benzene sulphonates may be linear or branched, including 2 alkyl substituted or mid chain branched type, substituted or unsubstituted, and may be derived from petrochemical material or biomaterial. Preferably, the branching group is an alkyl. Typically, the alkyl is selected from methyl, ethyl, propyl, butyl, pentyl, cyclic alkyl groups and mixtures thereof. Single or multiple alkyl branches could be present on the main hydrocarbyl chain of the starting alcohol(s) used to produce the sulfated anionic surfactant used in the detergent of the invention. Most preferably the branched sulfated anionic surfactant is selected from alkyl sulfates, alkyl ethoxy sulfates, and mixtures thereof.

In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof are also suitable anionic surfactants.

Other suitable anionic surfactants include alkyl ether carboxylates, typically comprising a C10-C26 linear or branched, preferably C10-C20 linear, most preferably C16-C18 linear alkyl alcohol and from 2 to 20, preferably 7 to 13, more preferably 8 to 12, most preferably 9.5 to 10.5 ethoxylates. The acid form or salt form, such as sodium or ammonium salt, may be used, and the alkyl chain may contain one cis or trans double bond.

Other suitable anionic surfactants are rhamnolipids. The rhamnolipids may have a single rhamnose sugar ring or two rhamnose sugar rings.

The anionic surfactant is preferably added to the detergent composition in the form of a salt. Preferred cations are alkali metal ions, such as sodium and potassium. However, the salt form of the anionic surfactant may be formed in situ by neutralization of the acid form of the surfactant with alkali such as sodium hydroxide or an amine, such as mono-, di-, or tri-ethanolamine. Further preferred agents for neutralizing anionic surfactants in their acid forms include ammonia, amines, oligamines, or alkanolamines. Alkanolamines are preferred. Suitable non-limiting examples including monoethanolamine, diethanolamine, triethanolamine, and other linear or branched alkanolamines known in the art; for example, highly preferred alkanolamines include 2-amino-1-propanol, 1-aminopropanol, monoisopropanolamine, or 1-amino-3-propanol. Amine neutralization may be done to a full or partial extent, e.g. part of the anionic surfactant mix may be neutralized with sodium or potassium and part of the anionic surfactant mix may be neutralized with amines or alkanolamines.

The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof. Preferably the surfactant comprises an anionic and a nonionic surfactant, preferably having a weight ratio of anionic to nonionic of from 30:1 to 1:2, preferably from 20:1 to 2:3 or to 1:1. Preferably the surfactant comprises an anionic and an amphoteric surfactant, most preferably amine oxide, preferably the weight ratio of anionic to amphoteric surfactant is from 100:1 to 5:1, more preferably from 50:1 to 10:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac^{™}, Lutensol^{™} and Pluronic^{™} from BASF, Dehypon^{™} series from Cognis and Genapol^{™} series from Clariant. In some examples, the non-ionic surfactant is selected from alcohol alkoxylate, an oxo-synthesised alcohol alkoxylate, Guerbet alcohol alkoxylates, alkyl phenol alcohol alkoxylates, or a mixture thereof. In some examples, the laundry detergent composition comprises between 0.01% and 10%, between 0.01% and 8%, between 0.1% and 6%, or between 0.15% and 5% by weight of the liquid laundry detergent composition of a non-ionic surfactant.

Suitable amphoteric or zwitterionic surfactants include amine oxides, and/or betaines. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of C1-3 alkyl groups and C1-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Other suitable surfactants include betaines, such as alkyl betaines, alkylamidobetaine, amidazoliniumbetaine, sulfobetaine (INCI sultaines) as well as phosphobetaines. A preferred composition according to the invention comprises from 0.1 to 15 wt%, preferably 0.5 to 5 or 10 wt% amine oxide surfactant.

The cleaning composition preferably comprises from 0.5wt% to about 40wt% of a non-ionic surfactant, preferably 1 to 30 wt% of the composition non-ionic surfactant. Other example weight ratio of anionic surfactant to nonionic surfactant are from 1:2 to 20:1, from 1:1 to 17.5:1, from 2:1 to 15:1, or from 2.5:1 to 13:1. Example weight ratio of linear alkylbenzene sulphonate to alkyl sulphate anionic surfactant are from 1:2 to 9:1, from 1:1 to 7:1, from 1:1 to 5:1, or from 1:1 to 4:1.

In some examples, the cleaning composition comprises between 1.5% and 20%, between 2% and 15%, between 3% and 10%, or between 4% and 8% by weight of the cleaning composition of soap, in some examples a fatty acid salt, in some examples an amine neutralized fatty acid salt, wherein in some examples the amine is an alkanolamine for example selected from monoethanolamine, diethanolamine, triethanolamine or a mixture thereof, in some examples monoethanolamine.

The cleaning composition preferably comprises one or more additional enzymes. Suitable additional enzymes are selected from the group consisting of acyl transferase, alginate lyase, aminopeptidase, amylase, carbohydrase, carboxypeptidase, catalase, cellulase, chitinase, carrageenase, cellobiohydrolase, chondroitinase, cutinase, cyclodextrin endoglucanase, dispersin, endo-beta-1, 4-glucanase, endo-beta-mannanase, esterase, exo-mannanase, fructosidase, glycosyltransferase, esterase, alpha-galactosidase, beta-galactosidase, alpha-glucosidase, beta-glucosidase, galactanases, glucoamylase, hemicellulase, hexosaminidase, hyaluronidase, keratinase, haloperoxidase, hexosaminidase, invertase, laccase, ligninase, lactase, lipase, mannanase, mannosidase, nuclease, oxidase, pectinolytic enzyme, peptidoglutaminase, peroxidase, phytase, polyphenoloxidase, additional proteolytic enzyme (protease), transglutaminase,, lipoxygenases, lysozymes, metalloproteases, nucleases (e.g. DNases and/or RNases), oxidases, oxidoreductases, pectate lyases, pectin acetyl esterases, pectinases, pentosanases, perhydrolases, peroxidases, phenoloxidases, phosphatases, phosphodiesterase, phospholipases, phytases, polygalacturonases, polyesterases, additional proteases, pullulanases, reductase, rhamnogalacturonase, tannase, transglutaminase, xylosidases, xylanase, xyloglucanase, xanthan lyase, xanthanase, xylan acetyl-esterases. Preferably the cleaning composition comprises additional enzyme selected from nuclease, such as DNase or RNase, mannanase, xyloglucanase, xanthan lyase, xanthanase, amylase and mixtures thereof.

Preferably the composition comprises additional enzymes selected from additional protease, amylase, xanthan lyase, xyloglucanase, xanthanase, mannanase, and mixtures thereof.

Preferably the composition comprises an additional protease. In one embodiment, the additional protease is a serine protease. In another embodiment, the additional protease is a metalloprotease, a fungal subtilisin, or an alkaline microbial protease or a trypsin-like protease. Suitable additional proteases include those of animal, vegetable or microbial origin. In some embodiments, the additional protease is a microbial protease. In other embodiments, the additional protease is a chemically or genetically modified mutant. In another embodiment, the additional protease is an alkaline microbial protease or a trypsin-like protease. In other embodiments, the additional protease does not contain cross-reactive epitopes with the subtilisin variant as measured by antibody binding or other assays available in the art. Exemplary alkaline proteases include subtilisins derived from, for example, *Bacillus* (e.g., *B lentus, B gibsonii, B pumilus,* TY-145, Carlsberg, subtilisin 309, subtilisin 147, and subtilisin 168), or fungal origin, such as, for example, those described in US Patent No. 8,362,222. Suitable additional proteases are discussed in more detail below.

Another embodiment is directed to a composition comprising one or more subtilisin variant described herein and one or more lipase. In some embodiments, the composition comprises from about 0.00001% to about 10%, about 0.0001% to about 10%, about 0.001% to about 5%, about 0.001% to about 2%, or about 0.005% to about 0.5% lipase by weight composition.

A still further embodiment is directed to a composition comprising one or more subtilisin variant described herein and one or more amylase. In one embodiment, the composition comprises from about 0.00001% to about 10%, about 0.0001% to about 10%, about 0.001% to about 5%, about 0.001% to about 2%, or about 0.005% to about 0.5% amylase by weight composition.

Yet a still further embodiment is directed to a composition comprising one or more subtilisin variant described herein and one or more cellulase. In one embodiment, the composition comprises from about 0.00001% to about 10%, 0.0001% to about 10%, about 0.001% to about 5%, about 0.001% to about 2%, or about 0.005% to about 0.5% cellulase by weight of composition. Any suitable cellulase may find use in a composition described herein.

An even still further embodiment is directed to a composition comprising one or more subtilisin variant described herein and one or more mannanase. In one embodiment, the composition comprises from about 0.00001% to about 10%, about 0.0001% to about 10%, about 0.001% to about 5%, about 0.001% to about 2%, or about 0.005% to about 0.5% mannanase by weight composition. An exemplary mannanase can be a chemically or genetically modified mutant.

In some embodiments, the cleaning compositions described herein further comprise a suitable pectin degrading enzyme. As used herein, "pectin degrading enzyme(s)" encompass arabinanase (EC 3.2.1.99), galactanases (EC 3.2.1.89), polygalacturonase (EC 3.2.1.15) exo-polygalacturonase (EC 3.2.1.67), exo-poly-alpha-galacturonosidase (EC 3.2.1.82), pectin lyase (EC 4.2.2.10), pectin esterase (EC 3.1.1.11), pectate lyase (EC 4.2.2.2), exo-polygalacturonate lyase (EC 4.2.2.9) and hemicellulases such as endo-1,3-β-xylosidase (EC 3.2.1.32), xylan-1,4-β-xylosidase (EC 3.2.1.37), xanthan lyase, and α-L-arabinofuranosidase (EC 3.2.1.55). Pectin degrading enzymes are natural mixtures of the above-mentioned enzymatic activities. Pectin enzymes therefore include the pectin methylesterases which hydrolyse the pectin methyl ester linkages, polygalacturonases which cleave the glycosidic bonds between galacturonic acid molecules, and the pectin transeliminases or lyases which act on the pectic acids to bring about non-hydrolytic cleavage of α-1,4 glycosidic linkages to form unsaturated derivatives of galacturonic acid.

Suitable pectin degrading enzymes include those of plant, fungal, or microbial origin. In some embodiments, the cleaning compositions described herein further comprise about 0.00001% to about 10%, about 0.0001% to about 10%, about 0.001% to about 5%, about 0.001% to about 2%, or about 0.005% to about 0.5% of pectin degrading enzyme by weight of the composition.

In some other embodiments, the cleaning compositions described herein further comprise a suitable beta-glucanase. Suitable beta-glucanases include, but are not limited to those of plant, fungal, or bacterial origin. Chemically or genetically modified mutants are included in some embodiments. Beta-glucanase as used herein means an endo beta-1 ,4-glucanase activity (e.g. endo- 1 ,4 beta-D-glucanase) that catalyzes the hydrolyses of a beta-1, 4-bonds connecting two glucosyl residues in a beta-glucan. Non-limiting examples of beta-glucanases as defined herein include cellulases (e.g. EC 3.2.1.4, e.g. having endo-cellulase activity on b-1 ,4 linkages between D- glucose units and licheninases (or lichenases) (e.g. EC 3.2.1.73) hydrolysing (1 ,4)-beta-D-glucosidic linkages in beta-D-glucans containing (1 ,3)- and (1 ,4)-bonds. Beta-glucanases (e.g. EC 3.2.1.4) can, for example, perform endohydrolysis of (1 ,4)-beta-D-glucosidic linkages in cellulose, lichenin and cereal beta-D-glucans and will also hydrolyze 1,4-linkages in beta-D-glucans containing 1,3-linkages. Examples of useful beta-glucanases have been described in the Bacillus genus, (e.g. *B agaradhaerens, B akibai, B mojavensis,* WO2021148364).

In some other embodiments, the cleaning compositions described herein further comprise a suitable xyloglucanase. Suitable xyloglucanases include, but are not limited to those of plant, fungal, or bacterial origin. Chemically or genetically modified mutants are included in some embodiments. As used herein, "xyloglucanase(s)" encompass the family of enzymes described by Vincken and Voragen at Wageningen University [Vincken et al (1994) Plant Physiol., 104, 99-107] and are able to degrade xyloglucans as described in Hayashi et al (1989) Annu. Rev. Plant. Physiol. Plant Mol. Biol., 40, 139-168. Vincken et al demonstrated the removal of xyloglucan coating from cellulose of the isolated apple cell wall by a xyloglucanase purified from *Trichoderma viride* (endo-IV-glucanase). This enzyme enhances the enzymatic degradation of cell wall-embedded cellulose and work in synergy with pectic enzymes. Rapidase LIQ+ from DSM contains a xyloglucanase activity. In some embodiments, the cleaning compositions described herein further comprise from about 0.00001% to about 10%, about 0.0001% to about 10%, about 0.001% to about 5%, about 0.001% to about 2%, or about 0.005% to about 0.5% xyloglucanase by weight of the composition. In certain other embodiments, xyloglucanases for specific applications are alkaline xyloglucanases, *i*.*e*., enzymes having an enzymatic activity of at least 10%, at least 25%, or at least 40% of its maximum activity at a pH ranging from 7 to 12. In certain other embodiments, the xyloglucanases are enzymes having a maximum activity at a pH of from about 7.0 to about 12.

A still further embodiment is directed to a composition comprising one or more subtilisin variant described herein and one or more nuclease, such as a DNase or RNase. In one embodiment, the composition comprises from about 0.00001% to about 10%, about 0.0001% to about 10%, about 0.001% to about 5%, about 0.001% to about 2%, or about 0.005% to about 0.5% nuclease by weight composition.

In some other embodiments, the cleaning compositions described herein further comprise a suitable peroxidase/oxidase. Suitable peroxidases/oxidases include those of plant, bacterial or fungal origin. Chemically modified or protein engineered mutants are included. Examples of useful peroxidases include peroxidases from Coprinus, e.g., from C. cinereus, and variants thereof as those described in WO 93/24618, WO 95/10602, and WO 98/15257.

Another embodiment is directed to a composition comprising one or more subtilisin variant described herein, and one or more perhydrolase, such as, for example, is described in WO2005/056782, WO2007/106293, WO 2008/063400, WO2008/106214, and WO2008/106215.

In yet another embodiment, the one or more subtilisin variant described herein and one or more additional enzyme contained in one or more composition described herein may each independently range from 0.001 to about 10% by weight composition, wherein the balance of the cleaning composition is one or more adjunct material.

The additional enzyme(s) may be produced, for example, by a microorganism belonging to the genus *Aspergillus, e.g., Aspergillus aculeatus, Aspergillus awamori, Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum*; *Humicola, e.g., Humicola insolens* or *Humicola lanuginosa*; or *Trichoderma, e.g., Trichoderma harzianum, Trichoderma koningii, Trichoderma longibrachiatum, Trichoderma reesei,* or *Trichoderma viride.*

Preferably the composition comprises a lipase or mixture of more than one lipase, a peroxidase or mixture of more than one peroxidase, one or more amylolytic enzymes, e.g., an alpha-amylase, glucoamylase, maltogenic amylase, and/or a cellulase or mixture thereof.

In general, the properties of the chosen enzyme(s) should be compatible with the selected detergent, (i.e., pH-optimum, compatibility with other enzymatic and non-enzymatic ingredients, etc.), and the enzyme(s) should be present in effective amounts. Where present, the additional enzymes are preferably, each present in the cleaning composition in an amount from at least 0.0001 mg, preferably from about 0.005 to about 10, more preferably from about 0.001 to about 10, especially from about 0.002 to about 5 mg of active further enzyme/ g of composition.

Proteases: The composition of the invention may comprise an additional a protease. A mixture of two or more proteases can contribute to an enhanced cleaning across a broader temperature, cycle duration, and/or substrate range. Suitable additional proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
Additional subtilisins (EC 3.4.21.62), especially those derived from *Bacillus,* such as *Bacillus sp., B. lentus, B. alkalophilus, B. subtilis, B. amyloliquefaciens, B. pumilus, B. gibsonii,* and *B. akibaii* described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569 and WO2016174234. Specifically, mutations S9R, A15T, V66A, A188P, V199I, Q239R, N255D, X9E, X200L, X256E, X9R, X19L, X60D (savinase numbering system); subtilisins from *B. Pumilus* such as the ones described in DE102006022224A1, WO2020/221578, WO2020/221579, WO2020/221580, including variants comprising amino acid substitutions in at least one or more of the positions selected from 9, 130, 133, 144, 224, 252, 271 (BPN' numbering system).
trypsin-type or chymotrypsin-type proteases, such as trypsin (e.g., of porcine or bovine origin), including the Fusarium protease described in WO 89/06270 and the chymotrypsin proteases derived from *Cellumonas* described in WO 05/052161 and WO 05/052146.
metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces spp.* described in WO2014194032, WO2014194054 and WO2014194117; from *Kribella alluminosa* described in WO2015193488; and from *Streptomyces* and *Lysobacter* described in WO2016075078.
protease having at least 90% identity to the subtilase from *Bacillus sp.* TY145, NCIMB 40339, described in WO92/17577 (Novozymes A/S), including the variants of this *Bacillus sp* TY145 subtilase described in WO2015024739, and WO2016066757.

Especially preferred proteases for the cleaning composition of the invention are metalloproteases and/or polypeptides having at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD
(viii) S9R+A15T+V68A+N218D+Q245R

Suitable commercially available protease enzymes include those sold under the trade names Alcalase^{®}, Savinase^{®}, Primase^{®}, Durazym^{®}, Polarzyme^{®}, Kannase^{®}, Liquanase^{®}, Liquanase Ultra^{®}, Savinase Ultra^{®}, Ovozyme^{®}, Neutrase^{®}, Everlase^{®}, Coronase^{®}, Blaze^{®}, Blaze Ultra^{®} and Esperase^{®} by Novozymes A/S (Denmark); those sold under the tradename Maxatase^{®}, Maxacal^{®}, Maxapem^{®}, Properase^{®}, Purafect^{®}, Purafect Prime^{®}, Purafect Ox^{®}, FN3^{®}, FN4^{®}, Excellase^{®}, Ultimase^{®} and Purafect OXP^{®} by Dupont; those sold under the tradename Opticlean^{®} and Optimase^{®} by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and can comprise a further mutation 101E/D, S156D, L262E, 206A/L/S/T/, 209K/V/W, 215W, 216N/S/T; KAP (Bacillus alkalophilus subtilisin with mutations A230V + S256G + S259N) from Kao and Lavergy^{®}, Lavergy^{®} Pro, Lavergy^{®} C Bright from BASF.

Especially preferred for use herein are commercial proteases selected from the group consisting of Properase^{®}, Blaze^{®}, Ultimase^{®}, Everlase^{®}, Savinase^{®}, Excellase^{®}, Blaze Ultra^{®}, BLAP and BLAP variants.

Preferred levels of additional protease in the product of the invention include from about 0.001 to about 10, more preferably from about 0.002 to about 7 and especially from about 0.005 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces*), e.g., from *H. lanuginosa* (*T. lanuginosus*) or from *H. insolens,* a *Pseudomonas lipase,* e.g., from *P. alcaligenes* or *P. pseudoalcaligenes, P. cepacia P. stutzeri, P. fluorescens, Pseudomonas* sp. strain SD 705, *P. wisconsinensis,* a *Bacillus* lipase, e.g., from *B. subtilis* (Dartois et al. (1993), Biochemica et Biophysica Acta, 1131, 253-360), *B. stearothermophilus* or *B. pumilus .*

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot O59952 (derived from *Thermomyces lanuginosus (Humicola lanuginosa)).* Preferred lipases include those sold under the tradenames Lipex^{®}, Lipolex^{®} and Lipoclean^{®}.

Other suitable lipases include: Liprl 139, e.g. as described in WO2013/171241; TfuLip2, e.g. as described in WO2011/084412 and WO2013/033318; Pseudomonas stutzeri lipase, e.g. as described in WO2018228880; *Microbulbifer thermotolerans* lipase, e.g. as described in WO2018228881; *Sulfobacillus acidocaldarius* lipase, e.g. as described in EP3299457; LIP062 lipase e.g. as described in WO2018209026; PinLip lipase e.g. as described in WO2017036901 and Absidia sp. lipase e.g. as described in WO2017005798.

A suitable lipase is a variant of SEQ ID NO:5 comprising:
(a) substitution T231R
   and
(b) substitution N233R or N233C
   and
(c) at least three further substitutions selected from E1C, D27R, N33Q, G38A, F51V, G91Q, D96E, K98L, K98I, D111A, G163K, H198S, E210Q, Y220F, D254S, I255A, and P256T;
where the positions correspond to the positions of SEQ ID NO:5 and wherein the lipase variant has at least 90% but less than 100% sequence identity to the polypeptide having the amino acid sequence of SEQ ID NO: 5 and wherein the variant has lipase activity.

One preferred lipase is a variant of SEQ ID NO: 5 comprising the following substitutions: T231R, N233R, D27R, G38A, D96E, D111A, G163K, D254S and P256T

One preferred lipase is a variant of SEQ ID NO: 5 comprising the following substitutions: T231R, N233R, N33Q, G91Q, E210Q, I255A.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.* disclosed in US 4,435,307 , US 5,648,263 , US 5,691,178, US 5,776,757 and US 5,691,178 .

In one aspect, preferred enzymes include microbial-derived endoglucanases exhibiting endo-beta-1,4-glucanase activity (E.C. 3.2.1.4), preferably selected from the group comprising:
(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 265, 255, 246, 237, 224 and 221 of the mature polypeptide of SEQ ID NO: 2, and t he variant has cellulase activity.;
(b) a glycosyl hydrolase having enzymatic activity towards both xyloglucan and amorphous cellulose substrates, wherein the glycosyl hydrolase is selected from GH families 5, 7, 12, 16, 44 or 74;
(c) a glycosyl hydrolase having a sequence of at least 90%, 94%, 97% and even 99% identity to the amino acid sequence SEQ ID NO:3 in WO09/148983;
(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;
(e) and mixtures thereof.

Suitable endoglucanases are sold under the tradenames Celluclean^{®} and Whitezyme^{®} (Novozymes A/S, Bagsvaerd, Denmark). Examples include Celluclean^{®} 5000L, Celluclean^{®} Classic 400L, Celluclean^{®} Classic 700T, Celluclean^{®} 4500T, Whitezyme^{®} 1.5T, Whitezyme^{®} 2.0L.

Other commercially available cellulases include Celluzyme^{®}, Carezyme^{®}, Carezyme^{®} Premium (Novozymes A/S), Clazinase^{®}, Puradax HA^{®}, Revitalenz^{®} 1000, Revitalenz^{®} 2000 (Genencor International Inc.), KAC-500(B)^{®} (Kao Corporation), Biotouch^{®} FCL, Biotouch^{®} DCL, Biotouch^{®} DCC, Biotouch^{®} NCD, Biotouch^{®} FCC, Biotouch^{®} FLX1 (AB Enzymes)

Suitable glucanases include endo-β-1,3-glucanases, preferably from E.C. class 3.2.1.39, preferably obtained from *Paenibacillus sp, Zobellia galactanivorans, Thermotoga petrophila* or *Trichoderma sp* micro-organism, preferably *Paenibacillus sp* or *Zobellia galactanivorans,* most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.
(b) variants exhibiting at least 85%, preferably 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.
(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, I206, M208, D209, E212, V213, V214, N214, L217, R218, N219, V222, T225, T227, G229, I235, K242, Y243, S244, F245, T246, I250, S255, A256, H286, V291, T316, V317, V318, N417, T418, A419, H420, P421, I428, M429, F440, R443, N444, K445, Q448, S451, A465, N470, S472.
(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.
(e) variants described in WO10/115021, especially those exhibiting at least 75%, or at least 85% or at least 90% or at least 95% with SEQ ID NO:2 in WO10/115021, the alpha-amylase derived from *Bacillus sp.* TS-23.
(f) variants exhibiting at least 89% identity with SEQ ID NO:1 in WO2016091688, especially those comprising deletions at positions H183+G184 and additionally one or more mutations at positions 405, 421, 422 and/or 428.
(g) variants described in WO2014099523, especially those exhibiting at least 60% amino acid sequence identity with the "PcuAmyl α-amylase" from *Paenibacillus curdlanolyticus* YK9 (SEQ ID NO:3 in WO2014099523).
(h) variants described in WO2014099523, especially those exhibiting at least 60% amino acid sequence identity with the "CspAmy2 amylase" from *Cytophaga sp.* (SEQ ID NO:1 & 6 in WO2014164777. Especially those comprising one of more of the following deletions and/or mutations based on SEQ ID NO:1 in WO2014164777: R178*, G179*, T38N, N88H, N126Y, T129I, N134M, F153W, L171R, T180D, E187P, I203Y, G476K, G477E, Y303D.
(i) variants exhibiting at least 85% identity with AmyE from *Bacillus subtilis* (SEQ ID NO:1 in WO2009149271).
(j) variants exhibiting at least 90% identity with the wild-type amylase from Bacillus sp. KSM-K38 with accession number AB051102.
(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H*, N54S, V56T, K72R, G109A, F113Q, R116Q, W167F, Q172G, A174S, G184T, N195F, V206L, K391A, P473R, G476K.
(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO:11 is substituted. Preferably, the methionine at this position is substituted with threonine or leucine, preferably leucine.

Suitable commercially available alpha-amylases include DURAMYL^{®}, LIQUEZYME^{®}, TERMAMYL^{®}, TERMAMYL ULTRA^{®}, NATALASE^{®}, SUPRAMYL^{®}, STAINZYME^{®}, STAINZYME PLUS^{®}, FUNGAMYL^{®}, ATLANTIC^{®}, ACHIEVE ALPHA^{®}, AMPLIFY^{®} PRIME, INTENSA^{®} and BAN^{®} (Novozymes A/S, Bagsvaerd, Denmark), KEMZYM^{®} AT 9000 Biozym Biotech Trading GmbH Wehlistrasse 27b A-1200 Wien Austria, RAPIDASE^{®} , PURASTAR^{®}, ENZYSIZE^{®}, OPTISIZE HT PLUS^{®}, POWERASE^{®}, PREFERENZ S^{®} series (including PREFERENZ S1000^{®} and PREFERENZ S2000^{®} and PURASTAR OXAM^{®} (DuPont., Palo Alto, California) and KAM^{®} (Kao, 14-10 Nihonbashi Kayabacho, 1-chome, Chuoku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from *C*. *cinereus,* and variants thereof as those described in WO 93/24618, WO 95/10602, and WO 98/15257.

Commercially available peroxidases include GUARDZYME^{®} (Novozymes A/S).

Pectate lyase: Suitable pectate lyases include those sold under the tradenames Pectawash^{®}, Pectaway^{®}, X-Pect^{®}, (all Novozymes A/S, Bagsvaerd, Denmark) Preferenz^{®} F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway^{®} (Novozymes A/S) such as Mannaway^{®} 200L and Mannaway Evity 4.0T Other commercially available mannanases include Effectenz^{®} M1000, Mannastar^{®} 375, Preferenz M100 and Purabrite^{®} (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.

Nucleases: Preferably the composition comprises a nuclease enzyme such as a RNase or DNase or mixtures thereof. The nuclease enzyme is an enzyme capable of cleaving the phosphodiester bonds between the nucleotide sub-units of nucleic acids. The nuclease enzyme herein is preferably a deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic activity. Thus, it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof.

DNase: Suitable DNases include wild-types and variants of DNases defined by SEQ ID NOS: 1, 2, 3, 4, 5, 6, 7, 8 and 9 in WO2017162836 (Novozymes), and SEQ ID NOS: 1, 2, 3, 4, 5, 6, 7, 8, 9, 10, 11, 12, 13, 14, 15, 16, 17, 18, 19, 20, 21, 22, 23, 24, 25, 28, 29, 30, 31, 32, 33, 34, 35, 36, 37, 38 39, 40, 41, 42, 43, 44, 45, 46, 47, 48, 49 50, 51, 52, 53, and 54 in WO2018108865, and variants of the Bacillus cibi DNase including those described in WO2018011277 (Novozymes), incorporated herein by reference. Preferred DNases are as claimed in EP3476935A.

RNase: suitable RNases include wild-types and variants of DNases defined by SEQ ID NOS: 3, 6, 9, 12, 15, 57, 58, 59, 60, 61, 62, 63, 64, 65, 66, 67, 72 and 73 in WO2018178061 (Novozymes), and SEQ ID NOS: 86, 87, 88, 89, 90, 91, 92, 93, 94, 95, 96, 97, 98, 99, 100, 101, 102, 103 and 104 in WO2020074499 (Novozymes), incorporated herein by reference.

Hexosaminidase: The composition preferably additionally comprises one or more hexosaminidase enzymes. The term hexosaminidase includes "dispersin" and the abbreviation "Dsp", which means a polypeptide having hexosaminidase activity. Suitable enzymes are found in EC 3.2.1.- that catalyze the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 are preferred, especially the variants with improved thermostability disclosed in that publication.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

Galactanase: Preferably the composition comprises a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The additional enzyme(s) may be included in the detergent composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in EP1794275 or EP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint^{®} (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint^{®} Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenylmethane polymeric colourants, alkoxylated thiophene polymeric colourants, and mixtures thereof. The compositions may comprise aesthetic dyes/colourants. Aesthetic colorants include Liquitint^{®} Blue AH, Liquitint^{®} Blue BB, Liquitint^{®} Blue 275, Liquitint^{®} Blue 297, Liquitint^{®} Blue BB, Cyan 15, Liquitint^{®} Green 101, Liquitint^{®} Orange 272, Liquitint^{®} Orange 255, Liquitint^{®} Pink AM, Liquitint^{®} Pink AMC, Liquitint^{®} Pink ST, Liquitint^{®} Violet 129, Liquitint^{®} Violet LS, Liquitint^{®} Violet 291, Liquitint^{®} Yellow FT, Liquitint^{®} Blue Buf, Liquitint^{®} Pink AM, Liquitint^{®} Pink PV, Acid Blue 80, Acid Blue 182, Acid Red 33, Acid Red 52, Acid Violet 48, Acid Violet 126, Acid Blue 9, Acid Blue 1, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Preferred hueing dyes may be provided by the incorporation of a leuco compound (leuco dye) comprising a leuco moiety, into the composition. These uncoloured dyes undergo a transformation (i) in the composition over time, (ii) in the wash liquor; and/or (iii) post-wash when deposited onto washed fabric, to generate the coloured form of the dye providing fabric shading and an increased whiteness appearance. In one aspect, the leuco moiety is selected from the group consisting of diarylmethane leuco moieties, triarylmethane leuco moieties, oxazine moieties, thiazine moieties, hydroquinone moieties, and arylaminophenol moieties. The leuco compound may comprise a leuco moiety and an alkyleneoxy moiety covalently bound to the leuco moiety, wherein the alkyleneoxy moiety comprises at least one ethylene oxide group, preferably the alkylene oxide moiety also comprises at least one propylene oxide group. In one aspect, preferred leuco compounds include those conforming to the structure of Formula (CVIII), 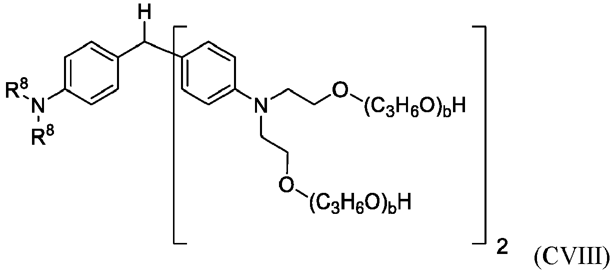 wherein R⁸ is H or CH₃ and each index b is independently on average about 1 to 2. Other suitable leuco dyes are disclosed in US. Patent Nos. 10,377,976, 10,377,977, 10,351,709, 10,385,294, 10,472,595, 10,479,961, 10,501,633, 10,577,570, 10,590,275, 10,633,618, 10,647,854, and 10,676,699, incorporated in their entirety herein by reference.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The detergent composition may further contain builders, such as builders based on carbonate, bicarbonate or silicates which may be Zeolites, such as Zeolite A, Zeolite MAP (Maximum Aluminium type P). Zeolites, useable in laundry preferably has the formula Na₁₂(AlO₂)₁₂(SiO₂)₁₂·27H₂O and the particle size is usually between 1-10 µm for zeolite A and 0.7-2 um for zeolite MAP. Other builders are Sodium metasilicate (Na₂SiO₃ · *n*H₂O or Na₂Si₂O₅ · *n* H₂O) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. However, the compositions of the invention are particularly suitable to enable a reduction in enzyme stabilizer. In particular, the cleaning composition of the invention may be a boron-free cleaning composition. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof. The cleaning compositions of the invention have been found to enable the reduction or omission of boron-containing protease stabilisers such as borate salts and phenyl boronic acid and derivatives thereof. Therefore, in a preferred embodiment the cleaning composition comprises less than 1.0% boron, or less than 0.5 wt% boron or more preferably less than 0.2 wt% boron. Most preferably the composition is free of boron-containing compounds, i.e. no boron-containing compounds have been added. In other embodiments, the composition is a composition free or substantially-free of enzyme stabilizers or peptide inhibitors.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof.

Amphiphilic cleaning polymers: Preferably, the composition comprises an amphiphilic cleaning polymer. Preferred suitable polymers are compounds having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof.

Amphiphilic alkoxylated grease cleaning polymers for use in the present invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block. Preferably they are present in an amount from 0.1 to 8 wt%, more preferably from 0.5 to 5 wt%.

The core structure may comprise a polyalkylenimine structure comprising, in condensed form, repeating units of formulae (I), (II), (III) and (IV): 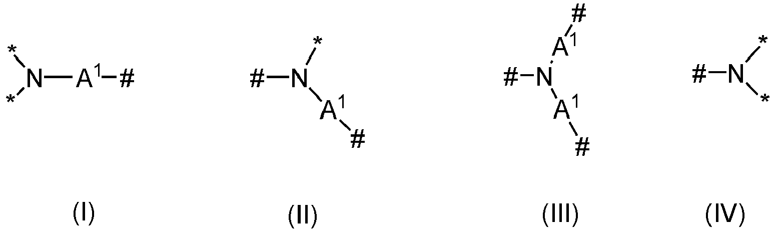 wherein # in each case denotes one-half of a bond between a nitrogen atom and the free binding position of a group A¹ of two adjacent repeating units of formulae (I), (II), (III) or (IV); * in each case denotes one-half of a bond to one of the alkoxylate groups; and A¹ is independently selected from linear or branched C₂-C₆-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b), 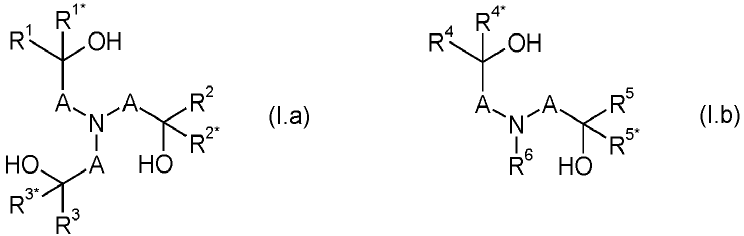 wherein A are independently selected from C₁-C₆-alkylene; R¹, R¹*, R², R²*, R³, R³*, R⁴, R⁴*, R⁵ and R⁵* are independently selected from hydrogen, alkyl, cycloalkyl or aryl, wherein the last three mentioned radicals may be optionally substituted; and R⁶ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V) 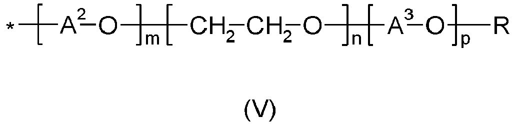 wherein * in each case denotes one-half of a bond to the nitrogen atom of the repeating unit of formula (I), (II) or (IV); A² is in each case independently selected from 1,2-propylene, 1,2-butylene and 1,2-isobutylene; A³ is 1,2-propylene; R is in each case independently selected from hydrogen and C₁-C₄-alkyl; m has an average value in the range of from 0 to about 2; n has an average value in the range of from about 20 to about 50; and p has an average value in the range of from about 10 to about 50.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers having a structure as defined by one of the following structures (I), (II) or (III):
(I) -[(OCHR¹-CHR²)ₐ-O-OC-Ar-CO-]_{d}
(II) -[(OCHR³-CHR⁴)_{b}-O-OC-sAr-CO-]ₑ
(III) -[(OCHR⁵-CHR⁶)_{c}-OR⁷]_{f}
wherein:
a, b and c are from 1 to 200;
d, e and f are from 1 to 50;
Ar is a 1,4-substituted phenylene;
sAr is 1,3-substituted phenylene substituted in position 5 with SO₃Me;
Me is Na, Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or mixtures thereof;
R¹, R², R³, R⁴, R⁵ and R⁶ are independently selected from H or C₁-C₁₈ n- or iso-alkyl; and
R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

The composition preferably comprises one or more organic acids selected from the group consisting of acetic acid, adipic acid, aspartic acid, carboxymethyloxymalonic acid, carboxymethyloxysuccinic acid, citric acid, formic acid, glutaric acid, hydroxyethyliminodiacetic acid, iminodiacetic acid, lactic acid, maleic acid, malic acid, malonic acid, oxydiacetic acid, oxydisuccinic acid, succinic acid, sulfamic acid, tartaric acid, tartaric-disuccinic acid, tartaric-monosuccinic acid, or mixtures thereof. Preferably, the detergent composition comprises an organic acid selected from the group consisting of acetic acid, lactic acid, and citric acid.

The composition preferably comprises an amine. Non-limiting examples of amines include, but are not limited to, etheramines, cyclic amines, polyamines, oligoamines (e.g., triamines, diamines, pentamines, tetraamines), or combinations thereof. The compositions described herein may comprise an amine selected from the group consisting of oligoamines, etheramines, cyclic amines, and combinations thereof. In some aspects, the amine is not an alkanolamine. In some aspects, the amine is not a polyalkyleneimine.

Examples of suitable oligoamines include Preferably the composition comprises oligoamines. Suitable oligoamines according to the present disclosure may include diethylenetriamine (DETA), 4-methyl diethylenetriamine (4-MeDETA), dipropylenetriamine (DPTA), 5-methyl dipropylenetriamine (5-MeDPTA), triethylenetetraamine (TETA), 4-methyl triethylenetetraamine (4-MeTETA), 4,7-dimethyl triethylenetetraamine (4,7-Me2TETA), 1,1,4,7,7-pentamethyl diethylenetriamine (M5-DETA), tripropylenetetraamine (TPTA), tetraethylenepentaamine (TEPA), tetrapropylenepentaamine (TPPA), pentaethylenehexaamine (PEHA), pentapropylenehexaamine (PPHA), hexaethyleneheptaamine (HEHA), hexapropyleneheptaamine (HPHA), N,N'-Bis(3-aminopropyl)ethylenediamine, 1,1,4,7,7-pentamethyl diethylenetriamine (M5-DETA), dipropylenetriamine (DPTA) or mixtures thereof most preferably diethylenetriamine (DETA). DETA may be preferred due to its low molecular weight and/or relatively low cost to produce.

The oligoamines of the present disclosure may have a molecular weight of between about 100 to about 1200 Da, or from about 100 to about 900 Da, or from about 100 to about 600 Da, or from about 100 to about 400 Da, preferably between about 100 Da and about 250 Da, most preferably between about 100 Da and about 175 Da, or even between about 100 Da and about 150 Da. For purposes of the present disclosure, the molecular weight is determined using the free base form of the oligoamine.

Etheramines: The cleaning compositions described herein may contain an etheramine. The cleaning compositions may contain from about 0.1% to about 10%, or from about 0.2% to about 5%, or from about 0.5% to about 4%, by weight of the composition, of an etheramine.

The etheramines of the present disclosure may have a weight average molecular weight of less than about grams/mole 1000 grams/mole, or from about 100 to about 800 grams/mole, or from about 200 to about 450 grams/mole, or from about 290 to about 1000 grams/mole, or from about 290 to about 900 grams/mole, or from about 300 to about 700 grams/mole, or from about 300 to about 450 grams/mole. The etheramines of the present invention may have a weight average molecular weight of from about 150, or from about 200, or from about 350, or from about 500 grams/mole, to about 1000, or to about 900, or to about 800 grams/mole.

Alkoxylated phenol compound: The cleaning compositions of the present disclosure may include an alkoxylated phenol compound. The alkoxylated phenol compound may be selected from the group consisting of an alkoxylated polyaryl phenol compound, an alkoxylated polyalkyl phenol compound, and mixtures thereof. The alkoxylated phenol compound may be an alkoxylated polyaryl phenol compound. The alkoxylated phenol compound may be an alkoxylated polyalkyl phenol compound.

The alkoxylated phenol compound may be present in the cleaning composition at a level of from about 0.2% to about 10%, or from about 0.5% to about 5%, by weight of the cleaning composition.

The alkoxylated phenol compound may have a weight average molecular weight between 280 and 2880.

The composition may optionally contain an anti-oxidant present in the composition from about 0.001 to about 2% by weight. Preferably the antioxidant is present at a concentration in the range 0.01 to 0.08% by weight. Mixtures of anti-oxidants may be used.

Anti-oxidants are substances as described in Kirk-Othmer (Vol. 3, page 424) and In Ullmann's Encyclopedia (Vol. 3, page 91).

One class of anti-oxidants used in the present invention is alkylated phenols, having the general formula: 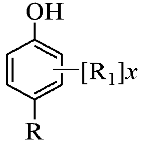 wherein R is C₁-C₂₂ linear or branched alkyl, preferably methyl or branched C₃-C₆ alkyl, C₁-C₆ alkoxy, preferably methoxy; R₁ is a C₃-C₆ branched alkyl, preferably tert-butyl; x is 1 or 2. Hindered phenolic compounds are a preferred type of alkylated phenols having this formula.

Examples of such hindered phenol antioxidants may include, but are not limited to: 2,6-bis(1-methylpropyl)phenol; 2,6-bis(1,1-dimethylethyl)-4-methyl-phenol (also known as hydroxy butylated toluene, "BHT"); 2-(1,1-dimethylethyl)-1,4-benzenediol; 2,4-bis(1,1-dimethylethyl)-phenol; 2,6-bis(1,1-dimethylethyl)-phenol; 3,5-bis(1,1-dimethylethyl)-4-hydroxybenzene propanoic acid, methyl ester; 2-(1,1-dimethylethyl)-4-methylphenol; 2-(1,1-dimethylethyl)-4,6-dimethyl-phenol; 3,5-bis(1,1-dimethylethyl)-4-hydroxybenzenepropanoic acid, 1,1'-[2,2-bis[[3-[3,5-bis(1,1-dimethylethyl)-4-hydroxyphenyl]-1-oxopropoxy]methyl]-1,3-propanediyl] ester; 3,5-bis(1,1-dimethylethyl)-4-hydroxybenzenepropanoic acid, octadecyl ester; 2,2'-methylenebis[6-(1,1-dimethylethyl)-4-methylphenol; 2-(1,1-dimethylethyl)-phenol; 2,4,6-tris(1,1-dimethylethyl)-phenol; 4,4'-methylenebis[2,6-bis(1,1-dimethylethyl)-phenol; 4,4',4"-[(2,4,6-trimethyl-1,3,5-benzenetriyl)tris(methylene)]tris[2,6-bis(1,1-dimethylethyl)-phenol]; N,N'-1,6-hexanediylbis[3,5-bis(1,1-dimethylethyl)-4-hydroxybenzenepropanamide; 3,5-bis(1,1-dimethylethyl)-4-hydroxy benzoic acid, hexadecyl ester; P-[[3,5-bis(1,1-dimethylethyl)-4-hydroxyphenyl]methylphosphonic acid, diethyl ester; 1,3,5-tris[[3,5-bis(1,1-dimethylethyl)-4-hydroxyphenyl]methyl]-1,3,5-Triazine-2,4,6(1H,3H,5H)-trione; 3,5-bis(1,1-5 dimethylethyl)-4-hydroxybenzenepropanoic acid, 2-[3-[3,5-bis(1,1-dimethylethyl)-4-hydroxyphenyl]-1-oxopropyl]hydrazide; 3-(1,1-dimethyl ethyl)-4-hydroxy-5-methylbenzenepropanoic acid, 1,1'-[1,2-ethanediylbis(oxy-2,1-ethanediyl)] ester; 4-[(dimethylamino)methyl]-2,6-bis(1,1-dimethylethyl)phenol; 4-[[4,6-bis(octylthio)-1,3,5-triazin-2-yl]amino]-2,6-bis(1,1-dimethylethyl)phenol; 3,5-bis(1,1-dimethylethyl)-4-hydroxy benzene propanoic acid, 1,1'-(thiodi-2,1-ethanediyl) ester; 3,5-bis(1,1-dimethylethyl)-4-hydroxybenzoic acid, 2,4-bis(1,1-dimethylethyl)phenyl ester; 3,5-bis(1,1-dimethylethyl)-4-hydroxybenzenepropanoic acid, 1,1'-(1,6-hexanediyl)ester; 3-(1,1-dimethylethyl)-4-hydroxy-5-methylbenzenepropanoic acid, 1,1'-[2,4,8,10-tetraoxaspiro[5.5]undecane-3,9-diylbis(2,2-dimethyl-2,1-ethanediyl)] ester; 3-(1,1-dimethylethyl)-b-[3-(1,1-dimethylethyl)-4-hydroxy phenyl]-4-hydroxy-b-methylbenzenepropanoic acid, 1,1'-(1,2-ethanediyl) ester; 2-[[3,5-bis(1,1-dimethylethyl)-4-hydroxyphenyl]methyl]-2-butylpropanedioic acid, 1,3-bis(1,2,2,6,6-pentamethyl-4-piperidinyl) ester; 3,5-bis(1,1-dimethylethyl)-4-hydroxybenzenepropanoic acid, 1-[2-[3-[3,5-bis(1,1-dimethylethyl)-4-hydroxyphenyl]-1-oxopropoxy]ethyl]-2,2,6,6-tetramethyl-4-piperidinyl ester; 3,4-dihydro-2,5,7,8-tetramethyl-2-[(4R,8R)-4,8,12-trimethyltridecyl]-(2R)-2H-1-benzopyran-6-ol; 2,6-dimethylphenol; 2,3,5-trimethyl-1,4-benzenediol; 2,4,6-trimethylphenol; 2,3,6-trimethylphenol; 4,4'-(1-methylethylidene)-bis[2,6-dimethylphenol]; 1,3,5-tris[[4-(1,1-dimethylethyl)-3-hydroxy-2,6-dimethylphenyl]methyl]-1,3,5-triazine-2,4,6(1H,3H,5H)-trione; 4,4'-methylenebis[2,6-dimethylphenol]; and mixtures thereof.

Preferably, the hindered phenol antioxidant comprises at least one phenolic -OH group having at least one C3-C6 branched alkyl at a position ortho to said at least one phenolic -OH group. More preferably, the hindered phenol antioxidant is an ester of 3,5-bis(1,1-dimethylethyl)-4-hydroxy-benzenepropanoic acid, and most preferably a C1-C22 linear alkyl ester of 3,5-bis(1,1-dimethylethyl)-4-hydroxy-benzenepropanoic acid. Commercially available C1-C22 linear alkyl esters of 3,5-bis(1,1-dimethylethyl)-4-hydroxy-benzenepropanoic acid include RALOX^{®} from Raschig USA (Texas, USA), which is a methyl ester of 3,5-bis(1,1-dimethylethyl)-4-hydroxybenzenepropanoic acid, and TINOGARD^{®} TS from BASF (Ludwigshafen, Germany), which is an octadecyl ester of 3,5-bis(1,1-dimethylethyl)-4-hydroxy-benzenepropanoic acid.

Furthermore, the anti-oxidant used in the composition may be selected from the group consisting of α-, β-, γ-, δ--tocopherol, ethoxyquin, 2,2,4-trimethyl-1,2-dihydroquinoline, 2,6-di-tert-butyl hydroquinone, tert-butyl hydroxyanisole, lignosulphonic acid and salts thereof, and mixtures thereof. It is noted that ethoxyquin (1,2-dihydro-6-ethoxy-2,2,4-trimethylquinoline) is marketed under the name Raluquin^{™} by the company Raschig^{™}.

Other types of anti-oxidants that may be used in the composition are 6-hydroxy-2,5,7,8-tetramethylchroman-2-carboxylic acid (Trolox^{™}) and 1,2-benzisothiazoline-3-one (Proxel GXL^{™}).

A further class of anti-oxidants which may be suitable for use in the composition is a benzofuran or benzopyran derivative having the formula: 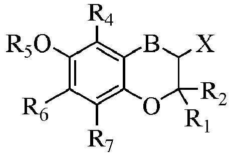 wherein R₁ and R₂ are each independently alkyl or R₁ and R₂ can be taken together to form a C₅-C₆ cyclic hydrocarbyl moiety; B is absent or CH₂; R₄ is C₁-C₆ alkyl; R₅ is hydrogen or - C(O)R₃ wherein R₃ is hydrogen or C₁-C₁₉ alkyl; R₆ is C₁-C₆ alkyl; R₇ is hydrogen or C₁-C₆ alkyl; X is -CH₂OH, or -CH₂A wherein A is a nitrogen comprising unit, phenyl, or substituted phenyl. Preferred nitrogen comprising A units include amino, pyrrolidino, piperidino, morpholino, piperazino, and mixtures thereof. ). The cleaning compositions of the present disclosure may comprise tannins selected from the group consisting of gallotannins, ellagitannins, complex tannins, condensed tannins, and combinations thereof

Bleaching system: The composition may contain a bleaching system, for example comprising a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; oxidized alpha-1,3-glucan; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

Solvents: When the composition is in a liquid form, it typically comprises less than 15%, or less than 12% by weight of the liquid laundry detergent composition of water. In some examples, the laundry detergent composition is a liquid laundry detergent composition comprising a non-aqueous solvent selected from 1,2-propanediol, dipropylene glycol, tripropyleneglycol, glycerol, sorbitol, polyethylene glycol or a mixture thereof. In some examples, the liquid laundry detergent composition comprises between 10% and 40%, or between 15% and 30% by weight of the liquid laundry detergent composition of the non-aqueous solvent.

In some embodiments, the cleaning compositions comprising one or more protease variant described herein is a liquid laundry detergent composition containing sulfite radical scavengers, protease stabilizers/inhibitors or combinations thereof (WO2022/157311).

In some embodiments, the cleaning composition comprising one or more protease variant described herein is a liquid laundry detergent composition as described in US20210317387A1, WO2021/219296, WO2021/127662, WO2021/041685, US11208619, US20220186144, WO2022/043045, WO2022/043138, WO2023/117494, WO2023/088776, WO2023/227332, and WO2024/002922.

In some embodiments, the cleaning composition comprising one or more protease variant described herein is a liquid laundry detergent composition comprising dispersing variants, such as but limiting to a liquid laundry detergent composition described in US20210317387A1.

In some embodiments, the cleaning composition comprising one or more protease variant described herein is a liquid laundry detergent composition is a highly alkaline textile washing agent, such as but limiting to a liquid laundry detergent composition described in WO2021/219296.

In some embodiments, the cleaning composition comprising one or more protease variant described herein is a liquid laundry detergent composition is a low density unit dose detergent with encapsulated fragrance, such as but limiting to a detergent composition described in WO2021/127662.

In some embodiments, the cleaning composition comprising one or more protease variant described herein is a liquid laundry detergent composition containing polyethylene glycol and an organic acid, such as but limiting to, a detergent composition described in WO2021/041685.

In some embodiments, the cleaning composition comprising one or more protease variant described herein is a detergent composition containing polyethylene glycol and an organic acid, such as but limiting to, a detergent composition described in WO2021/041685.

In some embodiments, the cleaning composition comprising one or more protease variant described herein is a detergent composition with effect on protein stains, such as but limiting to, a detergent composition described in US11208619.

In some embodiments, the cleaning composition comprising one or more protease variant described herein is a detergent composition containing soil release polymers, such as but limiting to, a detergent composition described in US20220186144.

Preferably the composition is substantially-free of boron i.e. contain, that contain trace amounts of boron, for example, less than about 1000 ppm (1mg/kg or liter equals 1 ppm), less than about 100 ppm, less than about 50 ppm, less than about 10 ppm, or less than about 5 ppm, or less than about 1 ppm, perhaps from other compositions or detergent constituents.

The cleaning adjunct material may comprise a biopolymer. In some instances, the biopolymer is a glucan. In further instances, the glucan is a alpha-1,6-glucan or an alpha-glucan derivative. The terms "alpha-1,6-glucan", "poly alpha-1,6-glucan", "alpha-1,6-glucan polymer", "dextran", and the like herein refer to a water-soluble alpha-glucan comprising glucose monomeric units linked together by glycosidic linkages, wherein at least about 40% of the glycosidic linkages are alpha-1,6. Alpha-1,6-glucan in some aspects comprises about, or at least about, 90%, 95%, or 100% alpha-1,6 glycosidic linkages. Other linkages that can be present in alpha-1,6-glucan include alpha-1,2, alpha-1,3, and/or alpha-1,4 linkages.

Dextran herein can be as disclosed (e.g., molecular weight, linkage/branching profile, production method), for example, in U.S. Patent Appl. Publ. Nos. 2016/0122445, 2017/0218093, 2018/0282385, 2020/0165360, or 2019/0185893, which are each incorporated herein by reference. In some aspects, a dextran for ester derivatization can be one produced in a suitable reaction comprising glucosyltransferase (GTF) 0768 (SEQ ID NO:1 or 2 of US2016/0122445), GTF 8117, GTF 6831, or GTF 5604 (these latter three GTF enzymes are SEQ ID NOs:30, 32 and 33, respectively, of US2018/0282385), or a GTF comprising an amino acid sequence that is at least 90%, 91%, 92%, 93%, 94%, 95%, 96%, 97%, 98%, or 99% identical to the amino acid sequence of GTF 0768, GTF 8117, GTF 6831, or GTF 5604.

Dextran herein can have alpha-1,2, alpha-1,3, and/or alpha-1,4 branches, for example. In some aspects, about, at least about, or less than about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45%, or 50% of all the glycosidic linkages of a branched dextran are alpha-1,2, alpha-1,3, and/or alpha-1,4 glycosidic branch linkages. Such branches typically are mostly (>90% or >95%), or all (100%), a single glucose monomer in length. In some aspects, dextran with alpha-1,2-branching can be produced enzymatically according to the procedures in U.S. Patent Appl. Publ. Nos. 2017/0218093 or 2018/0282385 (both incorporated herein by reference) where, for example, an alpha-1,2-branching enzyme such as GTFJ18T1 or GTF9905 can be added during or after the production of the dextran. In some aspects, any other enzyme known to produce alpha-1,2-branching can be used. Dextran with alpha-1,3-branching can be prepared, for example, as disclosed in Vuillemin et al. (2016, J. Biol Chem. 291:7687-7702) or Int. Patent Appl. Publ. No. WO2021/007264, which are incorporated herein by reference.

Terms used herein regarding "esters" (e.g., alpha-glucan ester derivative) can be as disclosed, for example, in U.S. Patent Appl. Publ. No. 2014/0187767, 2018/0155455, or 2020/0308371, or Int. Patent Appl. Publ. No. WO2021/252575, which are each incorporated herein by reference. The terms "alpha-glucan ester derivative", "alpha-glucan ester compound", "alpha-glucan ester" and the like are used interchangeably herein. An alpha-glucan ester derivative herein is an alpha-glucan that has been esterified with one or more organic groups (e.g., hydrophobic organic groups) such that the derivative has a degree of substitution (DoS) with one or more organic groups of up to about 3.0. An alpha-glucan ester derivative is termed an "ester" herein by virtue of comprising the substructure C_{G}OCOC, where "C_{G}" represents a carbon atom of a monomeric unit (e.g., glucose) of the alpha-glucan ester derivative (where such carbon atom was bonded to a hydroxyl group [OH] in the alpha-glucan precursor of the ester), and where "CO-C" is comprised in the acyl group. An example of an alpha-glucan ester derivative herein is benzoyl alpha-glucan.

Hydrophobic acyl groups of an alpha-glucan ester derivative herein can be as disclosed, for example, in U.S. Patent Appl. Publ. Nos. 2014/0187767, 2018/0155455, or 2020/0308371, or International Patent Appl. Publ. No. WO2021/252575, which are each incorporated herein by reference.

Terms used herein regarding "ethers" (e.g., alpha-glucan ether derivative) can be as disclosed, for example, in U.S. Patent Appl. Publ. Nos. 2016/0311935, 2018/0237816, or 2020/0002646, or International Pat. Appl. Publ. Nos. WO2021/257786 or WO2021/252569, which are each incorporated herein by reference. The terms "alpha-glucan ether derivative", "alpha-glucan ether compound", "alpha-glucan ether", and the like are used interchangeably herein. An alpha-glucan ether derivative herein is alpha-glucan that has been etherified with one or more organic groups (e.g., charged organic group such as cationic group) such that the derivative has a DoS with one or more organic groups of up to about 3.0. An alpha-glucan ether derivative is termed an "ether" herein by virtue of comprising the substructure C_{G}OC, where "C_{G}" represents a carbon atom of a monomeric unit (typically glucose) of the alpha-glucan ether derivative (where such carbon atom was bonded to a hydroxyl group [OH] in the alpha-glucan precursor of the ether), and where "C" is a carbon atom of an organic group.

An ether derivative of an alpha-glucan of the present disclosure can be substituted with at least one positively charged organic group herein that is ether-linked to the alpha-glucan. A positively charged organic group can be, for example, any of those disclosed in U.S. Patent Appl. Publ. Nos. 2016/0311935, 2018/0237816, or 2020/0002646, or International Pat. Appl. Publ. No. WO2021/257786, which are incorporated herein by reference. A positively charged organic group can comprise a substituted ammonium group, for example. Examples of substituted ammonium groups are primary, secondary, tertiary and quaternary ammonium group. One or more positively charged organic groups in some aspects can comprise trimethylammonium hydroxypropyl groups.

An alpha-glucan derivative (e.g., ester or ether) and/or a product comprising such a derivative is biodegradable in some aspects. Such biodegradability can be, for example, as determined by the Carbon Dioxide Evolution Test Method (OECD Guideline 301B, incorporated herein by reference), to be about, at least about, or at most about, 5%, 10%, 15%, 20%, 25%, 30%, 35%, 40%, 45%, 50%, 55%, 60%, 65%, 70%, 75%, 80%, 85%, 90%, 5-60%, 5-80%, 5-90%, 40-70%, 50-70%, 60-70%, 40-75%, 50-75%, 60-75%, 70-75%, 40-80%, 50-80%, 60-80%, 70-80%, 40-85%, 50-85%, 60-85%, 70-85%, 40-90%, 50-90%, 60-90%, or 70-90%, or any value between 5% and 90%, after 15, 30, 45, 60, 75, or 90 days of testing.

In another embodiment, the cleaning composition is a liquid or gel detergent, which is not unit dosed, that may be aqueous, typically containing at least 20% and up to 95% water by weight, such as up to about 70% water by weight, up to about 65% water by weight, up to about 55% water by weight, up to about 45% water by weight, or up to about 35% water by weight. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

The composition may also contain other conventional detergent ingredients such as e.g. fabric conditioners including clays, foam boosters, suds suppressors, anti-corrosion agents, soil-suspending agents, anti-soil re-deposition agents, dyes, bactericides, optical brighteners, hydrotropes, tarnish inhibitors, organic solvents such as ethanol or perfumes.

When the composition is in the form of a unit dose article, typically the water-soluble unit dose article comprises at least one water-soluble film orientated to create at least one unit dose internal compartment, wherein the at least one unit dose internal compartment comprises a detergent composition. In some examples the consumer product comprises a container and at least one water-soluble unit dose article, in some cases at least two water-soluble unit dose articles, in some cases at least 20 water-soluble unit dose articles, in some cases at least 30 water-soluble unit dose articles. A water-soluble unit dose article is in some examples in the form of a pouch. A water-soluble unit dose article comprises in some examples a unitary dose of a composition as a volume sufficient to provide a benefit in an end application. The water-soluble unit dose article comprises in some examples one water-soluble film shaped such that the unit-dose article comprises at least one internal compartment surrounded by the water-soluble film. The at least one compartment comprises a cleaning composition. The water-soluble film is sealed such that the cleaning composition does not leak out of the compartment during storage. However, upon addition of the water-soluble unit dose article to water, the water-soluble film dissolves and releases the contents of the internal compartment into the wash liquor. The unit dose article may comprise more than one compartment, at least two compartments, or at least three compartments, or at least four compartments, or even at least five compartments. The compartments may be arranged in superposed orientation, i.e. one positioned on top of the other. Alternatively, the compartments may be positioned in a side-by-side orientation, i.e. one orientated next to the other. The compartments may be orientated in a 'tyre and rim' arrangement, i.e. a first compartment is positioned next to a second compartment, but the first compartment at least partially surrounds the second compartment, but does not completely enclose the second compartment. Alternatively, one compartment may be completely enclosed within another compartment. In some examples the unit dose article comprises at least two compartments, one of the compartments being smaller than the other compartment. In some examples the unit dose article comprises at least three compartments, two of the compartments may be smaller than the third compartment, and in some examples the smaller compartments being superposed on the larger compartment. The superposed compartments are in some examples orientated side-by-side. In some examples each individual unit dose article may have a weight of between 10g and 40g, or even between 15g and 35g. The water-soluble film may be soluble or dispersible in water. Prior to be being formed into a unit dose article, the water-soluble film has in some examples a thickness of from 20 to 150 micron, in other examples 35 to 125 micron, in further examples 50 to 110 micron, in yet further examples about 76 micron. Example water soluble film materials comprise polymeric materials. The film material can, for example, be obtained by casting, blow-moulding, extrusion or blown extrusion of the polymeric material. In some examples, the water-soluble film comprises polyvinyl alcohol homopolymer or polyvinyl alcohol copolymer, for example a blend of polyvinylalcohol homopolymers and/or polyvinylalcohol copolymers, wherein the polyvinyl alcohol copolymers preferably are selected from sulphonated and carboxylated anionic polyvinylalcohol copolymers especially carboxylated anionic polyvinylalcohol copolymers, for example a blend of a polyvinylalcohol homopolymer and a carboxylated anionic polyvinylalcohol copolymer, alternatively a blend of two or more preferably two polyvinyl alcohol homopolymers. In some examples water soluble films are those supplied by Monosol under the trade references M8630, M8900, M8779, M8310. In some examples the film may be opaque, transparent or translucent. The film may comprise a printed area. The area of print may be achieved using techniques such as flexographic printing or inkjet printing. The film may comprise an aversive agent, for example a bittering agent. Suitable bittering agents include, but are not limited to, naringin, sucrose octaacetate, quinine hydrochloride, denatonium benzoate, or mixtures thereof. Example levels of aversive agent include, but are not limited to, 1 to 5000ppm, 100 to 2500ppm, or 250 to 2000ppm. The water-soluble film or water-soluble unit dose article or both may be coated with a lubricating agent. In some examples, the lubricating agent is selected from talc, zinc oxide, silicas, siloxanes, zeolites, silicic acid, alumina, sodium sulphate, potassium sulphate, calcium carbonate, magnesium carbonate, sodium citrate, sodium tripolyphosphate, potassium citrate, potassium tripolyphosphate, calcium stearate, zinc stearate, magnesium stearate, starch, modified starches, clay, kaolin, gypsum, cyclodextrins or mixtures thereof. In one embodiment, one or more cleaning composition described herein comprises an effective amount of one or more subtilisin variant described herein, alone or in combination with one or more additional enzyme.

The cleaning compositions described herein are typically formulated such that during use in aqueous cleaning operations, the wash water will have a pH of from about 4.0 to about 11.5, or even from about 5.0 to about 11.5, or even from about 5.0 to about 8.0, or even from about 7.5 to about 10.5. Liquid product formulations are typically formulated to have a pH from about 3.0 to about 9.0 or even from about 3 to about 5. Granular laundry products are typically formulated to have a pH from about 8 to about 11. In some embodiments, the cleaning compositions of the present invention can be formulated to have an alkaline pH under wash conditions, such as a pH of from about 8.0 to about 12.0, or from about 8.5 to about 11.0, or from about 9.0 to about 11.0. In some embodiments, the cleaning compositions of the present invention can be formulated to have a neutral pH under wash conditions, such as a pH of from about 5.0 to about 8.0, or from about 5.5 to about 8.0, or from about 6.0 to about 8.0, or from about 6.0 to about 7.5. In some embodiments, the neutral pH conditions can be measured when the cleaning composition is dissolved 1:100 (wt:wt) in de-ionised water at 20°C, measured using a conventional pH meter. Techniques for controlling pH at recommended usage levels include the use of buffers, alkalis, acids, etc., and are well known to those skilled in the art.

Suitable low pH cleaning compositions typically have a neat pH of from about 3.0 to about 5.0 or even from about 3.5 to about 4.5. Low pH cleaning compositions are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine, or hydrochloric acid, to provide such cleaning composition with a neat pH of from about 3.0 to about 5.0. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of the composition wherein the solvent is distilled water. In these embodiments, all pH measurements are taken at 20°C, unless otherwise indicated.

Suitable high pH cleaning compositions typically have a neat pH of from about 9.0 to about 11.0, or even a neat pH of from 9.5 to 10.5. Such cleaning compositions typically comprise a sufficient amount of a pH modifier, such as sodium hydroxide, monoethanolamine, or hydrochloric acid, to provide such cleaning composition with a neat pH of from about 9.0 to about 11.0. Such compositions typically comprise at least one base-stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, one or more subtilisin variant described herein is encapsulated to protect it during storage from the other components in the composition and/or control the availability of the variant during cleaning. In some embodiments, encapsulation enhances the performance of the variant and/or additional enzyme. In some embodiments, the encapsulating material typically encapsulates at least part of the subtilisin variant described herein. Typically, the encapsulating material is water-soluble and/or water-dispersible. In some embodiments, the encapsulating material has a glass transition temperature (Tg) of 0°C or higher. Exemplary encapsulating materials include, but are not limited to, carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, , and combinations thereof. In some embodiments, the encapsulating material is a starch (*See e.g.,* EP0922499, US 4,977,252, US 5,354,559, and US 5,935,826). In some embodiments, the encapsulating material is a microsphere made from plastic such as thermoplastics, acrylonitrile, methacrylonitrile, polyacrylonitrile, polymethacrylonitrile and mixtures thereof. Exemplary commercial microspheres include, but are not limited to EXPANCEL^{®} (Stockviksverken, Sweden); and PM 6545, PM 6550, PM 7220, PM 7228, EXTENDOSPHERES^{®}, LUXSIL^{®}, Q-CEL^{®}, and SPHERICEL^{®} (PQ Corp., Valley Forge, PA).

In some embodiments, one or more composition described herein finds use as a detergent additive, wherein said additive is in a solid or liquid form. Such additive products are intended to supplement and/or boost the performance of conventional detergent compositions and can be added at any stage of the cleaning process. In some embodiments, the density of the laundry detergent composition ranges from about 400 to about 1200 g/liter, while in other embodiments it ranges from about 500 to about 950 g/liter of composition measured at 20°C.

When the cleaning composition is an ADW detergent composition comprising one or more subtilisin variant described herein, the composition preferably comprises two or more non-ionic surfactants selected from ethoxylated non-ionic surfactants, alcohol alkoxylated surfactants, epoxy-capped poly(oxyalkylated) alcohols, and amine oxide surfactants present in amounts from 0-10% by wt; builders in the range of 5-60% by wt. comprising either phosphate (mono-phosphates, di-phosphates, tri-polyphosphates or oligomeric-polyphosphates), sodium tripolyphosphate-STPP or phosphate-free builders (amino acid based compounds, e.g., MGDA (methyl-glycine-diacetic acid) and salts and derivatives thereof, GLDA (glutamic-N,N-diacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), and B-alaninediacetic acid (B-ADA) and their salts), homopolymers and copolymers of poly-carboxylic acids and their partially or completely neutralized salts, monomeric polycarboxylic acids and hydroxycarboxylic acids and their salts in the range of 0.5-50% by wt; sulfonated/carboxylated polymers (provide dimensional stability to the product) in the range of about 0.1 to about 50% by wt; drying aids in the range of about 0.1 to about 10% by wt (selected from polyesters, especially anionic polyesters optionally together with further monomers with 3- 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 to about 20% by wt (sodium or potassium silicates, e.g., sodium disilicate, sodium meta-silicate and crystalline phyllosilicates); bleach-inorganic (e.g., perhydrate salts such as perborate, percarbonate, perphosphate, persulfate and persilicate salts) and organic (e.g., organic peroxyacids including diacyl and tetraacylperoxides, especially diperoxydodecanedioic acid, diperoxytetradecanedioic acid, and diperoxyhexadecanedioic acid); bleach activator-organic peracid precursors in the range from about 0.1 to about 10% by wt; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1-5% by wt (selected from benzatriazoles, metal salts and complexes, and silicates); enzymes in the range from about 0.01-5.0 mg of active enzyme per gram of ADW detergent composition (acyl transferases, alpha-amylases, beta-amylases, alpha-galactosidases, arabinosidases, aryl esterases, beta-galactosidases, beta-glucanases, carrageenases, catalases, cellobiohydrolases, cellulases, chondroitinases, cutinases, dispersins, endo-beta-1, 4-glucanases, endo-beta-mannanases, esterases, exo-mannanases, galactanases, glucoamylases, hemicellulases, hexosaminidase, hyaluronidases, keratinases, laccases, lactases, ligninases, lipases, lipoxygenases, mannanases, nucleases, oxidases, oxidoreductases, pectate lyases, pectin acetyl esterases, pectinases, pentosanases, peroxidases, phenoloxidases, phosphatases, phosphodiesterase, phospholipases, phytases, polyesterases, polygalacturonases, additional proteases, pullulanases, reductases, rhamnogalacturonases, beta-glucanases, tannases, transglutaminases, xanthan lyases, xylan acetyl-esterases, xylanases, xyloglucanases, xylosidases, and mixtures thereof); and enzyme stabilizer components (selected from oligosaccharides, polysaccharides and inorganic divalent metal salts).

An exemplary Automatic Dish Washing (ADW) composition is provided in the Table below.

### Exemplary ADW composition

| Ingredient | Weight in grams |
|---|---|
| Bleach Activator (tetraacetylethylenediamine; TAED) | 0.22 |
| SKS-6 sodium disilicate (Na₂Si₂O₅) | 0.8 |
| HEDP | 0.93 |
| Sodium carbonate | 1.5 |
| MGDA | 7.01 |
| Sulfonic acid group-containing polymer (Acusol^{™} 588) | 0.80 |
| Sodium percarbonate | 3.50 |
| Bleach catalyst (Manganese 1,4,7-triazacyclononane; MnTACN) | 0.256 |
| LUTENSOL^{®} TO7 | 0.90 |
| PLURAFAC^{®} SLF 180 | 0.75 |
| Dipropylene glycol | 0.40 |
| Minors | balance |
| Total % of full dose | 100 |

### Method of Use

The present invention also provides a method for treating/cleaning a surface, particularly a fabric surface, the method comprising in a contacting step, contacting the surface with an aqueous wash liquor comprising the subtilisin variant as described herein, and a cleaning adjunct. The surface may have a proteinaceous stain, such as a stain comprising egg or an egg-based stain, such as crème brûlée, baked cheese, BMI, or other protein-containing substance.

Preferably the amount of subtilisin variant in the wash liquor is from 0.01ppm to 10ppm, preferably from 0.1ppm to 1ppm. Preferably the wash liquor also contains anionic surfactant, preferably in an amount from 0.05 to 50g/l, more preferably from 0.2g/l to 5g/l or 0.5g/l to 3g/l.

The aqueous wash liquor may be formed by adding a composition as described above to water, for example in a washing machine or hand washing process. Alternatively, the aqueous wash liquor may be formed by adding the subtilisin variantand cleaning adjunct as separate components, into water to form the wash liquor. The surface, particularly fabric may be optionally subsequently washed, and/or rinsed and/or dried.

The subtilisin variant enzyme, and any additional enzyme may be present in the wash liquor, preferably in an amount corresponding to 0.001-100 mg of active enzyme protein per liter of wash liquor, preferably 0.005-5 mg of active enzyme protein per liter of wash liquor, more preferably 0.01-1 mg of enzyme protein per liter of wash liquor and in particular 0.1-1 mg of enzyme protein per liter of wash.

In the contacting step, or in a subsequent step, it may be preferred to use mechanical agitation to promote cleaning and removal of the broken-down soil by-products from the surface, particularly when the surface is a fabric surface. The wash liquor preferably has a pH of from about 7 or 8 to about 10.5. The composition may typically be employed at concentrations of from about 500 ppm to about 15,000 ppm in solution to form the wash liquor. The wash liquor preferably has a temperature from about 5 °C to about 40 °C, or preferably from 10 to 35 °C or 30 °C or 25 °C. Where the surface is a fabric surface, the water to fabric ratio is typically from about 1:1 to about 30:1. The compositions and methods herein are particularly useful for treating any fabric surface, synthetic or natural, including cotton, wool, silk, polyester, nylon, elastane or mixed fabric, such as polycotton.

The following examples are provided to demonstrate and illustrate certain preferred embodiments and aspects of the present disclosure and should not be construed as limiting.

### EXAMPLE 1

### Generation of enzyme variants

*Bacillus amyloliquefaciens* (BPN') wildtype subtilisin and variants thereof were produced as described below. The amino acid sequence of the mature BPN' parent enzyme is set forth as SEQ ID NO 1. All BPN' subtilisin variants were expressed using a DNA fragment comprising: a 5'AprE flanking region that contains a variant of the *B. subtilis rrnI*p2 promoter sequence (SEQ ID NO:2) (the *B. subtilis rrnIp2* promoter and engineered variant are more fully described in patent application WO2020112609), the nucleotide sequence encoding the *aprE* signal peptide sequence (SEQ ID NO:3), the nucleotide sequence encoding the *B. amyloliquefaciens* propeptide (SEQ ID NO:4), the sequence corresponding to the gene encoding the mature BPN' subtilisins, the BPN' terminator (SEQ ID NO:5), 3'AprE flanking sequences including a kanamycin gene expression cassette (SEQ ID NO:6), in consecutive order. This DNA fragment was assembled using standard molecular biology techniques. Linear DNA of expression cassettes were used to transform competent *B. subtilis* cells of a suitable strain. A library of BPN' subtilisin variants was generated by the methods described above.

The transformation mixtures were plated onto LA plates containing 1.6% skim milk and 5 ppm kanamycin and incubated overnight at 37°C. Single colonies were picked and grown in Luria broth at 37°C under antibiotic selection.

For protein expression experiments, transformed cells were grown in 96-well microtiter plates (MTPs) in cultivation medium (enriched semi-defined media based on MOPS buffer, with urea as major nitrogen source, glucose as the main carbon source, supplemented with 1% soytone for robust cell growth, containing antibiotic selection) for 3 days at 32°C, 250 rpm, with 70% humidity in a shaking incubator. After centrifugation and filtration, clarified culture supernatants containing the proteases of interest were used for assays.

### EXAMPLE 2

### Enzyme assays

Protein Concentration determination: An Agilent Infinity II 1290 UHPLC equipped with an Agilent, 300 SB-C3 RRHD (1.8 µm 2.1 x 50 mm) column was used for protein concentration quantitation. The column temperature was 65°C and samples were eluted from the column using a gradient of 0.1% trifluoroacetic acid (TFA) in water and 0.07% TFA in acetonitrile. Absorbance was measured at 220 nm, and peaks were integrated using OpenLab software (Agilent Technologies, USA). The protein concentrations of the samples were calculated based on a standard curve of the parent protease.

Protease Activity: The protease activities of the subtilisin parent and variants thereof were tested by measuring the hydrolysis of N-suc-AAPF-pNA substrate. For the AAPF assay, the reagent solutions used were: 100 mM Tris pH 8.6, 0.005% Tween^{®}-80 and 160 mM suc-AAPF-pNA in DMSO (suc-AAPF-pNA stock solution) (Sigma: S-7388). To prepare a working solution, 1 mL suc-AAPF-pNA stock solution was added to 100 mL of the Tris buffer and mixed. An enzyme sample was added to a microtiter plate (MTP) containing 1.6 mM suc-AAPF-pNA working solution and assayed for activity by measuring absorbance at 405 nm over 3-5 min using a SpectraMax plate reader in kinetic mode at room temperature. The protease activity was expressed as mOD/min.

Cleaning performance assays: The liquid laundry detergents used in cleaning performance and stability assays were Persil Small & Mighty Non-Bio Liquid Detergent "Persil Non-Bio" (PNB, Unilever), which was purchased in Sept 26, 2014 from a UK supermarket. For cleaning performance assays, the PNB detergent was diluted to 2.7 g/l in deionized water and brought to 5 mM HEPES (pH 8.2) with a water hardness of 12 gpg (3Ca: 1Mg). This detergent is considered boron-free since it contains ≤5mg/Kg of boron, when tested for elemental boron content.

Protease variants were tested for cleaning performance relative to the parent (BPN') on the technical soils C-05 (blood/milk/ink on woven cotton) and C-S-39 (full egg carbon with carbon black, aged, on woven cotton), both purchased from Center for Testmaterials BV, Vlaardingen, Netherlands. The soils were punched into small circular swatches and distributed into Costar 9017 or Greiner 655101 microtiter plates (MTPs). The microswatch-containing MTPs were first filled with detergent. Afterwards, quantities of the parent enzyme and variants were added to a final volume of 200 microliters. Assays were carried out at 25°C for 25 minutes with gentle shaking. Following the incubation period, 100-150 microliters of supernatant was transferred to a fresh MTP and absorbance was read at 600 nm for BMI swatches or at 405 nm for whole egg swatches using a SpectraMax plate reader. Absorbance results were obtained by subtracting the value for a blank control (no enzyme) from each sample value. For each condition and subtilisin variant in Example 2, a cleaning performance index (PI) was calculated by dividing the blank subtracted absorbance of the variant by that of the parent protease at the same concentration. The blank subtracted absorbance value for the parent protease at the corresponding concentration of the variant was determined using a standard curve of the parent protease, which was included in the test and was generated using a Langmuir fit or Hill Sigmoidal fit, as appropriate.

General Sample set up for Stability Assays:_Subtilisin enzymes were tested for stability in a 10% solution (v/v) of detergent, PNB, Detergent A (Det A), detergent B (Det B), and/or Detergent C (Det C). The composition of Detergent A is described in Table 1. The composition of Detergent B is described in Table 2. The composition of Detergent C is described in Table 5. BPN' and BPN' variants were tested at 40°C, 51°C, 53°C, 54°C, 56°C, 60°, and/or 66°C. The elevated temperature was set to enable discrimination of residual activity of the stressed sample compared to the unstressed sample during an incubation period of 20 minutes in a range appropriate to discern differences between variant enzymes and their parent or reference variant. The exact temperature required to obtain the desired range in stabilities may depend on the equipment makers and settings used. Incubation of samples across a temperature gradient can be helpful in determining this temperature. Fitting the residual activity across temperature gradients with an appropriate curve fit allows for comparing the relative stabilities of samples at any temperature in the tested range. An enzyme sample was mixed into the diluted detergent and the protease activity on AAPF substrate was measured immediately, to serve as the unstressed value. The samples were subsequently placed in a PCR plate, sealed and incubated at elevated temperature for 20 min using a thermocycler, then assayed for AAPF activity to obtain the stressed value. Residual activities were calculated by taking a ratio of the stressed to unstressed activity. All enzyme samples were assayed in triplicate for the assays. A lower limit cutoff for protein expression of 200 ppm was applied and data with 20% or lower CV (coefficient of variation) was analyzed.

| Table 1. Composition of Detergent A (Det A = Example Formula 26, below). | | | |
|---|---|---|---|
| | Raw material | % active | |
| C₁₁₋₁₈ alkyl benzene sulphonic acid | | | 9.2 |
| C₁₂₋₁₈ alkyl ethoxy sulphate (3-5 EO) | | | 4 |
| C₁₀₋₁₈ alcohol ethoxylate (average ethoxylation degree 7-9) | | | 4 |
| C₁₂₋₁₈ Fatty acid | | | 1.7 |
| Citric acid | | | 2.7 |
| Ethanolamine | | | 0.24 |
| NaOH | | | 3.1 |
| Chelant | | | 0.5 |
| Propylene glycol | | | 1.2 |
| Sodium cumene sulphonate | | | 1.7 |
| Water, minors | | | Balance |

| Table 2. Composition of Detergent B (Det B = Example Formula 22, below). | | |
|---|---|---|
| Raw material | % active | |
| C₁₁₋₁₈ alkyl benzene sulphonic acid | | 11 |
| C₁₂₋₁₈ alkyl ethoxy sulphate (3-5 EO) | | 2.5 |
| C₁₀₋₁₈ alcohol ethoxylate (average ethoxylation degree 7-9) | | 11.1 |
| Alkoxylated polyethyleneimine | | 1.8 |
| C₁₂₋₁₈ Fatty acid | | 0.3 |
| Sodium Citrate | | 0.7 |
| Borate | | 1.6 |
| Ethanolamine | | 2.2 |
| NaOH | | 0.006 |
| Chelant | | 0.27 |
| Propylene glycol | | 1.2 |
| Sodium cumene sulphonate | | 0.25 |
| Suds suppressor | | minor |
| Water, minors | | Balance |

### EXAMPLE 3

### Variant subtilisins with increased stability in presence of detergent

Variants of subtilisins BPN' were tested against the parent enzyme or reference variant to determine the relative improvement in stability when measured at 54°C, 56°C, 60° or 66°C for 20 minutes in detergent, as described in Example 2 and reported as fraction of stressed to unstressed enzyme activity. Tables 3 and 4 show the tests results obtained for a series of BPN' variants with significant stability enhancements relative to wildtype BPN' (SEQ ID NO:1).

Subtilisin variants in this Example generally displayed robust cleaning performance.

| Table 3. Stability in detergent of various BPN' variants in PNB detergent. | | |
|---|---|---|
| Sample | Mutations with respect to BPN' | Fraction, Residual activity, at 54°C |
| BPN-WT | none | 0.0 |
| BPN-00657 | S033T-S053G-N076D-S078N-S101N-G128S-Y217Q | 0.4 |
| BPN-00649 | S033T-A045V-S053G-N076D-S078N-S101N-G128S-S183N-Y217Q | 0.5 |
| BPN-00659 | S033T-S053G-N076D-S078N-S101N-G128S-Y217Q-T254A | 0.6 |
| BPN-00668 | S033T-A045V-S053G-N076D-S078N-S101N-G128S-Y217Q | 0.4 |
| BPN-00678 | S033T-S053G-N076D-S078N-S101N-G128S-S145R-Y217Q | 0.5 |
| BPN-00688 | S033T-S053G-N076D-S078N-S101N-G128S-S183N-Y217Q | 0.6 |
| BPN-00698 | S033T-A045V-S053G-N076D-S078N-S101N-G128S-S145R-S183N-Y217Q | 0.6 |
| BPN-00708 | S033T-A045V-S053G-N076D-S078N-S101N-G128S-S145R-Y217Q | 0.5 |
| BPN-00718 | S033T-S053G-N076D-S078N-S101N-G128S-S145R-S183N-Y217Q | 0.6 |
| BPN-00729 | S003Q-T022Y-A045V-S078N-Y217Q-N218S | 0.5 |
| BPN-00739 | S003Q-T022Y-S024Q-A045V-S078N-Y217Q-N218S | 0.7 |
| BPN-00749 | S003Q-T022Y-S024Q-A045V-S078N-S162Q-Y217Q-N218S | 0.7 |
| BPN-00766 | S024Q-S053G-N076D-S078N-S101N-G128S-Y217Q | 0.4 |
| BPN-00776 | S024Q-S053G-N076D-S078N-S101N-G128S-S162Q-Y217Q | 0.5 |
| BPN-00786 | S003Q-S024Q-S053G-N076D-S078N-S101N-G128S-S162Q-Y217Q | 0.6 |

| Table 4. Stability of additional BPN' variants in various detergents and temperature conditions. | | | | |
|---|---|---|---|---|
| | | Fraction Residual activity | | |
| Sample | Mutations with respect to BPN' | Det A at 56°C | PNB at 60°C | Det B at 66°C |
| BPN-WT | none | 0 | 0 | 0 |
| BPN-00657 | S033T-S053G-N076D-S078N-S101N-G128S-Y217Q | 0 | 0 | 0 |
| BPN-06105 | S003Q-S024Q-S033T-A045V-S053G-N076D-S078N-S101N-N109Q-N118R-G128S-S145R-S162Q-G169A-S183N-Y217Q-S248A-T254A | 0.6 | 0.3 | ND |
| BPN-06108 | S003Q-S024Q-S033T-A045V-S053G-N076D-S078N-S101N-N109Q-N118R-G128S-S145R-S162Q-S183N-Y217Q-S248A-T254A | 0.5 | 0.3 | ND |
| BPN-06109 | S003Q-Y006W-S024Q-S033T-A045V-S053G-N076D-S078N-S1 01N-N1 09Q-N118R-G128S-S145R-S162Q-S183N-Y217Q-S248A-T254A | 0.6 | 0.5 | ND |
| BPN-06110 | S003Q-T022Y-S024Q-S033T-A045V-S053G-N076D-S078N-S1 01N-N1 09Q-N118R-G128S-S145R-S162Q-S183N-Y217Q-S248A-T254A | 0.6 | 0.3 | ND |
| BPN-06122 | S003Q-S024Q-S033T-A045V-S053G-N076D-S078N-S101N-N109Q-N118R-G128 S-S145R-S162Q-S182Q-S183N-Y217Q-S248A-T254A | 0.5 | 0.3 | ND |
| BPN-06125 | S003Q-S024Q-S033T-A045V-S053G-N076D-S078N-S101N-N109Q-N118R-G128 S-S145R-S162Q-S183N-Y217Q-N218S-S248A-T254A | 0.7 | 0.6 | ND |
| BPN-06127 | S003Q-T022Y-S024Q-S033T-A045V-S053G-N076D-S078N-S1 01N-N1 09Q-N118R-G128S-S145R-S162Q-S183N-Y217Q-N218 S-S248A-T254A | 0.7 | 0.7 | ND |
| BPN-07061 | S003Q-Y006W-T022Y-S024Q-S033T-A045V-S053G-N076D-S078N-S101N-N109Q-N118R-G128S-S145R-S162Q-G169A-S183N-Y217Q-S248A-T254A | ND | 0.6 | ND |
| BPN-07065 | S003Q-Y006W-T022Y-S024Q-S033T-A045V-S053G-N076D-S078N-S101N-N109Q-N118R-G128S-S145R-S162Q-G166Q-S183N-Y217Q-S248A-T254A | ND | 0.7 | ND |
| BPN-07080 | S003Q-T022Y-S024Q-S033T-A045V-S053G-N076D-S078N-S101N-N1 09Q-N118R-G128S-S145R-S162Q-G166Q-S183N-Y217Q-N218S-S248A-T254A | ND | 0.8 | ND |
| BPN-07082 | S003Q-Y006W-T022Y-S024Q-S033T-A045V-S053G-N076D-S078N-S101N-N109Q-N118R-G128S-S145R-S162Q-S183N-Y217Q-N218S-S248A-T254A | ND | 0.8 | ND |
| BPN-07086 | S003Q-T022Y-S024Q-S033T-A045V-S053G-N076D-S078N-S101N-N1 09Q-N118R-G128S-S145R-S162Q-G166Q-S182Q-S183N-Y217Q-S248A-T254A | ND | 0.5 | ND |
| BPN-07094 | S003Q-T022Y-S024Q-S033T-A045V-S053G-N076D-S078N-S1 01N-N1 09Q-N118R-G128S-S145R-S162Q-G166Q-G169A-S183N-Y217Q-S248A-T254A | ND | 0.4 | ND |
| BPN-07104 | S003Q-Y006W-T022Y-S024Q-S033T-A045V-S053G-N076D-S078N-S101N-N109Q-N118R-G128S-S162Q-G169AS182Q-S183N-Y217Q-N218S-S248AT254A | ND | 0.9 | ND |
| BPN-07121 | S003Q-Y006W-T022Y-S024Q-S033T-A045V-S053G-N076D-S078N-S101N-N109Q-N118R-G128S-S145R-S162Q-S182Q-S183N-Y217Q-S248A-T254A | ND | 0.7 | ND |
| BPN-07133 | S003Q-T022Y-S024Q-S033T-A045V-S053G-N076D-S078N-S1 01N-N1 09Q-N118R-G128S-S145R-S162Q-G169AS182Q-S183N-Y217Q-N218S-S248A-T254A | ND | 0.8 | ND |
| BPN-0713 5 | S003Q-Y006W-T022Y-S024Q-S033T-A045V-S053G-N076D-S078N-S101N-N109Q-N118R-G128S-S145R-S162Q-G169A-S 182Q-S 183N-Y217Q-S248AT254A | ND | 0.7 | ND |
| BPN-07144 | S003Q-Y006W-T022Y-S024Q-S033T-A045V-S053G-N076D-S078N-S101N-N109Q-N118R-G128S-S145R-S162Q-S182Q-S183N-Y217Q-N218S-S248AT254A | ND | 0.8 | ND |
| BPN-07160 | S003Q-Y006W-T022Y-S024Q-S033T-A045V-S053G-N076D-S078N-S101N-N109Q-G128S-S145R-S162Q-G169A-S182Q-S183N-Y217Q-N218S-S248A-T254A | ND | 0.9 | ND |
| BPN-07163 | S003Q-T022Y-S024Q-S033T-A045V-S053G-N076D-S078N-S101N-N1 09Q-N118R-G128S-S145R-S162Q-G169AS183N-Y217Q-N218S-S248A-T254A | ND | 0.8 | ND |
| BPN-07171 | S003Q-Y006W-T022Y-S024Q-S033T-A045V-S053G-N076D-S078N-S101N-N109Q-N118R-G128S-S145R-S162Q-S183N-Y217Q-S248A-T254A | ND | 0.5 | ND |
| BPN-07183 | S003Q-Y006W-T022Y-S024Q-S033T-A045V-S053G-N076D-S078N-S101N-N109Q-N118R-G128S-S145R-S162Q-G169A-S183N-Y217Q-N218S-S248A-T254A | ND | 0.8 | ND |
| BPN-07188 | S003Q-T022Y-S024Q-S033T-A045V-S053G-N076D-S078N-S101N-N1 09Q-N118R-G128S-S145R-S162Q-S182Q-S183N-Y217Q-N218S-S248A-T254A | ND | 0.8 | ND |
| BPN-07189 | S003Q-T022Y-S024Q-S033T-A045V-S053G-N076D-S078N-S101N-N109Q-N118R-G128S-S145R-S162Q-G169AS182Q-S183N-Y217Q-S248A-T254A | ND | 0.4 | ND |
| BPN-07191 | S003Q-Y006W-T022Y-S024Q-S033TA045V-S053G-N076D-S078N-S101N-N109Q-N118R-G128S-S145R-S162Q-G169A-S182Q-S183N-Y217Q-N218S-S248A-T254A | ND | 0.8 | ND |
| BPN-07202 | S003Q-Y006W-T022Y-S024Q-S033T-A045V-S053G-N076D-S078N-S101N-N109Q-N118R-G128S-S145R-S162Q-G166Q-G169A-S182Q-S183N-Y217Q-N218S-S248A-T254A | ND | 0.9 | 0.8 |
| BPN-08004 | S003Q-T022Y-S024K-S033T-S053G-N076D-S078N-S101N-N118R-G128S-S182Q-S183N-Y217Q-M222Q-S248A-T254A | ND | 0.2 | 0.2 |
| BPN-08055 | S003Q-S024K-S033T-A045V-S053G-N076D-S078N-S101N-N109Q-N118R-G128S-S182Q-S183N-Q206Y-Y217Q-M222Q-T254A | ND | 0.3 | ND |
| BPN-08207 | S003Q-S024Q-S033T-A045V-S053G-N076D-S078N-S101N-N109Q-G128AS145R-S162Q-S182Q-S183N-Y217Q-M222Q-S248A-T254A | ND | 0.4 | 0.3 |
| BPN-08234 | S003Q-S024Q-S033T-A045V-S053G-N076D-S078N-S 101N-N109Q-G128 A- | ND | 0.3 | 0.3 |
| BPN-12542 | S003Q-Y006W-T022Y-S024Q-S033T-A045V-S053G-N076D-S078N-S101N-N109Q-G128S-G169A-S182Q-Y217Q-S248A | ND | 0.5 | 0.4 |
| BPN-12565 | S003Q-T022Y-S024Q-S033T-S053G-N076D-S078N-S101N-G128S-S145R-G169A-S182Q-Y217Q-N218S | ND | 0.6 | 0.6 |
| BPN-12606 | S003Q-T022Y-S024Q-S033T-A045V-S053G-N076D-S078N-S101N-N109Q-G128A-G169A-S182Q-S183N-S204Q-Q206Y-Y217Q-N218S-T254A | ND | 0.9 | 0.7 |
| BPN-12655 | S003Q-T022Y-S024Q-S033T-S053G-N076D-S078N-S101N-N118R-G128S-S182Q-Y217Q-N218S-T254A | ND | 0.5 | 0.3 |
| BPN-12658 | S003Q-Y006W-S024Q-S033T-S053G-N076D-S078N-S101N-G128S-G169AS182Q-Y217Q-N218S-S248A-T254A | ND | 0.8 | 0.6 |
| BPN-12663 | Y006W-S024Q-S033T-A045V-S053G-N076D-S078N-S101N-G128S-S145R-S162Q-S182Q-Y217Q-N218S-T254A | ND | 0.7 | 0.3 |
| BPN-12676 | S003Q-T022Y-S024Q-S033T-S053G-N076D-S078N-S101N-N118R-G128S-S162Q-S182Q-S183N-Y217Q-T254A | ND | 0.3 | 0.3 |
| BPN-12788 | S033T-A045V-S053G-N076D-S078N-S101N-N118R-G128S-S183N-Y217Q | ND | 0.4 | 0.4 |
| BPN-12811 | S024Q-S033T-A045V-S053G-N076D-S078N-S101N-G128A-S145R-Y217Q-S248A-T254A | ND | 0.7 | 0.7 |
| BPN-12819 | S024Q-S033T-S053G-N076D-S078N-S101N-N109Q-G128S-S145R-S162Q-G169A-Y217Q-N218S | ND | 0.4 | 0.3 |
| BPN-12823 | S003Q-S024Q-S033T-A045V-S053G-N076D-S078N-S101N-G128S-S182Q-Y217Q | ND | 0.4 | 0.5 |
| BPN-12878 | S003Q-T022Y-S024Q-S033T-S053G-N076D-S078N-S101N-N109Q-N118R-G128S-S162Q-G169A-S183N-Y217Q-N218S-T254A | ND | 0.6 | 0.6 |
| BPN-12919 | S003Q-Y006W-S024Q-S033T-S053G-N076D-S078N-S101N-N109Q-G128S-S145R-G169A-S183N-Y217Q-N218S-S248A | ND | 0.6 | 0.4 |
| BPN-12958 | Y006W-S024Q-S033T-A045V-S053G-N076D-S078N-S101N-G128S-S162Q-S183N-Y217Q-N218 S-T254A | ND | 0.6 | 0.3 |
| BPN-13018 | S003Q-S024Q-S033T-A045V-S053G-N076D-S078N-S101N-G128S-S145R-S162Q-S182Q-S183N-Y217Q-S248A-T254A | ND | 0.3 | 0.2 |
| BPN-13037 | S003Q-T022Y-S024Q-S033T-S053G-N076D-S078N-S101N-G128S-S145R-S162Q-S182Q-S183N-Y217Q-S248A-T254A | ND | 0.3 | 0.3 |
| BPN-13060 | Y006W-T022Y-S024Q-S033T-S053G-N076D-S078N-S101N-G128S-S183N-Y217Q-S248A-T254A | ND | 0.3 | 0.2 |
| BPN-13148 | S003Q-Y006W-T022Y-S024Q-S033T-S053G-S078N-S087D-S101N-N118R-G128S-S162Q-S182Q-Y217Q-T254A | ND | 0.3 | 0.3 |
| BPN-13238 | S003Q-T022Y-S024K-S 033T-A045 V-S053 G-N076D-S078N-S087D-S101N-N118R-G128S-S182Q-S183N-S204Q-Q206Y-Y217Q-M222Q-S248A-T254A | ND | 0.6 | 0.4 |
| BPN-13249 | S003Q-T022Y-S024K-S033T-S053G-N076D-S078N-S101N-G128S-S145R-S162Q-G166Q-S182Q-Q206Y-Y217Q-N218S-M222Q-T254A | ND | 0.6 | 0.8 |
| BPN-13263 | S003Q-Y006W-T022Y-S024Q-S033T-A045V-S053G-T055P-N076D-S078N-S087D-S101N-N118R-G128S-S182Q-S183N-S204Q-Q206Y-Y217Q-N218S-M222Q-S248A-T254A | ND | 0.8 | 0.6 |
| BPN-13266 | S003Q-S024Q-S033T-A045V-S053G-T055P-N076D-S078N-S101N-N109Q-G128A-S145R-S162Q-S182Q-S204Q-Y217Q-M222Q-T254A | ND | 0.5 | 0.3 |
| BPN-13413 | S033T-S053G-N076D-S078N-S101N-G128S-S145R-S183N-Y217Q-S248A | ND | 0.6 | 0.3 |

### EXAMPLE 4

### Additional variant subtilisins with increased stability in presence of detergent

Additional variants of subtilisin BPN' were tested against the parent enzyme (wildtype BPN', SEQ ID NO:1) to determine the relative improvement in stability in various detergent conditions using method described in Example 2. Stability was measured at 51 °C in Detergent C (composition of Detergent C (Det. C) is shown on Table 5), at 53°C in Detergent B, and at 40°C in PBN detergent for 20 minutes using the method described in Example 2.

| Table 5. Composition of Detergent C (Det C). | | |
|---|---|---|
| | Raw material | % active |
| | C₁₁₋₁₈ alkyl benzene sulphonic acid | 9.8 |
| | C₁₂₋₁₈ alkyl ethoxy sulphate (3-5 EO) | 2.2 |
| | C₁₀₋₁₈ alcohol ethoxylate (average ethoxylation degree 7-9) | 7.5 |
| | C₁₂₋₁₈ Fatty acid | 2.1 |
| | Citric acid | 3.1 |
| | Ethanolamine | 0.2 |
| | NaOH | 3.4 |
| | Chelant | 0.4 |
| | Propylene glycol | 0.7 |
| | Sodium cumene sulphonate | 0.7 |
| | Water, minors | Balance |

The stability improvements results for BPN' variants are reported as percent (%) residual activity after stress, and are shown on Table 6.

| Table 6. Stability of additional BPN' variants in various detergents and temperature conditions. | | | | |
|---|---|---|---|---|
| Sample ID | Substitution relative to WT BPN' parent | Residual Activity (%) | | |
| | | Det C at 51°C | Det B at 53°C | PNB at 40°C |
| BPN-WT | none | 7 | 13 | 10 |
| BPN-18934 | T055P-S204Q | 12 | 15 | 20 |
| BPN-18922 | S182Q-M222Q | 24 | 23 | 24 |
| BPN-19139 | S162Q-S204Q | 23 | 24 | 33 |
| BPN-19025 | Y006W-S024K | 20 | 26 | 28 |
| BPN-19003 | M222Q-T254A | 20 | 27 | 20 |
| BPN-19010 | Q206Y-M222Q | 26 | 28 | 34 |
| BPN-19182 | T055P-M222Q | 32 | 28 | 19 |
| BPN-18982 | N109Q-M222Q | 31 | 30 | 43 |
| BPN-18902 | S183N-S204Q | 19 | 33 | 22 |
| BPN-19047 | S162Q-M222Q | 36 | 34 | ND |
| BPN-19091 | Y006W-S183N | 31 | 37 | 40 |
| BPN-18888 | S204Q-M222Q | 38 | 40 | 40 |
| BPN-18901 | S183N-M222Q | 34 | 40 | 29 |
| BPN-19121 | Y006W-S204Q | 37 | 42 | 40 |
| BPN-18883 | M222Q-S248A | 25 | 43 | 26 |
| BPN-18871 | S204Q-T254A | 24 | 45 | 28 |
| BPN-19068 | Y006W-T254A | 53 | 62 | 38 |
| BPN-18899 | N109Q-S162Q-S182Q | 10 | 21 | 20 |
| BPN-18990 | N076D-S182Q-M222Q | 61 | 26 | 73 |
| BPN-19153 | A045V-S204Q-M222Q | 30 | 26 | 43 |
| BPN-18891 | S101N-S162Q-M222Q | 32 | 27 | 23 |
| BPN-18974 | T055P-N109Q-S204Q | 17 | 28 | 14 |
| BPN-19086 | Y006W-S024K-M222Q | 39 | 29 | ND |
| BPN-18926 | N109Q-S162Q-M222Q | 30 | 32 | 28 |
| BPN-19126 | T055P-S162Q-S204Q | 18 | 32 | 25 |
| BPN-18943 | S162Q-S182Q-S204Q | 18 | 34 | 26 |
| BPN-19066 | S162Q-S182Q-M222Q | 27 | 34 | 32 |
| BPN-19067 | S101N-S182Q-M222Q | 52 | 37 | 42 |
| BPN-19131 | T055P-S182Q-T254A | 16 | 37 | 23 |
| BPN-18889 | S204Q-Y217Q-M222Q | 31 | 40 | 60 |
| BPN-19053 | Y006W-S183N-Q206Y | 36 | 40 | 35 |
| BPN-19061 | N109Q-S182Q-M222Q | 40 | 41 | 30 |
| BPN-18999 | T055P-S204Q-M222Q | 52 | 41 | 33 |
| BPN-19018 | N109Q-S204Q-T254A | 22 | 41 | 22 |
| BPN-19075 | T055P-S182Q-M222Q | 29 | 42 | 33 |
| BPN-18931 | T055P-S162Q-T254A | 26 | 42 | 18 |
| BPN-19173 | T055P-M222Q-T254A | 30 | 43 | 24 |
| BPN-18887 | S003Q-S162Q-T254A | 31 | 43 | 42 |
| BPN-18925 | S078N-S162Q-T254A | 35 | 43 | 29 |
| BPN-18866 | N109Q-S204Q-M222Q | 46 | 45 | 38 |
| BPN-19049 | S003Q-S182Q-T254A | 22 | 45 | 28 |
| BPN-19141 | S003Q-M222Q-T254A | 53 | 46 | 47 |
| BPN-18906 | S033T-M222Q-T254A | 34 | 46 | 28 |
| BPN-18897 | S024Q-S204Q-M222Q | 57 | 47 | 53 |
| BPN-18893 | T022Y-S162Q-T254A | 26 | 47 | 29 |
| BPN-19103 | S078N-S182Q-T254A | 29 | 47 | 29 |
| BPN-19136 | S003Q-S162Q-M222Q | 47 | 47 | 99 |
| BPN-19163 | N109Q-M222Q-T254A | 17 | 50 | 20 |
| BPN-19109 | T022Y-S182Q-T254A | 23 | 51 | 31 |
| BPN-19174 | S087D-S162Q-T254A | 41 | 51 | 50 |
| BPN-18870 | N076D-S182Q-T254A | 61 | 52 | 80 |
| BPN-19058 | S204Q-Y217Q-T254A | 22 | 52 | 25 |
| BPN-18894 | N076D-S162Q-T254A | 62 | 52 | 59 |
| BPN-18970 | S078N-S204Q-T254A | 41 | 53 | 48 |
| BPN-19063 | G128S-S204Q-T254A | 25 | 54 | 27 |
| BPN-19033 | N076D-S204Q-T254A | 61 | 56 | 71 |
| BPN-19051 | S087D-S182Q-T254A | 55 | 56 | 53 |
| BPN-18935 | S182Q-S204Q-T254A | 29 | 56 | 24 |
| BPN-19055 | S101N-S204Q-T254A | 26 | 56 | 17 |
| BPN-19184 | S024Q-S204Q-T254A | 57 | 59 | 21 |
| BPN-18981 | G128A-S162Q-T254A | 12 | 60 | 11 |
| BPN-18962 | S182Q-M222Q-T254A | 44 | 62 | 37 |
| BPN-19056 | G128A-S204Q-T254A | 25 | 84 | 22 |
| BPN-19071 | T055P-N109Q-S182Q-S204Q | 22 | 23 | 25 |
| BPN-19054 | N109Q-S162Q-S182Q-M222Q | 16 | 24 | 30 |
| BPN-18949 | T055P-S182Q-S204Q-M222Q | 31 | 29 | 42 |
| BPN-19095 | T055P-N109Q-S162Q-S204Q | 19 | 33 | 21 |
| BPN-18898 | T055P-S162Q-S182Q-S204Q | 25 | 34 | 25 |
| BPN-19104 | Y006W-N109Q-S183N-S248A | 35 | 38 | 24 |
| BPN-18928 | T055P-S162Q-S182Q-M222Q | 41 | 40 | 47 |
| BPN-19078 | S024Q-S078N-S182Q-T254A | 34 | 40 | 39 |
| BPN-18877 | T055P-S162Q-S182Q-T254A | 22 | 41 | 31 |
| BPN-19070 | S162Q-S182Q-S248A-T254A | 13 | 44 | 15 |
| BPN-19176 | T055P-S162Q-S204Q-M222Q | 50 | 44 | 42 |
| BPN-18991 | Y006W-N109Q-S182Q-S248A | 36 | 45 | 45 |
| BPN-18956 | T055P-N109Q-S162Q-T254A | 15 | 46 | 73 |
| BPN-18939 | T05 5P-N109Q-S162Q-M222Q | 44 | 46 | 54 |
| BPN-19165 | S183N-M222Q-S248A-T254A | 37 | 46 | 31 |
| BPN-19024 | S078N-N109Q-S145R-T254A | 19 | 49 | 38 |
| BPN-19000 | T055P-S087D-S145R-T254A | 61 | 49 | 56 |
| BPN-19100 | N109Q-S182Q-S204Q-M222Q | 45 | 51 | 38 |
| BPN-18995 | S162Q-S182Q-S204Q-M222Q | 49 | 51 | 36 |
| BPN-19065 | Y006W-S162Q-S182Q-T254A | 37 | 51 | 43 |
| BPN-18946 | N109Q-S204Q-M222Q-T254A | 31 | 52 | 41 |
| BPN-18886 | T055P-S162Q-S204Q-T254A | 23 | 52 | 21 |
| BPN-19043 | Y006W-N109Q-S162Q-T254A | 29 | 52 | 32 |
| BPN-19097 | T055P-N109Q-S182Q-M222Q | 32 | 54 | 33 |
| BPN-18933 | Y006W-S024K-S183N-T254A | 26 | 54 | 29 |
| BPN-18904 | T055P-S182Q-S183N-T254A | 29 | 55 | 24 |
| BPN-18987 | T055P-S182Q-S204Q-T254A | 32 | 57 | 31 |
| BPN-18896 | S 162Q-S 182Q-M222Q-T254A | 33 | 61 | 45 |
| BPN-19181 | T055P-S 182Q-M222Q-T254A | 56 | 62 | 45 |
| BPN-19149 | S162Q-S182Q-S183N-T254A | 21 | 63 | 39 |
| BPN-18993 | Y006W-S024K-S182Q-T254A | 38 | 63 | 37 |
| BPN-19119 | S182Q-S204Q-M222Q-T254A | 64 | 65 | 55 |
| BPN-19052 | Y006W-S183N-S248A-T254A | 47 | 65 | 58 |
| BPN-19016 | S182Q-S183N-S204Q-T254A | 34 | 66 | -12 |
| BPN-19113 | Y006W-N109Q-S204Q-T254A | 54 | 67 | 52 |
| BPN-18912 | N109Q-N118R-G169A-M222Q | 42 | 67 | 38 |
| BPN-19076 | N109Q-S183N-S204Q-T254A | 30 | 68 | 27 |
| BPN-18960 | Y006W-T055P-S182Q-T254A | 51 | 69 | 57 |
| BPN-19014 | Y006W-S182Q-S248A-T254A | 64 | 72 | 54 |
| BPN-19037 | Y006W-S162Q-S204Q-T254A | 52 | 72 | 65 |
| BPN-19150 | S024K-S033T-G166Q-M222Q | 26 | 73 | 23 |
| BPN-18895 | S003Q-N076D-M222Q-T254A | 79 | 76 | 90 |
| BPN-19147 | S162Q-S182Q-S204Q-T254A | 36 | 78 | 20 |
| BPN-18985 | Y006W-G128S-Y217Q-T254A | 40 | 88 | 48 |
| BPN-19060 | Y006W-S183N-S204Q-T254A | 61 | 92 | 55 |
| BPN-19152 | S024Q-A045V-T055P-S078N-N109Q-S182Q | 34 | 27 | 45 |
| BPN-19111 | S024Q-N109Q-S145R-S 162Q-Y217Q-M222Q | 42 | 51 | 45 |
| BPN-19089 | S053G-N076D-S162Q-S182Q-S204Q-Y217Q | 92 | 52 | 78 |
| BPN-18953 | S003Q-S053G-S101N-N109Q-S183N-T254A | 27 | 53 | 24 |
| BPN-19046 | S024Q-S033T-N076D-S182Q-S183N-T254A | 63 | 58 | 130 |
| BPN-18882 | S003Q-S053G-T055P-N076D-G128A-T254A | 65 | 58 | 86 |
| BPN-19096 | S033T-N076D-S145R-S162Q-S182Q-T254A | 81 | 59 | 75 |
| BPN-19140 | Y006W-T055P-G128S-Y217Q-M222Q-T254A | 55 | 60 | 52 |
| BPN-19059 | S024Q-N076D-S162Q-S204Q-M222Q-T254A | 71 | 66 | 98 |
| BPN-18994 | S101N-S145R-S182Q-S183N-S204Q-N218S | 49 | 68 | 42 |
| BPN-18967 | S024K-S162Q-S182Q-Q206Y-M222Q-T254A | 46 | 69 | 25 |
| BPN-18957 | S024K-S087D-S145R-S183N-M222Q-T254A | 72 | 70 | 68 |
| BPN-19036 | S024Q-S101N-S145R-S182Q-S204Q-M222Q | 60 | 76 | 63 |
| BPN-19045 | T022Y-S087D-N109Q-S183N-M222Q-T254A | 71 | 78 | 77 |
| BPN-19012 | S024Q-N118R-G169A-S182Q-S183N-S248A | 28 | 78 | 25 |
| BPN-19085 | S024Q-S101N-S182Q-S204Q-M222Q-T254A | 69 | 80 | 54 |
| BPN-18908 | S003Q-N109Q-G128S-S162Q-G166Q-S183N | 35 | 82 | 38 |
| BPN-18968 | N076D-N109Q-S145R-S182Q-M222Q-T254A | 94 | 83 | 82 |
| BPN-18959 | Y006W-S024K-S101N-G128A-G169A-S183N | 30 | 84 | 32 |
| BPN-18937 | S101N-G 166Q-G169A-S182Q-S204Q-Q206Y | 65 | 87 | 39 |
| BPN-19084 | N109Q-S182Q-S183N-S204Q-Q206Y-T254A | 66 | 88 | 46 |
| BPN-19157 | S024K-S087D-G169A-S204Q-N218S-M222Q | 90 | 91 | 90 |
| BPN-19146 | Y006W -A045V-G128A-S145R-S182Q-T254A | 49 | 95 | 72 |
| BPN-19017 | Y006W-S087D-N109Q-S145R-S162Q-G169A | 79 | 95 | 75 |
| BPN-18996 | Y006W-T055P-S182Q-S183N-S204Q-Q206Y-M222Q-S248A-T254A | 100 | 88 | 84 |

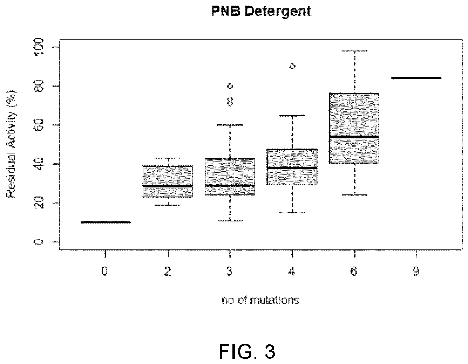

The cumulative contribution of substitutions were visualized by plotting the number of mutations versus the percent (%) residual activity for variants described in Table 6, for each detergent and temperature stress. The data for variants tested in Detergent C at 51 °C is shown on Figure 1. The data for variants tested in Detergent B at 53°C is shown on Figure 2. The data for variants tested in PNB detergent at 40°C is shown on Figure 3.

### DETERGENT EXAMPLES

Examples 1-6. Granular laundry detergent compositions designed for hand washing or top-loading washing machines.

| | 1 (wt %) | 2 (wt %) | 3 (wt %) | 4 (wt %) | 5 (wt %) | 6 (wt %) |
|---|---|---|---|---|---|---|
| Linear alkylbenzenesulfonate | 20 | 22 | 20 | 15 | 20 | 20 |
| C12-14 Dimethylhydroxyethyl ammonium chloride | 0.7 | 0.2 | 1 | 0.6 | 0.0 | 0.0 |
| AE3S | 0.9 | 1 | 0.9 | 0.0 | 0.5 | 0.9 |
| AE7 | 0.0 | 0.0 | 0.0 | 1 | 0.0 | 3 |
| Sodium tripolyphosphate | 5 | 0.0 | 4 | 9 | 2 | 0.0 |
| Zeolite A | 0.0 | 1 | 0.0 | 1 | 4 | 1 |
| 1.6R Silicate (SiO2:Na2O at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer¹ | 0.1 | 0.2 | 0.0 | 0.0 | 0.0 | 0.0 |
| Carboxymethyl cellulose | 1 | 0.3 | 1 | 1 | 1 | 1 |
| Subtilisin Variant as described herein (wt% active protein) | 0.05 | 0.05 | 0.06 | 0.1 | 0.3 | 0.2 |
| Protease (Savinase^{®}), 32.89 mg active/g) | 0.05 | 0 | 0.01 | 0 | 0 | 0.1 |
| ⁵DNase (mg active per 100g composition) | 2.0 | 0 | 4.4 | 0 | 0.4 | 0 |
| Lipase - Lipex^{®} (18 mg active /g) | 0.03 | 0.07 | 0.3 | 0.1 | 0.07 | 0.4 |
| ⁴Amylase Stainzyme^{®} Plus (mg active) | 3.0 | 5.0 | 3.0 | 2.2 | 6.0 | 6.0 |
| ⁷Hexosaminidase (mg active per 100g composition) | 0-2.0 | 0-6.0 | 0-5.6 | 0-2.2 | 0-4.0 | 0-1.5 |
| ⁸Pel-ase (mg active per 100g composition) | 0-4.3 | 0 | 0-3.4 | 0-7.2 | 0-1.9 | 3.3 |
| ⁹Psl-ase (mg active per 100g composition) | 0-3.2 | 0-3.2 | 0 | 0-1.2 | 0 | 5.3 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| MgSO4 | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | 0.78 | 3.63 |
| NOBS | 1.9 | 0.0 | 1.66 | 0.0 | 0.33 | 0.75 |
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet 9 | 0.0 | 0.0 | 0.0003 | 0.0005 | 0.0003 | 0.0 |
| Acid Blue 29 | 0.0 | 0.0 | 0.0 | 0.0 | 0.0 | 0.0003 |
| Sulfate/Moisture | Balance | | | | | |

Examples 7-13. Granular laundry detergent compositions designed for front-loading automatic washing machines.

| | 7 (wt%) | 8 (wt%) | 9 (wt%) | 10 (wt%) | 11 (wt%) | 12 (wt%) | 13 (wt%) |
|---|---|---|---|---|---|---|---|
| Linear alkylbenzenesulfonate | 8 | 7.1 | 7 | 6.5 | 7.5 | 7.5 | 11 |
| AE3S | 0 | 4.8 | 0 | 5.2 | 4 | 4 | 0 |
| C12-14 Alkylsulfate | 1 | 0 | 1 | 0 | 0 | 0 | 1 |
| AE7 | 2.2 | 0 | 3.2 | 0 | 0 | 0 | 1 |
| C10-12 Dimethyl hydroxyethylammonium chloride | 0.75 | 0.94 | 0.98 | 0.98 | 0 | 0 | 0 |
| Crystalline layered silicate (δ-Na2Si2O5) | 4.1 | 0 | 4.8 | 0 | 0 | 0 | 7 |
| Zeolite A | 5 | 0 | 5 | 0 | 2 | 2 | 4 |
| Citric Acid | 3 | 5 | 3 | 4 | 2.5 | 3 | 0.5 |
| Sodium Carbonate | 15 | 20 | 14 | 20 | 23 | 23 | 14 |
| Silicate 2R (SiO2:Na2O at ratio 2: 1) | 0.08 | 0 | 0.11 | 0 | 0 | 0 | 0.01 |
| Soil release agent | 0.75 | 0.72 | 0.71 | 0.72 | 0 | 0 | 0.1 |
| Acrylic Acid/Maleic Acid Copolymer | 1.1 | 3.7 | 1.0 | 3.7 | 2.6 | 3.8 | 2 |
| Carboxymethylcellulose | 0.15 | 1.4 | 0.2 | 1.4 | 1 | 0.5 | 0.2 |
| Subtilisin Variant as described herein (wt% active protein) | 0.05 | 0.05 | 0.06 | 0.1 | 0.3 | 0.2 | 0.06 |
| Protease - Purafect^{®} (84 mg active/g) | 0.1 | 0.1 | 0.2 | 0.05 | 0.12 | 0.13 | 0.008 |
| Lipase - Lipex^{®} (18.00 mg active/g) | 0.05 | 0.15 | 0.1 | 0 | 0 | 0 | 0.1 |
| Cellulase - CellucleanTM (15.6 mg active/g) | 0 | 0 | 0 | 0 | 0.1 | 0.1 | 0 |
| ⁴Amylase Stainzyme^{®} Plus (mg active) | 4.0 | 5.0 | 10 | 2.2 | 4.4 | 1.5 | 1.5 |
| Mannanase - Mannaway^{®} (4mg active/g) | 0.05 | 0.1 | 0 | 0.05 | 0.1 | 0 | 0.1 |
| ⁵DNase (mg active per 100g detergent) | 1.0 | 0 | 2.0 | 0 | 4.0 | 0 | 0 |
| ⁶Alginate lyase (mg active per 100g of detergent) | 0-3.3 | 0-9.2 | 0-12 | 0 | 0-3.7 | 0-13 | 0-3.3 |
| ⁷Hexosaminidase (mg active per 100g detergent) | 0-3.0 | 5.0 | 8.0 | 0-2.2 | 0-4.0 | 1.5 | 0.1 |
| ⁸Pel-ase (mg active per 100g composition) | 0-3.2 | 5.3 | 0 | 0 | 0 | 3.0 | 3.3 |
| ⁹Psl-ase (mg active per 100g composition) | 0.0 | 0.0 | 0.0 | 0-3.2 | 0-8.2 | 0 | 0.9 |
| TAED | 3.6 | 4.0 | 3.6 | 4.0 | 2.2 | 1.4 | 1 |
| Percarbonate | 13 | 13.2 | 13 | 13.2 | 16 | 14 | 10 |
| Na salt of Ethylenediamine-N,N'-disuccinic acid, (S,S) isomer (EDDS) | 0.2 | 0.2 | 0.001 | 0.2 | 0.2 | 0.2 | 0.001 |
| Hydroxyethane di phosphonate (HEDP) | 0.2 | 0.2 | 0.5 | 0.2 | 0.2 | 0.2 | 0.5 |
| MgSO4 | 0.42 | 0.42 | 0.42 | 0.42 | 0.4 | 0.4 | 0 |
| Perfume | 0.5 | 0.6 | 0.5 | 0.6 | 0.6 | 0.6 | 0.8 |
| Suds suppressor agglomerate | 0.05 | 0.1 | 0.05 | 0.1 | 0.06 | 0.05 | 0.05 |
| Soap | 0.45 | 0.45 | 0.45 | 0.45 | 0 | 0 | 0 |
| Sulphonated zinc phthalocyanine (active) | 0.000 7 | 0.001 2 | 0.000 7 | 0 | 0 | 0 | 0 |
| S-ACMC | 0.01 | 0.01 | 0 | 0.01 | 0 | 0 | 0 |
| Direct Violet 9 (active) | 0 | 0 | 0.000 1 | 0.000 1 | 0 | 0 | 0.001 |
| Sulfate/ Water & Miscellaneous | Balance | | | | | | |

| | | | | | | | |
|---|---|---|---|---|---|---|---|
| *DNase is shown as mgs of active enzyme per 100g of detergent. | | | | | | | |

### Examples 14-29. Heavy Duty Liquid laundry detergent compositions

| Quantities in wt% unless stated | 14 | 15 | 16 | 17 | 18 | 19 | 20 |
|---|---|---|---|---|---|---|---|
| C₁₂₋₁₅ Alkylethoxy(1.8)sulfate | 14.7 | 11.6 | 0.0 | 16.3 | 0.0 | 17.3 | 20 |
| C₁₁₋₁₈ Alkylbenzene sulfonate | 4.3 | 11.6 | 8.3 | 7.8 | 11.7 | 7.8 | 7 |
| C₁₆₋₁₈ Branched alkyl sulfate | 1.7 | 1.29 | 0.0 | 3.09 | 0.0 | 3.3 | 0 |
| C₁₂₋₁₄ Alkyl -9-ethoxylate | 0.9 | 1.07 | 0.0 | 1.31 | 0.0 | 1.31 | 5 |
| C₁₂ dimethylamine oxide | 0.6 | 0.64 | 0.0 | 1.03 | 0.0 | 1.03 | 2 |
| Citric acid | 3.5 | 0.65 | 3 | 0.66 | 2.27 | 0.67 | 1 |
| C₁₂₋₁₈ fatty acid | 1.5 | 2.32 | 3.6 | 1.52 | 0.82 | 1.52 | 1 |
| Sodium Borate (Borax) | 2.5 | 2.46 | 1.2 | 2.53 | 0.0 | 2.53 | 0 |
| Sodium C₁₂₋₁₈ alkyl ethoxy 3 sulfate | 0.0 | 0.0 | 2.9 | 0.0 | 3.9 | 0.0 | 0 |
| C₁₄₋₁₈ alkyl 7-ethoxylate | 0.0 | 0.0 | 4.2 | 0.0 | 1.9 | 0.0 | 0 |
| C₁₂₋₁₈ Alkyl -7-ethoxylate | 0.0 | 0.0 | 1.7 | 0.0 | 0.5 | 0.0 | 0 |
| Ca chloride dihydrate | 0.0 | 0.0 | 0.0 | 0.0 | 0.045 | 0.0 | 0 |
| Ca formate | 0.09 | 0.09 | 0.0 | 0.09 | 0.0 | 0.09 | 0.09 |
| alkoxylated ethylene diamine¹ | 0.0 | 0.0 | 1.2 | 0.0 | 0.66 | 0.0 | 0.0 |
| Random graft co-polymer² | 0.0 | 1.46 | 0.5 | 0.0 | 0.83 | 0.0 | 0.0 |
| Ethoxylated Polyethylenimine ² | 1.5 | 1.29 | 0.0 | 1.44 | 0.0 | 1.44 | 1.44 |
| Diethylene triamine pentaacetic acid | 0.34 | 0.64 | 0.0 | 0.34 | 0.0 | 0.34 | 0.34 |
| Diethylene triamine penta (methylene phosphonic acid) | 0.0 | 0.0 | 0.3 | 0.0 | 0.3 | 0.0 | 0.0 |
| 1-hydroxyethyidene-1,1-diphosphonic acid | 0.0 | 0.0 | 0.0 | 0.0 | 0.18 | 0.0 | 0.0 |
| Dihydroxybenzene-3,5-disulfonic acid disodium salt hydrate | 0.0 | 0.0 | 0.0 | 0.0 | 0.0 | 0.19 | 0.19 |
| Tinopal AMS-GX | 0.0 | 0.06 | 0.0 | 0.0 | 0.0 | 0.29 | 0.29 |
| Tinopal CBS-X | 0.2 | 0.17 | 0.0 | 0.29 | 0.0 | 0.0 | 0.0 |
| Tinopal TAS-X B36 | 0.0 | 0.0 | 0.0 | 0.0 | 0.091 | 0.0 | 0.0 |
| Amphiphilic alkoxylated grease cleaning polymer ³ | 1.28 | 1 | 0.4 | 1.93 | 0.0 | 1.93 | 1.93 |
| CHEC | 0.0 | 0.0 | 0.2 | 0.0 | 0.0 | 0.0 | 0.0 |
| Ethanol | 2 | 1.58 | 1.6 | 5.4 | 1.2 | 3.57 | 0 |
| Propylene Glycol | 3.9 | 3.59 | 1.3 | 4.3 | 0.0 | 3.8 | 3.8 |
| Diethylene glycol | 1.05 | 1.54 | 0.0 | 1.15 | 0.0 | 1.15 | 1.15 |
| Polyethylene glycol | 0.06 | 0.04 | 0.0 | 0.1 | 0.0 | 0.1 | 0.1 |
| Protease as described herein | 0.008 | 0.05 | 0.03 | 0.15 | 0.01 | 0.04 | 0.009 |
| ⁴Amylase Amplify^{®} (mg active per 100g detergent) | 8.0 | 7.0 | 2.5 | 4.0 | 3.0 | 1.7 | 3 |
| ⁵DNase (mg active per 100g detergent) | 0 | 2.0 | 0 | 2.0 | 1.25 | 1.0 | 5 |
| ⁶Alginate lyase (mg active per 100g detergent) | 3.2 | 4.1 | 0 | 0 | 0 | 0-5.0 | 0-17.3 |
| ⁷Hexosaminidase (mg active per 100g detergent) | 0-7.0 | 0-3.0 | 0-8.0 | 0-2.2 | 0-1.25 | 0-10 | 0-0.1 |
| ⁸Pel-ase (mg active per 100g composition) | 0-2.2 | 0 | 0-1.2 | 0-0.8 | 0-0.10 | 0-8.3 | 0-2.2 |
| ⁹Psl-ase (mg active per 100g composition) | 0-3.4 | 4.4 | 0 | 0 | 0 | 0-4.3 | 0-5.3 |
| Monoethanolamine | 3.05 | 2.41 | 0.4 | 1.26 | 0.31 | 1.13 | 1.13 |
| NaOH | 2.44 | 1.8 | 0.0 | 3.01 | 3.84 | 0.24 | 0.24 |
| Sodium Cumene Sulphonate | 0.0 | 0.0 | 1 | 0.0 | 0.95 | 0.0 | 0.0 |
| Sodium Formate | 0.0 | 0.11 | 0.0 | 0.09 | 0.2 | 0.12 | 0.12 |
| Polyethoxylated azo thiophene dye | 0.001 | 0.001 | 0.001 | 0.05 | 0.000 1 | 0.000 1 | 0.000 1 |
| Water, Aesthetics (Dyes, perfumes) and Minors (Enzymes including lipase, additional amylase each at 0.2% active protein, solvents, structurants) | To balance | | | | | | |

| | 21 | 22 | 23 | 24 | 25 | 26 | 27 | 28 | 29 |
|---|---|---|---|---|---|---|---|---|---|
| | (wt% ) | (wt% ) | (wt% ) | (wt% ) | (wt %) | (wt% ) | (wt% ) | (wt% ) | (wt %) |
| C10-18 Alkylethoxy(1.5-3) sulfate | 26 | 2.5 | 2.5 | 2.7 | 1.5 | 4 | 6 | 6.9 | 1.35 |
| C11-18 Alkylbenzene sulfonate | 0 | 11 | 15 | 11.6 | 6 | 9.2 | 10 | 3 | 6 |
| C10-18 (optionally branched) alkyl sulfate | 0 | 0 | 0 | 1 | 3 | 0 | 0 | 0 | 0 |
| C10-18 Alkyl -(3-9)-ethoxylate | 5.9 | 11.1 | 5 | 11.7 | 7.3 | 4.1 | 11.0 | 5.1 | 11.9 |
| C12 dimethylamine oxide | 3 | 0.2 | 1 | 0.2 | 0.2 | 0.53 | 0.3 | 0 | 0.49 |
| Citric acid | 0 | 0.69 | 0.25 | 0.54 | 0.54 | 2.7 | 2.7 | 0.57 | 0.6 |
| C12-18 fatty acid | 0 | 0.33 | 0.33 | 0.35 | 0.35 | 1.73 | 2 | 2.85 | 1.43 |
| Sodium Borate (Borax) | 1 | 1.63 | 1.63 | 0 | 0 | 0 | 0 | 0 | 0 |
| Ca chloride dihydrate | 0 | 0 | 0 | 0 | 0 | 0.01 | 0.01 | 0.03 | 0.03 5 |
| Ca formate | 0 | 0.1 | 0.1 | 0.1 | 0.1 | 0 | 0 | 0 | 0 |
| ¹Alkoxylated ethylene diamine | 1.2 | 0 | 0 | 0 | 0 | 0.66 | 0.66 | 0 | 0 |
| Random graft copolymer1 | 0.5 | 0 | 0 | 0 | 0 | 1.27 | 1.7 | 0 | 0 |
| Ethoxylated Polyethylenimine 2 | 0 | 1.82 | 1.82 | 1.9 | 1.9 | 0 | 0 | 0.26 | 1 |
| Diethylene triamine pentaacetic acid | 0 | 0 | 0 | 0 | 0 | 0 | 0 | 0.28 | 0.3 |
| Diethylene triamine penta (methylene phosphonic acid) | 0.3 | 0 | 0.2 | 0 | 0.3 | 0.51 | 1 | 0 | 0 |
| 1-hydroxyethyidene-1,1-diphosphonic acid | 0 | 0 | 0 | 0 | 0.1 | 0 | 0 | 0 | 0 |
| Tetrasodium glutamate diacetate | 0 | 0.27 | 1 | 0.28 | 0.28 | 0 | 0.3 | | |
| Dihydroxybenzene-3,5-disulfonic acid disodium salt hydrate | 0 | 0 | 0 | 0 | 0 | 0 | 0 | 0 | 0 |
| Tinopal AMS-GX | 0 | 0.052 | 0.025 | 0.055 | 0.05 5 | 0 | 0.025 | 0 | 0 |
| Tinopal CBS-X | 0 | 0 | 0.025 | 0 | 0 | 0.046 | 0.025 | 0 | 0.04 |
| Tinopal TAS-X B36 | 0 | 0 | 0 | 0 | 0 | 0 | 0 | 0 | 0 |
| Amphiphilic alkoxylated grease cleaning polymer³ | 0.4 | 1 | 1 | 1 | 1 | 0 | 0 | 0 | 0.78 |
| CHEC | 0.2 | 0 | 0 | 0 | 0 | 0 | 0.17 | 0.15 | 0.2 |
| Ethanol | 1.6 | 1 | 1 | 1.11 | 1.11 | 0.42 | 0.42 | 0.82 | 0 |
| Propylene Glycol | 1.3 | 1.21 | 1.21 | 1.27 | 1.27 | 1.25 | 1.25 | 2.44 | 0.33 |
| Diethylene glycol | 0 | 0 | 0 | 0 | 0 | 0 | 0 | 0 | 0 |
| Polyethylene glycol | 0 | 0 | 0 | 0 | 0 | 0 | 0 | 0 | 0 |
| Protease as defined herein | 0.008 | 0.05 | 0.03 | 0.15 | 0.01 | 0.04 | 0.05 | 0.04 | 0.00 9 |
| ⁴Amylase Amplify^{®} (mg active) | 2.5 | 8 | 7 | 8 | 7 | 7 | 7 | 8 | 7 |
| ⁵DNase (mg active per 100g detergent) | 2 | 2.2 | 2 | 2.2 | 2 | 2 | 2 | 2.2 | 2 |
| ⁶Alginate lyase (mg active per 100g of detergent) | 2.1 | | 4.1 | 3.2 | 4.1 | 4.1 | 4.1 | 3.2 | 4.1 |
| ⁷Hexosaminidase (mg active per 100g detergent) | 2.5 | 7 | 3 | 7 | 3 | 3 | 3 | 7 | 3 |
| ⁸Pel-ase (mg active per 100g composition) | 3.9 | 2.2 | 1.1 | 2.2 | 1.1 | 1.1 | 1.1 | 2.2 | 1.1 |
| ⁹Psl-ase (mg active per 100g composition) | 3.2 | 3.4 | 4.4 | 3.4 | 4.4 | 4.4 | 4.4 | 3.4 | 4.4 |
| Monoethanolamine | 0.4 | 2.2 | 2.2 | 2.32 | 2.32 | 0.24 | 0.24 | 0.56 | 0.63 |
| NaOH | 0 | 0.006 | 0.006 | 0.007 | 0.00 7 | 3.11 | 3.11 | 0.9 | 1 |
| Sodium Cumene Sulphonate | 1 | 0.25 | 0.25 | 0.26 | 0.26 | 1.72 | 1.72 | 0 | 2 |
| Sodium Formate | 0 | 0 | 0 | 0 | 0 | 0.034 | 0.034 | 0.01 | 0.01 |
| Polyethoxylated azo thiophene dye | 0.001 | 0.025 | 0.025 | 0.026 | 0.02 6 | 0 | 0 | 0 | 0 |
| Antimicrobial | 0 | 0 | 0 | 0 | 0 | 0 | 0.03 | 0.025 | 0.04 |
| Water, Aesthetics (Dyes, perfumes) and Minors (Enzymes including lipase, each at 0.2% active protein, solvents, structurants) | balance | | | | | | | | |

Examples 30-36. Unit Dose Laundry detergent compositions. Such unit dose formulations can comprise one or multiple compartments.

| | 30 (wt%) | 31 (wt%) | 32 (wt%) | 33 (wt%) | 34 (wt%) | 35 (wt%) | 36 (wt%) |
|---|---|---|---|---|---|---|---|
| Alkylbenzene sulfonic acid | 14.5 | 14.5 | 14.5 | 14.5 | 14.5 | 23 | 24.6 |
| C12-18 alkyl ethoxy 2.5-3 sulfate | 7.5 | 7.5 | 7.5 | 7.5 | 7.5 | 16 | 12.5 |
| C12-18 alkyl 7-ethoxylate | 13.0 | 13.0 | 13.0 | 13.0 | 13.0 | 3.1 | 3.8 |
| polyethylene glycol ether (Lutensol XL 100) | 0 | 0 | 0 | 0 | 0 | 1.0 | 0.5 |
| Citric Acid | 0.6 | 0.6 | 0.6 | 0.6 | 0.6 | 0.9 | 0.7 |
| Fatty Acid (e.g. palm kernel) | 14.8 | 14.8 | 14.8 | 14.8 | 14.8 | 6.5 | 5.3 |
| Ethoxylated polyethyleneimine³ | 1.6 | 1 | 1.6 | 1.2 | 1.6 | 0.6 | 1.6 |
| Amphiphilic graft copolymer⁵ | 1.9 | 1.8 | 2.6 | 2.2 | 2.6 | 1.6 | 2.6 |
| Zwitterionic polyamine⁶ | 1.2 | 1.2 | 1.8 | 1.3 | 1.8 | 0.8 | 1.8 |
| Amylase (wt% active protein) | 0.01 | 0.006 | 0.003 | 0.007 | 0.03 | 0.04 | 0.005 |
| Ethoxylated Polyethylenimine² | 4.0 | 4.0 | 4.0 | 4.0 | 4.0 | 4.0 | 4.0 |
| Protease as defined herein (wt% active protein) | 0.11 | 0.03 | 0.09 | 0.05 | 0.07 | 0.2 | 0.07 |
| Xyloglucanase (wt% active protein) | 0.005 | 0.002 | 0.008 | 0.001 | 0.005 | 0.007 | 0.005 |
| ⁵DNase (wt% active protein) | 0.005 | 0.01 | 0.02 | 0.02 | 0.01 | 0.02 | 0.01 |
| ⁴Amylase Amplify^{®} (wt% active protein) | 0.001 | 0.002 | 0.003 | 0.001 | 0.005 | 0.007 | 0.002 |
| Mannanase (wt% active protein) | 0.001 | 0.002 | 0.003 | 0.001 | 0.005 | 0.007 | 0.003 |
| ⁷Hexosaminidase (wt% active protein) | 0-0.001 | 0-0.002 | 0-0.003 | 0-0.001 | 0-0.005 | 0-0.007 | 0-0.002 |
| ⁸Pel-ase (wt% active protein) | 0-0.002 | 0-0.003 | 0-0.002 | 0-0.001 | 0-0.005 | 0-0.007 | 0-0.002 |
| ⁹Psl-ase (wt% active protein) | 0-0.005 | 0-0.002 | 0-0.003 | 0-0.001 | 0-0.005 | 0-0.007 | 0-0.002 |
| Hydroxyethane diphosphonic acid | 1.2 | 1.2 | 1.2 | 1.2 | 1.2 | 0 | 2.2 |
| Silicone anti-foam | 0.3 | 0.3 | 0.4 | 0.3 | 0.3 | 0.2 | 0.3 |
| Brightener 49 | 0.3 | 0.3 | 0.3 | 0.3 | 0.3 | 0.2 | 0.4 |
| P-diol | 15.8 | 13.8 | 13.8 | 13.8 | 13.8 | 12.2 | 12.3 |
| Glycerol | 6.1 | 6.1 | 6.1 | 6.1 | 6.1 | 4.0 | 4.7 |
| MEA | 8.0 | 8.0 | 8.0 | 8.0 | 8.0 | 8.6 | 10.2 |
| DPG (dipropylene glycol) | 1.7 | 1.7 | 1.7 | 1.7 | 1.7 | 0 | 1.7 |
| TPG (tripropylene glycol) | 0.1 | 0.1 | 0.1 | 0.1 | 0 | 0.5 | 0.1 |
| Sorbitol | 0.1 | 0.1 | 0.1 | 0.1 | 0.1 | 0.2 | 0.1 |
| Texcare SRA300 anionic polyester terephthalate | 0.1 | 0.2 | 0.1 | 0.4 | 0.3 | 0.1 | 0.6 |
| Cumene sulphonate | 0.0 | 0.0 | 0.0 | 0.0 | 2.0 | 0.0 | 0.0 |
| Cyclohexyl dimethanol | 0.0 | 0.0 | 0.0 | 2.0 | 0.0 | 0.0 | 0.0 |
| K₂SO₃ | 0.4 | 0.1 | 0.0 | 0.0 | 0.0 | 0.0 | 0.4 |
| MgCl₂ | 0.3 | 0.2 | 0.0 | 0.0 | 0.0 | 0.0 | 0.3 |
| Water | 10 | 10 | 10 | 10 | 10 | 10 | 10 |
| Structurant | 0.14 | 0.14 | 0.14 | 0.14 | 0.14 | 0.14 | 0.14 |
| Hueing dye | - | 0.03 | 0.03 | 00.3 | 0.03 | 0.02 | 0.05 |
| Perfume | 1.9 | 1.9 | 2 | 1.9 | 1.9 | 1.9 | 2.1 |
| Buffers (monoethanolamine) | To pH 8.0 | | | | | | |
| Solvents & minors | To 100% | | | | | | |

### Example 37. Multiple Compartment Unit Dose Composition

The following is a multi-compartment water soluble unit dose laundry article comprising a larger bottom compartment while having two smaller compartments in a side by side configuration superposed on top of the bottom compartment, following the Ariel 3-in-1 Pods design, as commercially available in the UK in January 2020. The below compositions are enclosed in a polyvinyl alcohol based water soluble outer film, more specifically a water soluble film comprising a blend of a polyvinylalcohol homopolymer and a carboxylated anionic polyvinylalcohol copolymer, and a water soluble middle film comprising a blend of polyvinyl alcohol homopolymers, alternatively a blend of a polyvinylalcohol homopolymer and a carboxylated anionic polyvinylalcohol copolymer.

| Ingredients | Full article Composition (wt%) | Bottom compartment Composition (wt%) | Top compartment Composition 1 (wt%) | Top compartment Composition 2 (wt%) |
|---|---|---|---|---|
| Volume | 25.5ml | 22.3ml | 1.6ml | 1.6ml |
| Fatty alcohol ethoxylate nonionic surfactant, C12-14 average degree of ethoxylation of 7 | 3.5 | 3.7 | 2.6 | 1.6 |
| Lutensol XL100 | 0.4 | 0.5 | - | - |
| Linear C11-14 alkylbenzene sulphonate | 24.2 | 24.9 | 18.9 | 19.4 |
| C12-15 AE3S Ethoxylated alkyl sulphate with an average degree of ethoxylation of 3 | 12.3 | 12.6 | 9.7 | 9.7 |
| Citric acid | 0.7 | 0.7 | 0.5 | 0.5 |
| Palm Kernel Fatty acid | 5.2 | 5.4 | 4.1 | 4.1 |
| Nuclease enzyme (wt% active protein) | 0.009 | 0.011 | - | - |
| Protease as described herein (wt% active protein) | 0.05 | 0.06 | 0.03 | 0.05 |
| Amylase enzyme (wt% active protein) | 0.004 | 0.005 | 0.001 | 0.004 |
| Xyloglucanase enzyme (wt% active protein) | 0.005 | - | 0.073 | - |
| Mannanase enzyme (wt% active protein) | 0.003 | 0.003 | - | - |
| Lipase enzyme (wt% active protein) | 0.012 | - | 0.187 | - |
| Ethoxylated polyethyleneimine³ | 1.5 | 1.6 | 1.2 | 1.2 |
| Amphiphilic graft copolymer⁵ | 2.0 | 2.3 | - | - |
| Zwitterionic polyamine⁶ | 1.8 | 1.9 | 1.4 | 1.4 |
| Anionic polyester terephthalate (Texcare SRA300) | 0.4 | - | - | 5.8 |
| HEDP chelant | 2.2 | 2.2 | 1.7 | 1.7 |
| Brightener 49 | 0.3 | 0.4 | 0.01 | 0.01 |
| Silicone anti-foam | 0.3 | 0.3 | - | - |
| Hueing dye | 0.04 | - | 0.69 | - |
| 1,2 PropaneDiol | 13.6 | 12.8 | 11.3 | 26.4 |
| Glycerine | 6.0 | 5.0 | 17.3 | 8.3 |
| DPG (DiPropyleneGlycol) | 0.8 | 0.8 | 0.6 | 0.6 |
| TPG (TriPropyleneGlycol) | 0.06 | 0.06 | - | - |
| Sorbitol | 0.6 | 0.05 | 8.8 | - |
| Monoethanolamine | 10.0 | 10.4 | 7.9 | 8.0 |
| K2SO3 | 0.4 | 0.4 | 0.04 | 0.4 |
| MgCl2 | 0.3 | 0.3 | 0.2 | 0.2 |
| water | 10.9 | 10.9 | 11.8 | 9.9 |
| Hydrogenated castor oil | 0.1 | 0.1 | - | 0.1 |
| Perfume | 1.6 | 1.9 | - | - |
| Aesthetic dye & Minors (incl. preservative) | Balance to 100 | Balance to 100 | Balance to 100 | Balance to 100 |
| pH (10% product concentration in demineralized water at 20°C) | 7.4 | 7.4 | 7.4 | 7.4 |

Raw Materials and Notes for Composition Examples 1-29

Linear alkylbenzenesulfonate having an average aliphatic carbon chain length C11-C18

C12-18 Dimethylhydroxyethyl ammonium chloride

AE3S is C12-15 alkyl ethoxy (3) sulfate

AE7 is C12-15 alcohol ethoxylate, with an average degree of ethoxylation of 7

AE9 is C12-16 alcohol ethoxylate, with an average degree of ethoxylation of 9

HSAS is a mid-branched primary alkyl sulfate with carbon chain length of about 16-17 as disclosed in US 6,020,303 and US 6,060,443

Polyacrylate MW 4500 is supplied by BASF

Carboxymethyl cellulose is Finnfix^{®} V supplied by CP Kelco, Amhem, Netherlands

CHEC is a cationically modified hydroxyethyl cellulose polymer.

Phosphonate chelants are, for example, diethylenetetraamine pentaacetic acid (DTPA) Hydroxyethane di phosphonate (HEDP)

Savinase^{®}, Natalase^{®}, Stainzyme^{®}, Lipex^{®}, CellucleanTM, Mannaway^{®} and Whitezyme^{®} are all products of Novozymes, Bagsvaerd, Denmark.

Purafect^{®}, Purafect Prime^{®} are products of Genencor International, Palo Alto, California, USA

Fluorescent Brightener 1 is Tinopal^{®} AMS, Fluorescent Brightener 2 is Tinopal^{®} CBS-X, Direct Violet 9 is Pergasol^{®} Violet BN-Z NOBS is sodium nonanoyloxybenzenesulfonate

TAED is tetraacetylethylenediamine

S-ACMC is carboxymethylcellulose conjugated with C.I. Reactive Blue 19product name AZO-CM-CELLULOSE

Soil release agent is Repel-o-tex^{®} PF

Acrylic Acid/Maleic Acid Copolymer is molecular weight 70,000 and acrylate:maleate ratio 70:30

EDDS is a sodium salt of ethylenediamine-N,N'-disuccinic acid, (S,S) isomer Suds suppressor agglomerate is supplied by Dow Corning, Midland, Michigan, USA

HSAS is mid-branched alkyl sulfate

Liquitint^{®} Violet CT polymeric hueing dye, supplied by Milliken, Spartanburg, South Carolina, USA

Polyethoxylated azo thiophene dye is Violet DD^{™} polymeric hueing dye, supplied by Milliken, Spartanburg, South Carolina, USA.

¹Alkoxylated ethylene diamine is bis((C2H5O)(C2H4O)n)(CH3)-N+-CxH2x-N+-(CH3)-bis((C2H5O)(C2H4O)n); n is 20 to 30; x is 3 to 8, optionally sulphated or sulphonated

² Random graft co-polymeris a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.

³ Polyethyleneimine (MW = 600) with 20 ethoxylate groups per -NH, e.g. Lutensol FP620 ex BASF.

⁴ Amphiphilic alkoxylated polymer is a polyethylenimine (MW 600), e.g.prepared from a polymer that is derivatised to contain 24 ethoxylate groups per -NH and 16 Propoxylate groups per -NH.

5Polyethylene glycol graft polymer comprising a polyethylene glycol backbone (Pluriol E6000) and hydrophobic vinyl acetate side chains, comprising 40% by weight of the polymer system of a polyethylene glycol backbone polymer and 60% by weight of the polymer system of the grafted vinyl acetate side chains

⁶Lutensit Z96 (zwitterionic polyamine ex BASF - zwitterionic hexamethylene diamine according to below formula : 100% quaternized and about 40% of the polyethoxy (EO24) groups are sulfonated).

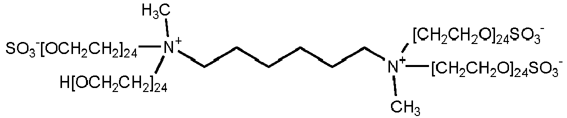

⁴Amylase otherwise indicated is shown as mgs of active enzyme per 100g of detergent.

⁵DNase as described herein (unless otherwise indicated mgs of active enzyme per 100g of detergent). DNase may comprise minor amounts of super oxide dismutase impurity.

⁶Alginate lyase (otherwise indicated shown as mgs of active enzyme per 100g of detergent)

⁷Hexosaminidase as described herein (otherwise indicated shown as mgs of active enzyme per 100g of detergent)

⁸Pel-ase as described herein (otherwise indicated shown as mgs of active enzyme per 100g of detergent)

⁹Psl-ase as described herein (otherwise indicated shown as mgs of active enzyme per 100g of detergent)

Proxel GXL, 20% aqueous dipropylene glycol solution of 1,2-benzisothiazolin-3-one, supplied by Lonza.

^{b} N,N-bis(hydroxyethyl)-N,N-dimethyl ammonium chloride fatty acid ester. The iodine value of the parent fatty acid of this material is between 18 and 22. The material as obtained from Evonik contains impurities in the form of free fatty acid, the monoester form of N,N-bis(hydroxyethyl)-N,N-dimethyl ammonium chloride fatty acid ester, and fatty acid esters of N,N-bis(hydroxyethyl)-N-methylamine.

^{c} MP10^{®}, supplied by Dow Corning, 8% activity

^{d} as described in US 8,765,659, expressed as 100% encapsulated perfume oil

^{e} Rheovis^{®} CDE, cationic polymeric thickener supplied by BASF

^{f} N,N-dimethyl octanamide and N,N-dimethyl decanamide in about a 55:45 weight ratio, tradename Steposol^{®} M-8-10 from the Stepan Company

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A cleaning composition, comprising (a) a subtilisin variant comprising two or more substitutions comprising X006W, X024K, X055P, X109Q, X162Q, X182Q, X183N, X204Q, X206Y, X222Q, X248A, X254A, or a combination thereof, wherein the variant has at least 80% but less than 100% identity to the amino acid sequence of SEQ ID NO: 1, wherein the positions are numbered by correspondence with the amino acid sequence of SEQ ID NO:1 and (b) a cleaning adjunct.

2. A cleaning composition according to claim 1, wherein the substitution comprises Y006W, S024K, T055P, N109Q, S162Q, S182Q, S183N, S204Q, Q206Y, M222Q, S248A, T254A or a combination thereof.

3. A cleaning composition according to claim 1, wherein the variant comprises an additional substitution comprising X003Q, X022Y, X033T, X045V, X053G, X076D, X078N, X087D, X101N, X118R, X128A, X128S, X145R, X166Q, X169A, X217Q, X218S, or a combination thereof.

4. A cleaning composition according to claim 1, wherein the variant comprises one or more additional substitutions comprising S003Q, T022Y, S033T, A045V, S053G, N076D, S078N, S087D, S101N, N118R, G128A, G128S, S145R, G166Q, G169A, Y217Q, N218S, or a combination thereof.

5. A cleaning composition according to claim 1, wherein the subtilisin variant comprises two mutations comprising X006W-X024K, X006W-X055P, X006W-X109Q, X006W-X162Q, X006W-X183N, X006W-X182Q, X006W-X204Q, X006W-X206Y, X006W-X222Q, X006W-X248A, X006W-X254A, X024K-X055P, X024K-X109Q, X024K-X162Q, X024K-X183N, X024K-X182Q, X024K-X204Q, X024K-X206Y, X024K-X222Q, X024K-X248A, X024K-X254A, X055P-X109Q, X055P-X162Q, X055P-X183N, X055P-X182Q, X055P-X204Q, X055P-X206Y, X055P-X222Q, X055P-X248A, X055P-X254A, X109Q-X162Q, X109Q-X183N, X109Q-X182Q, X109Q-X204Q, X109Q-X206Y, X109Q-X222Q, X109Q-X248A, X109Q-X254A, X162Q-X183N, X162Q-X182Q, X162Q-X204Q, X162Q-X206Y, X162Q-X222Q, X162Q-X248A, X162Q-X254A, X183N-X182Q, X183N-X204Q, X183N-X206Y, X183N-X222Q, X183N-X248A, X183N-X254A, X182Q-X204Q, X182Q-X206Y, X182Q-X222Q, X182Q-X248A, X182Q-X254A, X204Q-X206Y, X204Q-X222Q, X204Q-X248A, X204Q-X254A, X206Y-X222Q, X206Y-X248A, X206Y-X254A, X222Q-X248A, X222Q-X254A, X248A-X254A, or a combination thereof, wherein the positions are numbered by correspondence with the amino acid sequence of SEQ ID NO:1.

6. A cleaning composition according to claim 1, wherein the subtilisin variant comprises three mutations comprising X006W-X024K-X055P, X006W-X024K-X109Q, X006W-X024K-X162Q, X006W-X024K-X183N, X006W-X024K-X182Q, X006W-X024K-X204Q, X006W-X024K-X206Y, X006W-X024K-X222Q, X006W-X024K-X248A, X006W-X024K-X254A, X006W-X055P-X109Q, X006W-X055P-X162Q, X006W-X055P-X183N, X006W-X055P-X182Q, X006W-X055P-X204Q, X006W-X055P-X206Y, X006W-X055P-X222Q, X006W-X055P-X248A, X006W-X055P-X254A, X006W-X109Q-X162Q, X006W-X109Q-X183N, X006W-X109Q-X182Q, X006W-X109Q-X204Q, X006W-X109Q-X206Y, X006W-X109Q-X222Q, X006W-X109Q-X248A, X006W-X109Q-X254A, X006W-X162Q-X183N, X006W-X162Q-X182Q, X006W-X162Q-X204Q, X006W-X162Q-X206Y, X006W-X162Q-X222Q, X006W-X162Q-X248A, X006W-X162Q-X254A, X006W-X183N-X182Q, X006W-X183N-X204Q, X006W-X183N-X206Y, X006W-X183N-X222Q, X006W-X183N-X248A, X006W-X183N-X254A, X006W-X182Q-X204Q, X006W-X182Q-X206Y, X006W-X182Q-X222Q, X006W-X182Q-X248A, X006W-X182Q-X254A, X006W-X204Q-X206Y, X006W-X204Q-X222Q, X006W-X204Q-X248A, X006W-X204Q-X254A, X006W-X206Y-X222Q, X006W-X206Y-X248A, X006W-X206Y-X254A, X006W-X222Q-X248A, X006W-X222Q-X254A, X006W-X248A-X254A, X024K-X055P-X109Q, X024K-X055P-X162Q, X024K-X055P-X183N, X024K-X055P-X182Q, X024K-X055P-X204Q, X024K-X055P-X206Y, X024K-X055P-X222Q, X024K-X055P-X248A, X024K-X055P-X254A, X024K-X109Q-X162Q, X024K-X109Q-X183N, X024K-X109Q-X182Q, X024K-X109Q-X204Q, X024K-X109Q-X206Y, X024K-X109Q-X222Q, X024K-X109Q-X248A, X024K-X109Q-X254A, X024K-X162Q-X183N, X024K-X162Q-X182Q, X024K-X162Q-X204Q, X024K-X162Q-X206Y, X024K-X162Q-X222Q, X024K-X162Q-X248A, X024K-X162Q-X254A, X024K-X183N-X182Q, X024K-X183N-X204Q, X024K-X183N-X206Y, X024K-X183N-X222Q, X024K-X183N-X248A, X024K-X183N-X254A, X024K-X182Q-X204Q, X024K-X182Q-X206Y, X024K-X182Q-X222Q, X024K-X182Q-X248A, X024K-X182Q-X254A, X024K-X204Q-X206Y, X024K-X204Q-X222Q, X024K-X204Q-X248A, X024K-X204Q-X254A, X024K-X206Y-X222Q, X024K-X206Y-X248A, X024K-X206Y-X254A, X024K-X222Q-X248A, X024K-X222Q-X254A, X024K-X248A-X254A, X055P-X109Q-X162Q, X055P-X109Q-X183N, X055P-X109Q-X182Q, X055P-X109Q-X204Q, X055P-X109Q-X206Y, X055P-X109Q-X222Q, X055P-X109Q-X248A, X055P-X109Q-X254A, X055P-X162Q-X183N, X055P-X162Q-X182Q, X055P-X162Q-X204Q, X055P-X162Q-X206Y, X055P-X162Q-X222Q, X055P-X162Q-X248A, X055P-X162Q-X254A, X055P-X183N-X182Q, X055P-X183N-X204Q, X055P-X183N-X206Y, X055P-X183N-X222Q, X055P-X183N-X248A, X055P-X183N-X254A, X055P-X182Q-X204Q, X055P-X182Q-X206Y, X055P-X182Q-X222Q, X055P-X182Q-X248A, X055P-X182Q-X254A, X055P-X204Q-X206Y, X055P-X204Q-X222Q, X055P-X204Q-X248A, X055P-X204Q-X254A, X055P-X206Y-X222Q, X055P-X206Y-X248A, X055P-X206Y-X254A, X055P-X222Q-X248A, X055P-X222Q-X254A, X055P-X248A-X254A, X109Q-X162Q-X183N, X109Q-X162Q-X182Q, X109Q-X162Q-X204Q, X109Q-X162Q-X206Y, X109Q-X162Q-X222Q, X109Q-X162Q-X248A, X109Q-X162Q-X254A, X109Q-X183N-X182Q, X109Q-X183N-X204Q, X109Q-X183N-X206Y, X109Q-X183N-X222Q, X109Q-X183N-X248A, X109Q-X183N-X254A, X109Q-X182Q-X204Q, X109Q-X182Q-X206Y, X109Q-X182Q-X222Q, X109Q-X182Q-X248A, X109Q-X182Q-X254A, X109Q-X204Q-X206Y, X109Q-X204Q-X222Q, X109Q-X204Q-X248A, X109Q-X204Q-X254A, X109Q-X206Y-X222Q, X109Q-X206Y-X248A, X109Q-X206Y-X254A, X109Q-X222Q-X248A, X109Q-X222Q-X254A, X109Q-X248A-X254A, X162Q-X183N-X182Q, X162Q-X183N-X204Q, X162Q-X183N-X206Y, X162Q-X183N-X222Q, X162Q-X183N-X248A, X162Q-X183N-X254A, X162Q-X182Q-X204Q, X162Q-X182Q-X206Y, X162Q-X182Q-X222Q, X162Q-X182Q-X248A, X162Q-X182Q-X254A, X162Q-X204Q-X206Y, X162Q-X204Q-X222Q, X162Q-X204Q-X248A, X162Q-X204Q-X254A, X162Q-X206Y-X222Q, X162Q-X206Y-X248A, X162Q-X206Y-X254A, X162Q-X222Q-X248A, X162Q-X222Q-X254A, X162Q-X248A-X254A, X183N-X182Q-X204Q, X183N-X182Q-X206Y, X183N-X182Q-X222Q, X183N-X182Q-X248A, X183N-X182Q-X254A, X183N-X204Q-X206Y, X183N-X204Q-X222Q, X183N-X204Q-X248A, X183N-X204Q-X254A, X183N-X206Y-X222Q, X183N-X206Y-X248A, X183N-X206Y-X254A, X183N-X222Q-X248A, X183N-X222Q-X254A, X183N-X248A-X254A, X182Q-X204Q-X206Y, X182Q-X204Q-X222Q, X182Q-X204Q-X248A, X182Q-X204Q-X254A, X182Q-X206Y-X222Q, X182Q-X206Y-X248A, X182Q-X206Y-X254A, X182Q-X222Q-X248A, X182Q-X222Q-X254A, X182Q-X248A-X254A, X204Q-X206Y-X222Q, X204Q-X206Y-X248A, X204Q-X206Y-X254A, X204Q-X222Q-X248A, X204Q-X222Q-X254A, X204Q-X248A-X254A, X206Y-X222Q-X248A, X206Y-X222Q-X254A, X206Y-X248A-X254A, X222Q-X248A-X254A, or a combination thereof, wherein the positions are numbered by correspondence with the amino acid sequence of SEQ ID NO:1.

7. A cleaning composition according to claim 1, wherein the subtilisin variant comprises two mutations comprising, Y006W-S024K, Y006W-T055P, Y006W-N109Q, Y006W-S162Q, Y006W-S183N, Y006W-S182Q, Y006W-S204Q, Y006W-Q206Y, Y006W-M222Q, Y006W-S248A, Y006W-T254A, S024K-T055P, S024K-N109Q, S024K-S162Q, S024K-S183N, S024K-S182Q, S024K-S204Q, S024K-Q206Y, S024K-M222Q, S024K-S248A, S024K-T254A, T055P-N109Q, T055P-S162Q, T055P-S183N, T055P-S182Q, T055P-S204Q, T055P-Q206Y, T055P-M222Q, T055P-S248A, T055P-T254A, N109Q-S162Q, N109Q-S183N, N109Q-S182Q, N109Q-S204Q, N109Q-Q206Y, N109Q-M222Q, N109Q-S248A, N109Q-T254A, S162Q-S183N, S162Q-S182Q, S162Q-S204Q, S162Q-Q206Y, S162Q-M222Q, S162Q-S248A, S162Q-T254A, S183N-S182Q, S183N-S204Q, S183N-Q206Y, S183N-M222Q, S183N-S248A, S183N-T254A, S182Q-S204Q, S182Q-Q206Y, S182Q-M222Q, S182Q-S248A, S182Q-T254A, S204Q-Q206Y, S204Q-M222Q, S204Q-S248A, S204Q-T254A, Q206Y-M222Q, Q206Y-S248A, Q206Y-T254A, M222Q-S248A, M222Q-T254A, S248A-T254A, or a combination thereof, wherein the positions are numbered by correspondence with the amino acid sequence of SEQ ID NO:1.

8. A cleaning composition according to claim 1, wherein the subtilisin variant comprises three mutations comprising Y006W-S024K-T055P, Y006W-S024K-N109Q, Y006W-S024K-S162Q, Y006W-S024K-S183N, Y006W-S024K-S182Q, Y006W-S024K-S204Q, Y006W-S024K-Q206Y, Y006W-S024K-M222Q, Y006W-S024K-S248A, Y006W-S024K-T254A, Y006W-T055P-N109Q, Y006W-T055P-S162Q, Y006W-T055P-S183N, Y006W-T055P-S182Q, Y006W-T055P-S204Q, Y006W-T055P-Q206Y, Y006W-T055P-M222Q, Y006W-T055P-S248A, Y006W-T055P-T254A, Y006W-N109Q-S162Q, Y006W-N109Q-S183N, Y006W-N109Q-S182Q, Y006W-N109Q-S204Q, Y006W-N109Q-Q206Y, Y006W-N109Q-M222Q, Y006W-N109Q-S248A, Y006W-N109Q-T254A, Y006W-S162Q-S183N, Y006W-S162Q-S182Q, Y006W-S162Q-S204Q, Y006W-S162Q-Q206Y, Y006W-S162Q-M222Q, Y006W-S162Q-S248A, Y006W-S162Q-T254A, Y006W-S183N-S182Q, Y006W-S183N-S204Q, Y006W-S183N-Q206Y, Y006W-S183N-M222Q, Y006W-S183N-S248A, Y006W-S183N-T254A, Y006W-S182Q-S204Q, Y006W-S182Q-Q206Y, Y006W-S182Q-M222Q, Y006W-S182Q-S248A, Y006W-S182Q-T254A, Y006W-S204Q-Q206Y, Y006W-S204Q-M222Q, Y006W-S204Q-S248A, Y006W-S204Q-T254A, Y006W-Q206Y-M222Q, Y006W-Q206Y-S248A, Y006W-Q206Y-T254A, Y006W-M222Q-S248A, Y006W-M222Q-T254A, Y006W-S248A-T254A, S024K-T055P-N109Q, S024K-T055P-S162Q, S024K-T055P-S183N, S024K-T055P-S182Q, S024K-T055P-S204Q, S024K-T055P-Q206Y, S024K-T055P-M222Q, S024K-T055P-S248A, S024K-T055P-T254A, S024K-N109Q-S162Q, S024K-N109Q-S183N, S024K-N109Q-S182Q, S024K-N109Q-S204Q, S024K-N109Q-Q206Y, S024K-N109Q-M222Q, S024K-N109Q-S248A, S024K-N109Q-T254A, S024K-S162Q-S183N, S024K-S162Q-S182Q, S024K-S162Q-S204Q, S024K-S162Q-Q206Y, S024K-S162Q-M222Q, S024K-S162Q-S248A, S024K-S162Q-T254A, S024K-S183N-S182Q, S024K-S183N-S204Q, S024K-S183N-Q206Y, S024K-S183N-M222Q, S024K-S183N-S248A, S024K-S183N-T254A, S024K-S182Q-S204Q, S024K-S182Q-Q206Y, S024K-S182Q-M222Q, S024K-S182Q-S248A, S024K-S182Q-T254A, S024K-S204Q-Q206Y, S024K-S204Q-M222Q, S024K-S204Q-S248A, S024K-S204Q-T254A, S024K-Q206Y-M222Q, S024K-Q206Y-S248A, S024K-Q206Y-T254A, S024K-M222Q-S248A, S024K-M222Q-T254A, S024K-S248A-T254A, T055P-N109Q-S162Q, T055P-N109Q-S183N, T055P-N109Q-S182Q, T055P-N109Q-S204Q, T055P-N109Q-Q206Y, T055P-N109Q-M222Q, T055P-N109Q-S248A, T055P-N109Q-T254A, T055P-S162Q-S183N, T055P-S162Q-S182Q, T055P-S162Q-S204Q, T055P-S162Q-Q206Y, T055P-S162Q-M222Q, T055P-S162Q-S248A, T055P-S162Q-T254A, T055P-S183N-S182Q, T055P-S183N-S204Q, T055P-S183N-Q206Y, T055P-S183N-M222Q, T055P-S183N-S248A, T055P-S183N-T254A, T055P-S182Q-S204Q, T055P-S182Q-Q206Y, T055P-S182Q-M222Q, T055P-S182Q-S248A, T055P-S182Q-T254A, T055P-S204Q-Q206Y, T055P-S204Q-M222Q, T055P-S204Q-S248A, T055P-S204Q-T254A, T055P-Q206Y-M222Q, T055P-Q206Y-S248A, T055P-Q206Y-T254A, T055P-M222Q-S248A, T055P-M222Q-T254A, T055P-S248A-T254A, N109Q-S162Q-S183N, N109Q-S162Q-S182Q, N109Q-S162Q-S204Q, N109Q-S162Q-Q206Y, N109Q-S162Q-M222Q, N109Q-S162Q-S248A, N109Q-S162Q-T254A, N109Q-S183N-S182Q, N109Q-S183N-S204Q, N109Q-S183N-Q206Y, N109Q-S183N-M222Q, N109Q-S183N-S248A, N109Q-S183N-T254A, N109Q-S182Q-S204Q, N109Q-S182Q-Q206Y, N109Q-S182Q-M222Q, N109Q-S182Q-S248A, N109Q-S182Q-T254A, N109Q-S204Q-Q206Y, N109Q-S204Q-M222Q, N109Q-S204Q-S248A, N109Q-S204Q-T254A, N109Q-Q206Y-M222Q, N109Q-Q206Y-S248A, N109Q-Q206Y-T254A, N109Q-M222Q-S248A, N109Q-M222Q-T254A, N109Q-S248A-T254A, S162Q-S183N-S182Q, S162Q-S183N-S204Q, S162Q-S183N-Q206Y, S162Q-S183N-M222Q, S162Q-S183N-S248A, S162Q-S183N-T254A, S162Q-S182Q-S204Q, S162Q-S182Q-Q206Y, S162Q-S182Q-M222Q, S162Q-S182Q-S248A, S162Q-S182Q-T254A, S162Q-S204Q-Q206Y, S162Q-S204Q-M222Q, S162Q-S204Q-S248A, S162Q-S204Q-T254A, S162Q-Q206Y-M222Q, S162Q-Q206Y-S248A, S162Q-Q206Y-T254A, S162Q-M222Q-S248A, S162Q-M222Q-T254A, S162Q-S248A-T254A, S183N-S182Q-S204Q, S183N-S182Q-Q206Y, S183N-S182Q-M222Q, S183N-S182Q-S248A, S183N-S182Q-T254A, S183N-S204Q-Q206Y, S183N-S204Q-M222Q, S183N-S204Q-S248A, S183N-S204Q-T254A, S183N-Q206Y-M222Q, S183N-Q206Y-S248A, S183N-Q206Y-T254A, S183N-M222Q-S248A, S183N-M222Q-T254A, S183N-S248A-T254A, S182Q-S204Q-Q206Y, S182Q-S204Q-M222Q, S182Q-S204Q-S248A, S182Q-S204Q-T254A, S182Q-Q206Y-M222Q, S182Q-Q206Y-S248A, S182Q-Q206Y-T254A, S182Q-M222Q-S248A, S182Q-M222Q-T254A, S182Q-S248A-T254A, S204Q-Q206Y-M222Q, S204Q-Q206Y-S248A, S204Q-Q206Y-T254A, S204Q-M222Q-S248A, S204Q-M222Q-T254A, S204Q-S248A-T254A, Q206Y-M222Q-S248A, Q206Y-M222Q-T254A, Q206Y-S248A-T254A, M222Q-S248A-T254A, or a combination thereof, wherein the positions are numbered by correspondence with the amino acid sequence of SEQ ID NO: 1.

9. A cleaning composition according to claim 1, wherein the subtilisin variant is derived from a parent having about 85% or more sequence identity to SEQ ID NO: 1.

10. A cleaning composition according to claim 9, wherein the subtilisin variant comprises an amino acid sequence having about 90% to less than 100% sequence identity to the amino acid sequence of SEQ ID NO: 1.

11. A cleaning composition according to claim 1, wherein the subtilisin variant has improved stability when compared to a reference subtilisin lacking the two, three, four or more substitutions.

12. A cleaning composition according to claim 11 wherein the improved stability is measured as a residual activity (fraction of 1) greater than 0.2 after 20 minutes at 54, 56, 60, or 66 degrees Celsius in a 10% detergent solution in comparison to the respective parent according to the assay described in Example 2, or an improved stability measured as a percent residual activity of about 20% or more after 20 minutes at 40, 51, or 53 degrees Celsius in a 10% detergent solution according to the assay described in Example 2.

13. A cleaning composition according to claim 1, wherein the subtilisin variant has protease activity.

14. A cleaning composition according to claim 1, wherein the subtilisin variant does not have an amino acid sequence identical to a naturally occurring molecule.

15. A cleaning composition according to claim 1, wherein the composition is free of boron-containing compounds.

16. A cleaning composition according to claim 1, wherein the cleaning composition is a laundry detergent, optionally in liquid form.

17. A cleaning composition according to claim 1, wherein the cleaning adjunct comprises a nonionic surfactant.

18. A cleaning composition according to claim 1, wherein the subtilisin variant is derived from a parent having about 95% or more sequence identity to SEQ ID NO: 1, and the subtilisin variant has about 90% but less than 100% sequence identity to SEQ ID NO: 1.

19. A method of cleaning a kitchen, bathroom, dish, or fabric surface, the method comprising contacting the surface with an aqueous wash liquor comprising a composition according to claim 1.

20. A method of cleaning according to claim 19, wherein the surface is a fabric surface.
